# EUROPEAN PATENT SPECIFICATION

(11) **EP 3 905 881 B1**
(45) Date of publication and mention of the grant of the patent: **25.11.2026**
(21) Application number: 20701135.4
(22) Date of filing: 02.01.2020
(51) Int. Cl.: C12Q 1/6806

(54) **URINE STABILIZATION**
URINSTABILISIERUNG
STABILISATION D'URINE

(30) Priority: 04.01.2019 EP 19150438
(43) Date of publication of application: 10.11.2021
(60) Divisional application: 24172186.9 / 4 397 771
(73) Proprietor: QIAGEN GmbH, 40724 Hilden (DE)
(72) Inventor: GRÖLZ, Daniel, 40724 Hilden (DE)
(74) Representative: Hoffmann Eitle
(86) International application number: PCT/EP2020/050006
(87) International publication number: WO 2020/141187

(56) References cited:
- WO-A1-02/083140
- WO-A1-2014/146780
- WO-A1-2014/151437
- WO-A1-2015/140218
- WO-A1-2017/085321
- WO-A1-93/15234

## Description

### FIELD OF THE INVENTION

The present invention provides methods and uses for stabilizing urine samples, as well as a stabilizing composition for stabilizing a urine sample mixed with a urine sample. The technology of the present invention allows the simultaneous stabilization of cell-free and genomic DNA as well as stabilization of cells in urine.

### BACKGROUND OF THE INVENTION

Most of the DNA in the body is located within cells, but a small amount of nucleic acids can also be found circulating freely in human body fluids, such as blood and urine (commonly referred to as extracellular, cell-free or also circulating DNA). The presence of elevated levels of extracellular DNA and other extracellular nucleic acids in many medical conditions, malignancies, and infectious processes is of interest *inter alia* for screening, diagnosis, prognosis, surveillance for disease progression, for identifying potential therapeutic targets, and for monitoring treatment response.

The degradation of extracellular DNA in the body fluid sample is a problem as well as a possible dilution of extracellular DNA by genetic material from damaged or decaying cells after sample collection depending on the sample type.

To avoid respectively reduce the above described problems it is e.g. recommended to either obtain blood plasma from whole blood basically directly after the blood is drawn and/or to stabilize the blood sample until plasma can be obtained. Stabilization and isolation of extracellular DNA from blood samples is meanwhile well established. For stabilization of the blood sample, several dedicated blood collection tubes are commercial available (Streck, Roche, PreAnalytiX, Biomatrica, Norgene and other). Stabilization methods are e.g. described in WO 2013/045457, WO 2013/045458, WO 2014/146780, WO 2014/146782, WO 2014/146781, WO 2014/049022, WO 2015/140218 and WO 2017/085321.

Drawing blood, despite being much less invasive compared to biopsy taking, is still invasive and can be difficult or impossible especially in case of elderly people, infants or cancer patients under treatment. A truly noninvasive sample as an alternative source for therapy monitoring, resistance failure testing, or even diagnosis would be a valuable complement to current liquid biopsy techniques. Purification of cell-free DNA from urine offers here a convenient and completely non-invasive alternative.

That urine can be used as a liquid biopsy has been shown in many peer reviewed articles. In addition to conventional cytohistological analysis of i.e. urothelial carcinoma, gDNA and cell-free DNA from cancerous or malignant cells in urine have been associated with cancer specific sequence, epigenetic and structural changes. It has been published that urine from bladder- (Birkenkamp-Demtröder K et al. Genomic Alterations in Liquid Biopsies from Patients with Bladder Cancer. Eur Urol. 2016 Jul;70(1):75-82), prostate- (Salvi S et al. Urine Cell-Free DNA Integrity Analysis for Early Detection of Prostate Cancer Patients. Dis Markers. 2015;2015:574120), lung- (Chen S, Zhao J, Cui L, Liu Y. Urinary circulating DNA detection for dynamic tracking of EGFR mutations for NSCLC patients treated with EGFR-TKIs. Clin Transl Oncol. 2017 Mar;19(3):332-340) and other solid tumor cancer type patients (Fujii T et al. Mutation-Enrichment Next-Generation Sequencing for Quantitative Detection of KRAS Mutations in Urine Cell-Free DNA from Patients with Advanced Cancers. Clin Cancer Res. 2017 Jul 15;23(14):3657-3666) contain tumor derived cell-free DNA. Furthermore, the isolation of foetal DNA from urine samples has been described. Therefore, besides blood, also urine is an important liquid biopsy sample.

However, both nucleated cells as well as cell-free DNA in urine are unstable and rapidly lyse or degrade upon urine sample collection. In addition exfoliated, nucleated cells such as normal epithelial cells or cancer derived cells in urine release genomic DNA which leads to a dilution of cell-free DNA. DNA degradation is particularly rapid in urine and essentially instantly occurs after sample collection. Therefore, efficient stabilization methods are needed to preserve the cell-free DNA in urine samples by reducing degradation of the comprised cell-free DNA during storage. In this respect it is noted that efficient preservation of the cell-free DNA comprised in the original urine sample is difficult to determine based on the total amount of cell-free DNA that is comprised in the urine sample after storage. This is because the originally present cell-free DNA that becomes degraded during storage can be supplemented with DNA that is released from cells that are comprised in the urine sample. Hence, even if the total amount of DNA in the cell-free urine fraction is the same upon urine sample collection and after storage, the originally present cell-free DNA may nevertheless be degraded and the DNA that is comprised in the cell-free urine fraction after storage may essentially correspond to DNA that has been released from cells during storage. However, it is the cell-free DNA originally present in the cell-free urine fraction that is of particular analytical value and not the DNA that is released during storage from the cells into the cell-free liquid urine fraction. If the originally present cell-free DNA is degraded during storage it is not available for subsequent analysis. Therefore, an important aim of urine stabilization is to stabilize the cell-free DNA that is comprised in urine at the time of sample collection. In addition, it is desirous that the urine stabilization technology achieves a stabilization of comprised cells so that cells can be retrieved from the stabilized urine sample for further use, such as cytohistological test methods and/or isolation of genomic DNA from the cells. Known urine stabilization methods do not sufficiently address these aims.

One known urine stabilization method (Streck - Cell free DNA urine preserve) is based on the use of a formaldehyde releaser. The cell-free DNA urine preservative is provided in 5ml vials. The reagent from one vial has to be mixed with 25 to 100ml urine. This approach has several disadvantages. It requires that unstabilized urine is brought to a lab or physician's office. Thereby the timepoint when the urine is stabilized is delayed. In addition for stabilization the primary collection cup has to be opened and the stabilization reagents have to be added from the vial and mixed with the urine. These manual interactions are inconvenient and error-prone. Furthermore, reagents containing formaldehyde have the disadvantage to be toxic and to chemically modify biomolecules like proteins and nucleic acids. This can reduce the amount of recoverable and hence analyzable cell-free DNA. Since the number of the cell-free DNA of interest can be limited in urine samples this is a huge disadvantage.

Trovagene has a reagent (NextCollect) which according to the manufacturer stabilizes cell-free DNA in urine. This reagent was integrated into the cup of a urine collection container. Zymo and Norgen offer reagents which preserve urine for subsequent total DNA applications. However, a separate analysis of genomic and cell-free DNA is not possible.

Furthermore, several other urine collection devices are known in the art. E.g. Becton Dickenson (BD) offers a product-line for transfer of urine into a vacutainer. The BD Vacutainer^{®} urine collection cup is a collection container with a cup including a needle, for transfer of urine into a vacutainer without the need to open the cup. The BD Vacutainer^{®} urine collection straw is a device for transfer of urine from any container to a vacutainer. One end of the device is placed into the urine, the other end includes a needle which can be attached to a vacutainer. The BD Vacutainer^{®} Urinanlysis Preservative Tubes are vacutainer with spray dried bacterial preservative with a 8ml draw volume or Tubes without additive and draw volumes of 8 to 10ml, intended to be used with urine. None of these vacutainer do preserve DNA.

Improved methods are therefore needed that allow the efficient stabilization of urine samples as liquid biopsy sample. The stabilization should allow the subsequent isolation of cell-free DNA as well as cells comprised in the urine sample.

It is thus one object of the present invention to provide means for stabilizing urine samples. In particular, it is one object to overcome at least one of the drawbacks of the prior art urine stabilization methods. It is furthermore one object to provide a stabilization technology which subsequently allows the recovery of cell-free DNA and/or intracellular DNA, such as genomic DNA and furthermore, enables a cytological analysis of the stabilized cells.

WO 2014/151437 relates to a preservative reagent for stabilizing cells in urine specimen, in particular the stabilization of tumor cells in urine for size-selective microfiltration with the CellSieve^{™} membrane.

WO 02/083140 relates to quinazoline containing compounds that are inhibitors of the serine/threonine kinase AKT, pharmaceutical compositions comprising said compounds and their use in the treatment of cancer.

WO 93/15234 relates to activated stable Factor XIII (plasma transglutaminase) and a process of producing said factor.

### SUMMARY OF THE INVENTION

The invention can be defined by the appending claims.

The present invention provides an effective stabilization technology for urine samples, which preserves extracellular DNA (also referred to herein as cell-free DNA) as well as comprised cells and intracellular nucleic acids such as genomic DNA. The provided stabilization technology in particular allows to inhibit the degradation of cell-free DNA as well as bacterial growth in urine samples. Accordingly, the stabilization technology of the invention stabilizes cell-free DNA in urine as well as genomic DNA and the cytology of cells found within urine. The present invention thereby provides a solution for simultaneous stabilization of cell-free and genomic DNA as well as stabilization of cells in urine. Thereby, multimodality testing, including cell-free and genomic DNA testing as well as conventional cytological staining is possible with urine samples that are stabilized with the technology according to the present invention.

Furthermore, disclosed but not claimed as such is a sample collection, stabilization and preparation system for urine. The stabilization chemistry of the invention can be comprised in a urine reception container (not claimed as such), preferably an evacuated tube, which can be used in combination with established urine collection containers and established nucleic acid purification and assay technologies. Thereby, the disclosure also provides a standardized system for molecular analyses from urine samples including the whole workflow from urine collection to insight nucleic acid and/or cell analyses.

According to a first aspect, a method for stabilizing a urine sample, in particular extracellular DNA comprised therein, according to claim 1 is provided comprising contacting the urine sample for stabilization with a stabilizing composition which comprises a chelating agent, preferably EDTA, and at least one poly(oxyethylene) polymer, preferably polyethylene glycol, wherein bacterial growth is inhibited in the stabilized urine sample, wherein the stabilizing composition has a pH in the range of > 7 to ≤ 9.5, and wherein the stabilization does not involve the use of a cross-linking agent that induces protein-nucleic acid and/or protein-protein crosslinks such as formaldehyde, formaline, paraformaldehyde or a formaldehyde releaser.

According to a second aspect, a method for processing a urine sample according to claim 15 is provided comprising the steps of
a) stabilizing the urine sample according to the method of the first aspect; and
b) separating cells from the stabilized urine sample, thereby providing (i) a cell sample and (ii) a cell-depleted supernatant.

According to a third aspect, disclosed but not claimed as such is a stabilizing composition for stabilizing a urine sample, in particular extracellular DNA comprised therein, wherein bacterial growth is inhibited in the stabilized urine sample, the composition comprising
- a chelating agent, preferably EDTA,
- at least one poly(oxyethylene) polymer, preferably polyethylene glycol, and optionally
- a preservative for inhibiting bacterial growth,
wherein the stabilizing composition has a pH in the range of > 7 to ≤ 9.5, and wherein the stabilizing composition does not comprise a cross-linking agent that induces protein-DNA and/or protein-protein crosslinks such as formaldehyde, formaline, paraformaldehyde or a formaldehyde releaser.

A respective stabilizing composition allows the storage and/or handling, e.g. shipping, of the stabilized urine sample at room temperature for days without substantially compromising the quality of the sample. The stabilization composition can be part of a closed urine collection system which is easy to handle, safe and without the need for manual interactions.

According to a fourth aspect, the present invention pertains to the use of a stabilizing composition according to third aspect for stabilizing a urine sample, as claimed in claim 16.

The stabilizing composition according to the third aspect can be e.g. used for stabilization in the method according to the first aspect.

According to a fifth aspect, disclosed but not claimed as such is a reception device for receiving a urine sample, wherein the reception device comprises
- a chelating agent, preferably EDTA,
- at least one poly(oxyethylene) polymer, preferably polyethylene glycol, and
- optionally a preservative for inhibiting bacterial growth.

The reception device is suitable for stabilizing a urine sample. Bacterial growth can be inhibited in the stabilized urine sample. An according reception device, which preferably is an evacuated collection tube, preferably comprises a stabilizing composition according to the third aspect. Providing such respective reception device has the advantage that the urine sample is immediately stabilized as soon as the sample is contained in the respective device. The reception device can be used for stabilization in the method according to the first aspect.

According to a sixth aspect, disclosed but not claimed as such is a method for collecting a urine sample. According to one embodiment, the urine sample is collected into a chamber of a device according to the fifth aspect of the present invention. According to a further embodiment, the method for collecting a urine sample comprises
a) collecting urine in a container, preferably a urine cup; and
b) stabilizing the urine sample using the method according to the first aspect.

The urine collected in step a) may be transferred for stabilization in step b) into a reception device according to the fifth aspect, wherein the reception device preferably comprises a stabilizing composition according to the third aspect.

According to a seventh aspect, disclosed but not claimed as such is a method of producing a composition according to the third aspect, wherein the components of the stabilizing composition are combined, preferably in a liquid composition such as a solution. The term "solution" as used herein in particular refers to a liquid composition, preferably an aqueous composition. It may be a homogenous mixture of only one phase but it is also within the scope of the present invention that a solution comprises solid components such as e.g. precipitates.

Other objects, features, advantages and aspects of the present application will become apparent to those skilled in the art from the following description and appended claims.

### BRIEF DESCRIPTION OF THE FIGURES

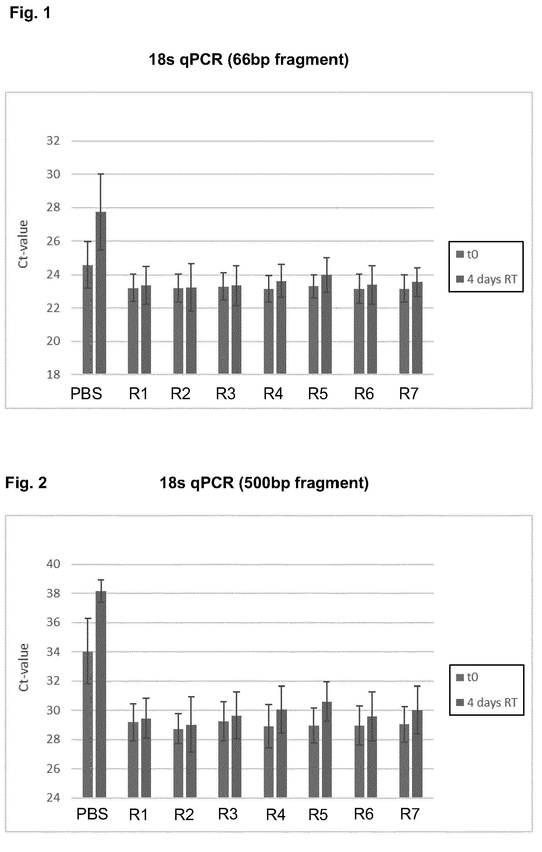

**Figure 1****:** Shows the stabilization of cell-free DNA in urine. Shown are average Ct values of 18s rDNA 66bp fragment by qPCR from 6 donors with standard deviation. Urine was either mixed with 1.7ml phosphate buffered saline (PBS) or stabilization reagents according to the present disclosure. DNA was isolated from the processed, cell-depleted urine sample directly (t0, left columns) or after 4 days storage at RT (right columns).
**Figure 2****:** Shows the stabilization of large DNA in urine. Shown are average Ct values of 18s rDNA 500bp fragment by qPCR from 6 donors with standard deviation. Urine was either mixed with 1.7ml phosphate buffered saline (PBS) or stabilization reagents according to the present disclosure. DNA was isolated from the cell-depleted urine sample directly (t0, left columns) or after 4 days storage at RT (right columns).

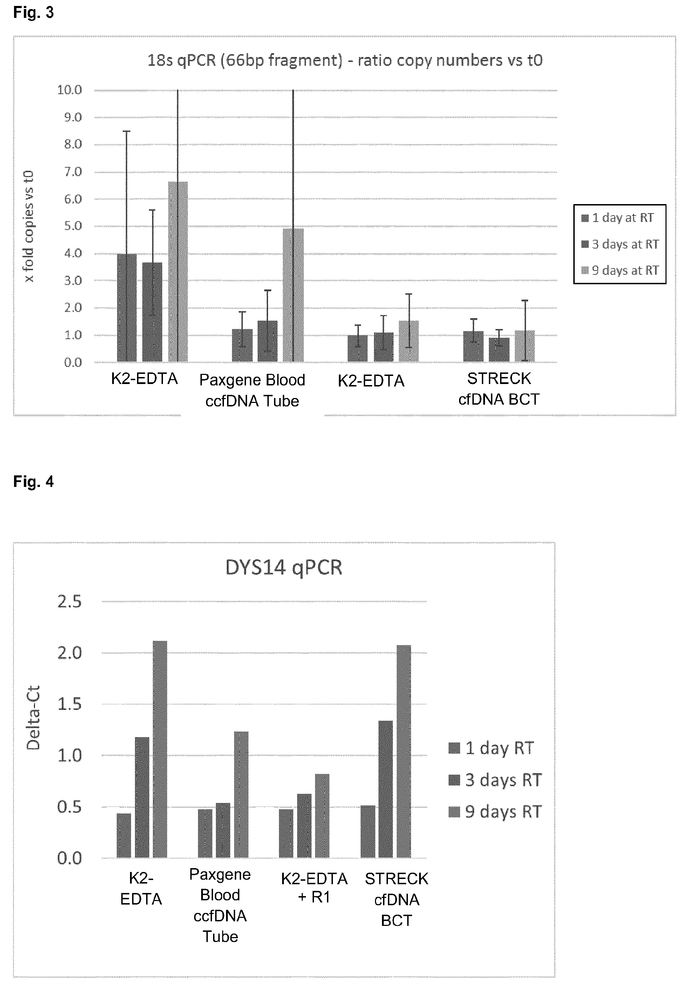

**Figure 3****:** Cell-free DNA in urine collected from BD Vacutainer Urine Collection Cup directly into vacutainer. Increase of copy numbers of 18s rDNA 66bp fragments after storage versus t0, determined by qPCR from 4 donors (two female and two male) with standard deviation is shown. Urine was collected into BD Vacutainer collection cups and transferred into different vacutainers. For stabilization with R1, urine was transferred into spray dried K2-EDTA tubes and 1.7ml R1 reagent was added afterwards. Cell-free DNA was isolated directly or after 1 day (left column), 3 days (middle column) and 9 days (right column) storage at RT from the cell-depleted urine sample.
**Figure 4****:** Preservation of cell-free DNA from male urine spiked in female urine collected from BD Vacutainer Urine Collection Cup directly into vacutainer. Average delta Ct values (DYS14 qPCR, 2 female donors) between samples stored at RT and samples processed at timepoint t0 are shown. Cell-free DNA was isolated directly or after 1 day (left column), 3 days (middle column) and 9 days (right column) storage at RT. A lower Delta-Ct value is indicative of a better preservation of the cell-free DNA that is comprised in the urine sample.

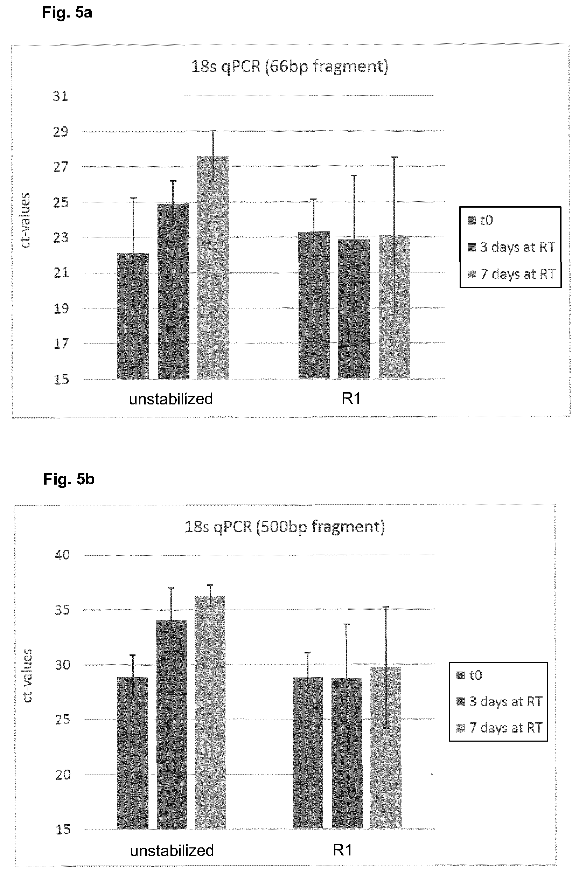

**Figure 5****:** Stabilization of cell-free DNA in urine used for multimodality testing. Average Ct values of 18s rDNA 66 fragment (Fig. 5a) and 500bp fragment (Fig. 5b) by qPCR from 3 donors, duplicate sample preparation and triplicate PCR with standard deviation are shown. Urine was either unstabilized or mixed after collection with 1.7ml reagent R1. Cell-free DNA was isolated from the processed urine sample directly after collection (t0, left column) or after 3 days (middle column) and 7 days (right column) storage at RT.

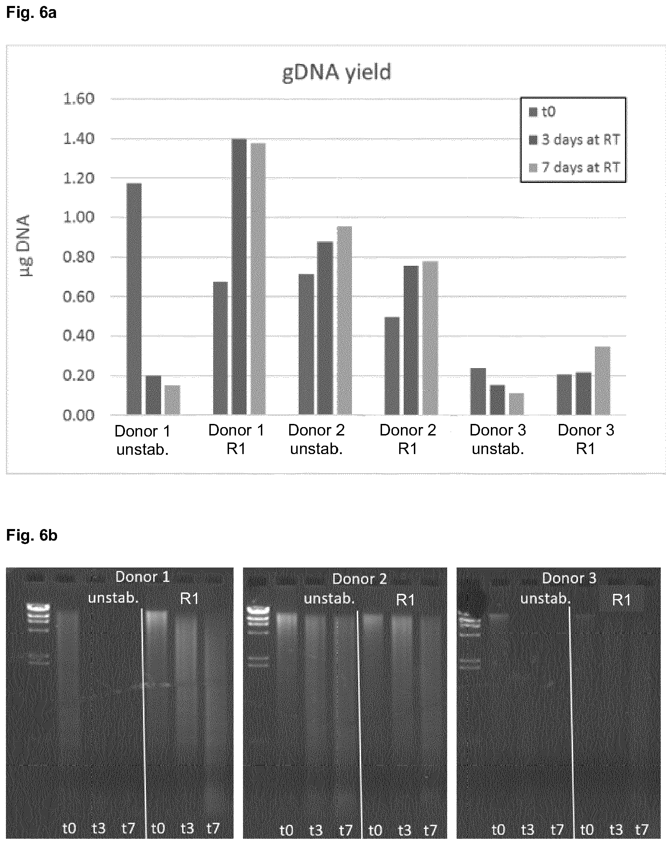

**Figure 6****:** Stabilization of genomic DNA derived from urine cells, in a multimodality test setup. Average yield from 2/3 of the cell pellet from three different donors, measured on a TapeStation (Fig. 6a) and visualized on an agarose gel (Fig. 6b). Urine was either unstabilized or mixed after collection with 1.7ml reagent R1. gDNA was isolated from cell pellets generated by centrifugation directly after collection (t0, left column) or after 3 days (middle column) and 7 days (right column) storage at RT.

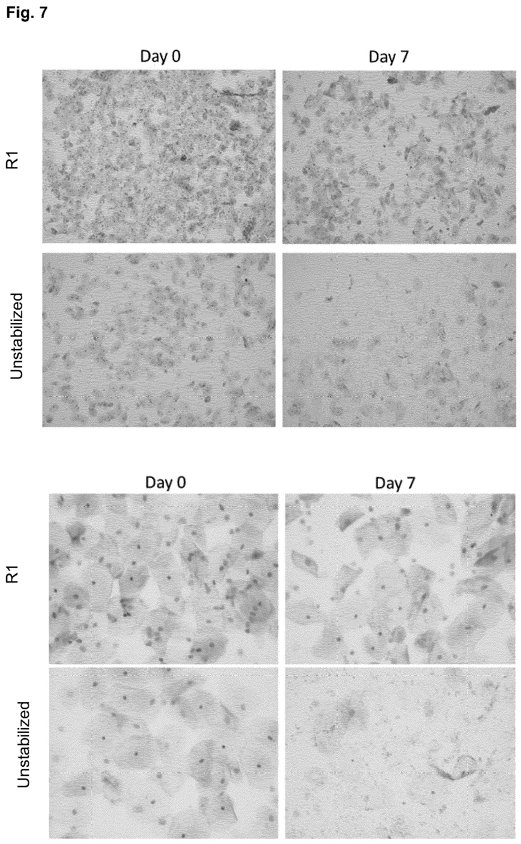

**Figure 7****:** Stabilization of cell cytology from urine cells, in a multimodality test setup. Urine was either unstabilized or mixed after collection with 1.7ml reagent R1. Cells from cell pellets generated by one centrifugation directly after collection (t0) or after 7 days storage at RT were centrifuged on a glass slide, air-dried and stained with Papanicolaou.

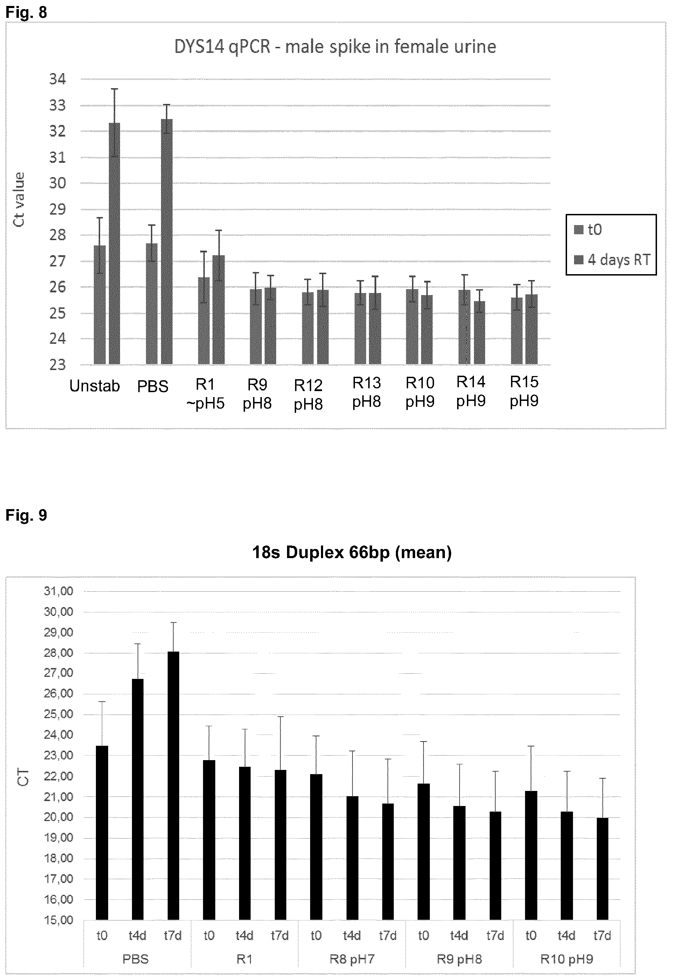

**Figure 8****:** Effect of pH on preservation of cell-free DNA as demonstrated based on male derived DYS14 DNA spiked into female urine. Average Ct values of DYS14 qPCR from 6 female donors with standard deviation are shown. Female urine was either used pure (Unstab.) or mixed with 1.7ml phosphate buffered saline (PBS) or various stabilizing reagents according to the present disclosure. Cell-free DNA was isolated directly (t0, left column) or after 4 days (right column) storage at RT.
**Figure 9** shows the stabilization of cell-free DNA urine with stabilizing reagents according to the invention. The average Ct values of 18S rDNA 66bp fragment by qPCR from 6 donors with standard deviation are shown. Urine was mixed with either 1.7 ml phosphate buffered saline (PBS) or stabilization reagents according to the invention set to different pH values. Cell-free DNA was isolated directly (t0), or after 4 (t4d) or 7 (t7d) days of storage after urine processing.

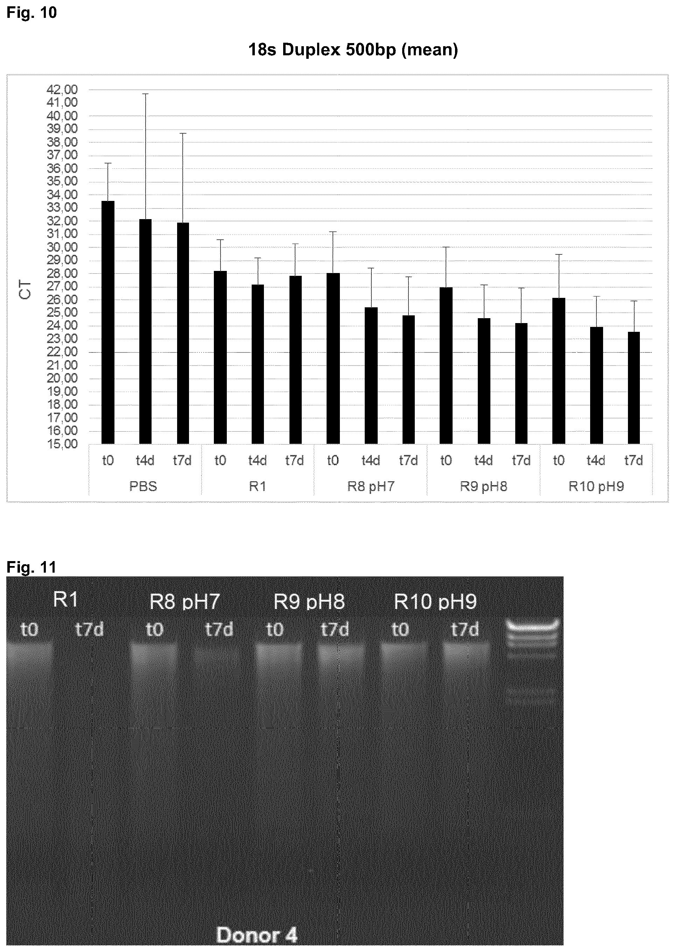

**Figure 10** shows the stabilization of large cell-free DNA in urine with reagents according to the invention. The average Ct values of 18S rDNA 500bp fragment by qPCR from 6 donors with standard deviation are shown. Urine was mixed with either 1.7 ml phosphate buffered saline (PBS) or stabilization reagents according to the invention at different pH values. Cell-free DNA was isolated from processed urine directly (t0), or after 4 (t4d) or 7 (t7d) days of storage.
**Figure 11** shows the stabilization of genomic DNA derived from urine cells. Urine was stabilized with 1.7 ml of a stabilizing reagent according to invention at different pH values. gDNA was isolated from cell pellets generated by centrifugation directly after collection (t0) or after 7 days (t7d) of storage at room temperature.

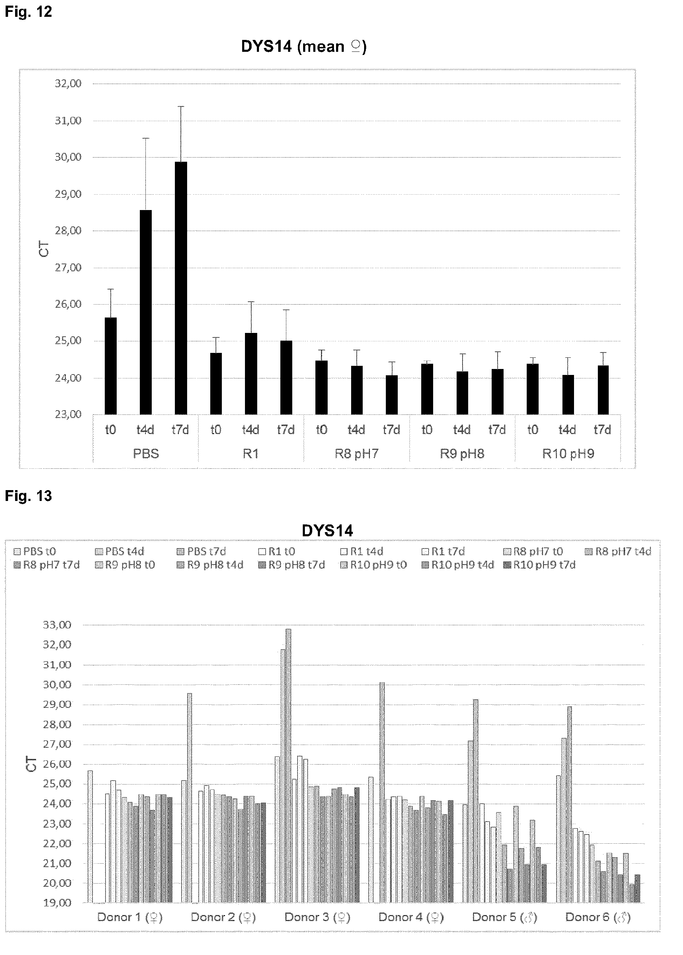

**Figure 12** shows the effect of pH on preservation of cell-free male derived DYS14 DNA in female urine. Average Ct values of DYS14 qPCR from 4 female donors with standard deviation are shown. Female urine was either mixed with 1.7ml phosphate buffered saline (PBS) or stabilization reagents according to the invention at different pH values. Cell-free DNA was isolated directly (t0) or after 4 (t4d) or 7 (t7d) days storage at RT.
**Figure 13** shows the effect of pH on preservation of cell-free male derived DYS14 DNA in urine. Ct values of DYS14 qPCR from 4 female and 2 male donors are shown. Urine was either mixed with 1.7ml phosphate buffered saline (PBS) or stabilization reagents according to the invention set to different pH values. Cell-free DNA was isolated directly (t0) or after 4 (t4d) or 7 (t7d) days storage at RT. The male donors (donors 5 and 6) show an increase of DYS14 DNA in the cfDNA over time, when a reagent according to the invention with a neutral or basic pH is used.

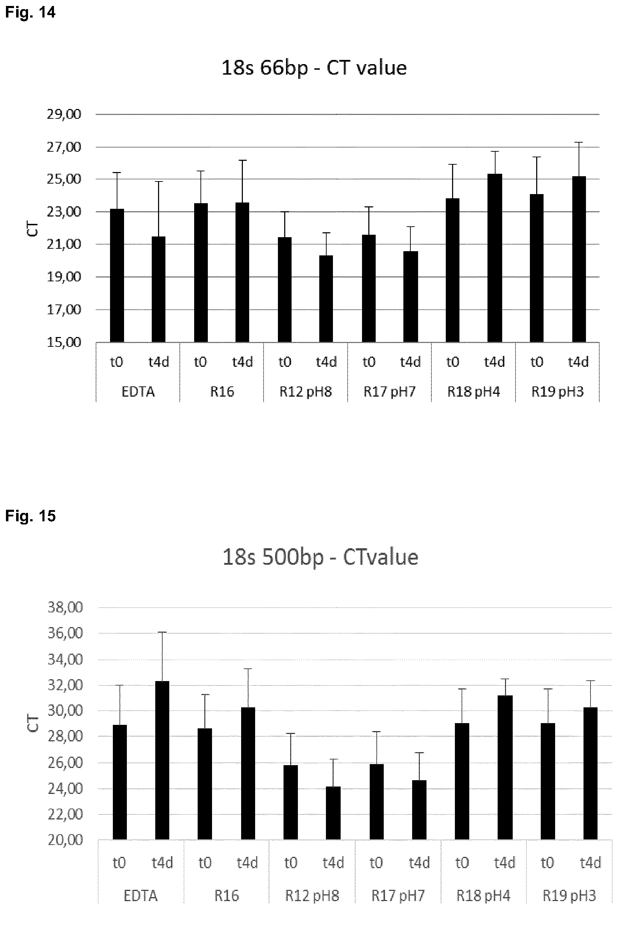

**Figure 14** shows the stabilization of small DNA in cell-free urine with reagents according to the invention. The average Ct values of 18S rDNA 66bp fragment by qPCR from 6 donors with standard deviation are shown. Urine was mixed with either 1.7 ml phosphate buffered saline (PBS) or stabilization reagents according to the invention at different pH values. Cell-free DNA was isolated directly (t0), or after 4 (t4d) days of storage.
**Figure 15** shows the stabilization of large DNA in cell-free urine with reagents according to the invention. The mean Ct values of 18S rDNA 500bp fragment by qPCR from 6 donors with standard deviation are shown. Urine was mixed with either 1.7 ml of stabilization reagents according to the invention set to different pH values. Cell-free DNA was isolated directly (t0), or after 4 (t4d) days of storage.

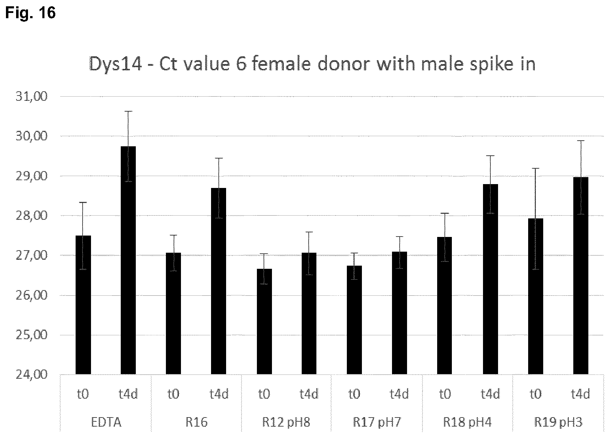

**Figure 16** shows the effect of pH on preservation of cell-free male derived DYS14 DNA in female urine. Average Ct values of DYS14 qPCR from 6 female donors with standard deviation are shown. Female urine was either mixed with 1.7ml of stabilization reagents according to the invention set to different pH values. Cell-free DNA was isolated directly (t0) or after 4 (t4d) days storage at RT.

### DETAILED DESCRIPTION OF THIS INVENTION

The different aspects of the present disclosure are now described in further detail:

### A. METHOD OF STABILIZATION

According to a first aspect, a method for stabilizing a urine sample, in particular extracellular DNA comprised therein, is provided comprising contacting the urine sample for stabilization with a stabilizing composition which comprises a chelating agent, preferably EDTA, and at least one poly(oxyethylene) polymer, preferably polyethylene glycol, wherein bacterial growth is inhibited in the stabilized urine sample, wherein the stabilizing composition has a pH in the range of > 7 to ≤ 9.5, and wherein the stabilization does not involve the use of a cross-linking agent that induces protein-nucleic acid and/or protein-protein crosslinks such as formaldehyde, formaline, paraformaldehyde or a formaldehyde releaser. Details of the method according to the first aspect are also defined in claims 1 to 14.

Details of the compounds used for stabilization as well as further stabilizing agents that can be used for stabilizing the urine sample are disclosed in the following. As disclosed herein, the compounds used for stabilization are preferably comprised in a stabilizing composition that is contacted with the urine sample. Preferably, the stabilizing composition according to the third aspect is used for stabilizing the urine sample.

### Chelating agent

The urine stabilization according to the present invention involves the use of a chelating agent, preferably ethylenediamine tetraacetic acid (EDTA). A chelating agent is an organic compound that is capable of forming coordinate bonds with metals through two or more atoms of the organic compound. Chelating agents according to the present invention include, but are not limited to ethylenedinitrilotetraacetic acid (EDTA), diethylenetriaminepentaacetic acid (DTPA), ethylene glycol tetraacetic acid (EGTA) and N,N-bis(carboxymethyl)glycine (NTA) and furthermore, e.g. citrate or oxalate. Other chelating agents include but are not limited to salts of carboxylic acids such as citrate or oxalate and any combination thereof.

According to a preferred embodiment, EDTA is used as chelating agent. All disclosures and embodiments described in this application for a chelating agent in general, specifically apply and particularly refer to the preferred embodiment EDTA even if not explicitly stated. As used herein, the term "EDTA" indicates *inter alia* the EDTA portion of an EDTA compound such as, for example, K₂EDTA, K₃EDTA or Na₂EDTA.

Using a chelating agent such as EDTA has the advantageous effect that nucleases such as DNases and RNases are inhibited, thereby reducing degradation of the cell-free DNA comprised in the urine sample by nucleases. Therefore, the use of a chelating agent such as EDTA for stabilizing a urine sample is highly advantageous.

According to one embodiment, the final concentration of the chelating agent, preferably EDTA, in the stabilized urine sample and thus in the mixture that is obtained when contacting the urine sample with the chelating agent, the poly(oxyethylene) polymer, the preservative and optionally one or more additional additives lies in a range of 0.9mg/ml to 40mg/ml, such as e.g. 2mg/ml to 40mg/ml. Exemplary and preferred ranges are disclosed in the following, wherein the disclosed ranges are particularly suitable for the use of EDTA as chelating agent. The final concentration in the stabilized urine sample may be at least 4mg/ml, at least 5 mg/ml or at least 7mg/ml. The use of a chelating agent, such as preferably EDTA, in higher concentrations for stabilizing urine is advantageous to achieve a strong stabilizing effect. Therefore, in embodiments, the final concentration of the chelating agent, which preferably is EDTA, in the stabilized urine sample is at least 7mg/ml, preferably at least 10mg/ml. In the examples, a concentration of at least 15mg/ml was used. According to one embodiment, the final concentration of the chelating agent, preferably EDTA, lies in a range of 10mg/ml to 35 mg/ml or 10mg/ml to 30mg/ml. Also suitable is a final concentration in a range of 12mg/ml to 30mg/ml or 15mg/ml to 25mg/ml. In the examples, EDTA was used in a final concentration that lies in a range of 15mg/ml to 20mg/ml in the stabilized urine.

The chelating agent such as EDTA is comprised in the stabilizing composition that is contacted with the urine sample for stabilization. Preferably, the stabilizing composition according to the third aspect is used for this purpose.

However, a chelating agent such as EDTA alone does not achieve a sufficient stabilization effect for the purposes described herein as is demonstrated in the examples. Therefore, it is used in combination with further stabilizing agents as described herein.

### Poly(oxyethylene) polymer, preferably polyethylene glycol

The urine stabilization according to the present invention furthermore involves the use of at least one poly(oxyethylene) polymer as stabilizing agent. The term poly(oxyethylene) polymer in particular refers to an oligomer or polymer of ethylene oxide. It comprises at least two ethylene oxide units. Poly(oxyethylene) polymers are known in low and high molecular weights. Their molecular weight are usually multitudes of 44, the molecular weight of its monomer, and can range up to 100000. The molecular weight is indicated herein in Da. The poly(oxyethylene) polymer may be linear or branched or may have other geometries. A linear poly(oxyethylene) polymer is preferred. The poly(oxyethylene) polymer may be unsubstituted or substituted and preferably is a polyethylene glycol. Polyethylene glycol has in various molecular weights and in various concentrations a stabilization effect on cells and therefore can be used in order to stabilize a urine sample. However, also other poly(oxyethylene) polymers may be used that achieve a stabilization effect as is achieved by polyethylene glycol. As mentioned, also substituted poly(oxyethylene) polymers having a stabilizing effect may be used such as alkyl poly(oxyethylene) polymers, e.g. alkylpolyethylene glycols, but also poly(oxyethylene) esters, poly(oxyethylene) amines, poly(oxyethylene) thiol compounds, poly(oxyethylene) glycerides and others.

Polyethylene glycol is preferably used as poly(oxyethylene) polymer for urine stabilization. It preferably is unbranched and may be unsubstituted or substituted. Known substituted forms of polyethylene glycol include alkylpolyethylene glycols that are e.g. substituted at one or both ends with a C1-C5 alkyl group. Most preferably, unsubstituted polyethylene glycol of the formula HO-(CH₂CH₂O)ₙ-H is used. All disclosures and embodiments described in this application for the poly(oxyethylene) polymer in general, specifically apply and particularly refer to the preferred embodiment polyethylene glycol and specifically unsubstituted polyethylene glycol even if not explicitly stated.

The poly(oxyethylene) polymer can be used in various molecular weights. Higher molecular weight poly(oxyethylene) polymers were found to be more effective stabilizing agents than lower molecular weight poly(oxyethylene) polymers. To achieve an efficient stabilization with a lower molecular weight poly(oxyethylene) polymer, the use of a higher concentration is generally recommendable compared. For some applications it is preferred though to keep the amount of additives used for stabilization low. Therefore, in embodiments, a high molecular weight poly(oxyethylene) polymer is contacted with the urine sample for stabilization, as it allows to use lower concentrations of the poly(oxyethylene) polymer while achieving a strong stabilization effect.

Thus, according to one embodiment, a high molecular weight poly(oxyethylene) polymer having a molecular weight of at least 1500 is used as stabilizing agent and is contacted with the urine sample. The high molecular weight poly(oxyethylene) polymer may have a molecular weight that lies in a range selected from 1500 to 40000. Suitable ranges include 2000 to 35000, 2500 to 30000, 3000 to 25000, 3500 to 20000 and 4000 to 15000. Preferred is a range of 5000 to 20000, such as 5000 to 15000 or 6000 to 10000. These molecular weights are particularly preferred for the use of a polyethylene glycol, in particular an unsubstituted polyethylene glycol. Unsubstituted polyethylene glycol was also used in the examples. The molecular weight of a poly(oxyethylene) polymer having a specific molecular weight may vary within certain ranges conditional to manufacturing as is well-known to the skilled person.

The high molecular weight poly(oxyethylene) polymer is used in a concentration, wherein it exerts or supports the stabilization of the urine sample. Suitable concentrations can be determined by the skilled person, for example by testing different concentrations of a specific high molecular weight poly(oxyethylene) polymer in the test assays described in the examples. The achieved stabilization effect and the preferred concentration may depend on whether one or more additional stabilizing agents are used. Preferred combinations are described herein. According to one embodiment, the mixture that is obtained after contacting the urine sample with the high molecular weight poly(oxyethylene) polymer and optionally further additives comprises the high molecular weight poly(oxyethylene) polymer in a final concentration that lies in a range of 0.05% to 8% (w/v). Exemplary suitable ranges for the final concentration in the stabilized urine sample include 0.1% to 4% (w/v), 0.2% to 3% (w/v), 0.25% to 2.5% (w/v) and 0.3% to 2% (w/v). According to one embodiment, the high molecular weight poly(oxyethylene) polymer is used in lower concentration ranges such as 0.2% to 1.5% (w/v), 0.25% to 1.25% (w/v), 0.3% to 1% (w/v) or 0.4% to 0.75% (w/v). Using a high molecular weight poly(oxyethylene) polymer in a concentration of 1.25% (w/v) or less or 1% (w/v) or less and in particular in a concentration of 0.75% (w/v) or less can be advantageous in certain embodiments. The downstream processing, in particular the purification of cell-free DNA from the cell-free supernatant may be improved. It is advantageous to use a stabilization technology that is compatible with most standard nucleic acid isolation methods.

According to one embodiment, the poly(oxyethylene) polymer, which preferably is a polyethylene glycol, used for stabilization has a molecular weight below 1500 and may be a low molecular weight poly(oxyethylene) polymer having a molecular weight of 1000 or less. It is used in a concentration, wherein it can support the urine sample stabilizing effect. Suitable concentrations for different sample types can be determined by the skilled person, e.g. by testing different concentrations in the test assays described in the examples. A respective poly(oxyethylene) polymer, such as a low molecular weight poly(oxyethylene) polymer having a molecular weight of 1000 or less, can be present in the mixture that is obtained after contacting the urine sample with said poly(oxyethylene) polymer and optionally further additives in a final concentration that lies in a range of 0.5% to 10%. Exemplary ranges include 1.5% to 9%, 2% to 8%, 2 to 7%, 2.5% to 7% and preferably 3% to 6%. The percentage values refer to (w/v) in case the poly(oxyethylene) polymer is a solid and to (v/v) in case the poly(oxyethylene) polymer is a liquid. The low molecular weight poly(oxyethylene) polymer may have a molecular weight that lies in a range selected from 100 to 1000, 150 to 800 and 150 to 700. Preferably, the molecular weight lies in a range of 200 to 600 and more preferably 200 to 500 such as 200 to 400.

As described herein, at least two poly(oxyethylene) polymers may be used for stabilization, which differ in their molecular weight. They may be of the same kind and preferably both are a polyethylene glycol such as preferably an unsubstituted polyethylene glycol. According to one embodiment, the difference in the molecular weight is at least 500, at least 750, or at least 1000. According to one embodiment, the difference in the molecular weight is at least 2500, at least 3500 or at least 5000. It is advantageous to use two poly(oxyethylene) polymers that differ in their molecular weight. The stabilization effect of poly(oxyethylene) polymers appears to depend on their molecular weight. It was found that the higher molecular weight polymers provide a better stabilization efficiency. This embodiment wherein at least two poly(oxyethylene) polymers are used for stabilization that differ in their molecular weight is advantageous, because it allows to provide balanced compositions of poly(oxyethylene) polymers having the desired characteristics with respect to the stabilization effect to be achieved and characteristics required e.g. for certain downstream uses, such as the purification of cell-free DNA from the cell-depleted stabilized urine sample.

According to one embodiment, a high molecular weight poly(oxyethylene) polymer as defined above which has a molecular weight of at least 1500, preferably in a range of 5000 to 20000 such as 5000 to 15000, is used in combination with a low molecular weight poly(oxyethylene) polymer having a molecular weight of 1000 or less, preferably in a range of 200 to 600, and the urine sample is contacted with both types of poly(oxyethylene) polymers. Suitable embodiments are described herein. Using a high molecular weight poly(oxyethylene) polymer in combination with a low molecular weight poly(oxyethylene) polymer is advantageous, because the low molecular weight poly(oxyethylene) polymer allows to reduce the concentration of the high molecular weight poly(oxyethylene) polymer required to achieve an effective stabilization of the urine sample. This embodiment is advantageous because it provides more freedom with respect to the volume or amount of stabilizing composition that can be used. Therefore, stabilizing the extracellular nucleic acid population in a urine sample using a combination of a high and a low molecular weight poly(oxyethylene) polymer is a preferred embodiment. The low molecular weight poly(oxyethylene) polymer can be of the same kind as the high molecular weight poly(oxyethylene) polymer which was described above and preferably is a polyethylene glycol, such as an unsubstituted polyethylene glycol. Suitable and preferred concentrations and concentration ranges for the high and low molecular weight poly(oxyethylene) polymer are described above and may also be used in the embodiment wherein both types of polymers are used in combination.

According to one embodiment, the mixture that is obtained after contacting the urine sample with the high and the low molecular weight poly(oxyethylene) polymer and optionally further additives used for stabilization, comprises the high molecular weight poly(oxyethylene) polymer, which may have e.g. a molecular weight in the range of e.g. 4000 to 20000, in a final concentration that lies in a range of 0.15% to 1.5%, e.g. 0.2% to 1% (w/v), preferably 0.25% to 0.75% (w/v) such as 0.5% (w/v) and the low molecular weight poly(oxyethylene) polymer, which preferably has a molecular weight that lies in a range of 200 to 800, preferably 200 to 600, in a final concentration that lies in a range selected from 1.5% to 8%, preferably 2% to 7%, more preferred 2.5% to 6%. A polyethylene glycol, preferably unsubstituted, is preferably used as the high and as the low poly(oxyethylene) polymer. Suitable embodiments for the high and low molecular weight poly(oxyethylene) polymer are also described above and in conjunction with the different aspects and embodiments.

The urine sample for stabilization is contacted with the stabilizing composition which comprises a chelating agent and at least one poly(oxyethylene) polymer. Preferably, the stabilizing composition according to the third aspect is used for this purpose.

### Inhibition of bacterial growth

The method furthermore achieves that bacterial growth is inhibited in the stabilized urine sample. Bacterial load in urine samples can vary between a high load to almost sterile. Bacteria rapidly grow in urine. It is therefore advantageous to inhibit their growth efficiently because this increases the stabilization effect and the quality of the recovered nucleic acids. Bacteria internalize and digest DNA. Bacteria are a source for nucleic acid degrading enzymes such as DNases and also RNases. Therefore, inhibiting bacterial growth in the stabilized urine sample supports the prevention of nucleic acid degradation by such mechanisms. It furthermore reduces the risk that bacterial DNA is copurified from the sample and might interfere in downstream assays, such as sequencing applications or PCR.

Chelating agents such as EDTA can achieve in higher concentrations a bacteriostatic effect. To achieve inhibition of bacterial growth in the stabilized urine sample, the chelating agent, which is preferably EDTA, may thus be used in a high concentration. Suitable high concentrations were described above. Alternatively or preferably additionally, the urine sample may be contacted for stabilization with a preservative for inhibiting bacterial growth. If such preservative is used in addition to the chelating agent, which preferably is EDTA, the preservative accordingly differs from the used chelating agent.

According to one embodiment the method thus comprises additionally contacting the urine sample for stabilization with a preservative for inhibiting bacterial growth. The preservative may be an antimicrobial and preferably has antibacterial and optionally also antifungal effects. The preservative may comprise or consist of a chemical compound. The preservative may also comprise or consist of two or more compounds that inhibit bacterial growth. The preservative is used in a concentration so that bacterial growth is inhibited in the stabilized urine sample. Suitable concentrations can be determined by the skilled person for individual preservatives. The final concentration of the preservative in the stabilized urine may lie e.g. in the range of 0.01 to 0.7% w/v, such as 0.03 to 0.5 % w/v, or may lie in the range of 0.01 to 0.7% v/v, such as 0.03 to 0.5 %v/v.

According to one embodiment, the preservative may comprise or consist of sodium azide. As is demonstrated by the examples, sodium azide is particularly suitable. It may be used in a final concentration of e.g. 0.01% to 0.5% (w/v) in the stabilized urine sample. According to embodiments, the final concentration of sodium azide in the stabilized urine sample is in the range of 0.02% to 0.4% (w/v) such as 0.03% to 0.35% (w/v) or 0.04% to 0.3% (w/v).

Furthermore, the preservative may comprise or consist of at least one isothiazolinone compound, preferably methylchloroisothiazolinone and/or methylisothiazolinone. These compounds may be mixed in a ratio of 3:1. A commercially available preservative for inhibiting bacterial growth that comprises isothiazoline compounds is ProClin300. As is demonstrated by the examples, it is suitable for use in the stabilization of urine. It may be used in a final concentration of e.g. 0.03% to 0.1% (v/v) such as 0.05% (v/v) in the stabilized urine sample.

Further examples of preservatives that can be used include but are not limited to chlorhexidine or sodium borate. Further preservatives for inhibiting bacterial growth and useful concentrations can be determined by the skilled person following the disclosure of the present application.

According to one embodiment, the preservative for inhibiting bacterial growth is comprised in the stabilizing composition that is contacted with the urine sample for stabilization. Preferably, the stabilizing composition according to the third aspect is used for this purpose.

### pH

As is demonstrated in the examples, the pH influences the stabilization result. The stabilizing composition may be used to adjust the pH in the stabilized urine sample. It can thereby be achieved that the pH of the stabilized urine sample lies in a certain pH range. For this purpose, the stabilizing composition that is contacted for stabilization with the urine sample preferably comprises a buffering agent.

The stabilizing composition that is contacted with the urine sample has a pH that lies in the range of > 7 to ≤ 9.5. The pH may be in the range of 7.2 to 9.5, 7.3 to 9.5 or 7.4 to 9.5. The pH of the stabilizing composition may also be ≤ 9.25, ≤ 9 or ≤ 8.75, such as ≤ 8.5, ≤ 8.3 or ≤ 8. These pH values may be combined as upper value with the pH ranges indicated before and the resulting ranges also form part of the present disclosure. In one embodiment, the pH of the stabilizing composition is in the range of 7.5 to ≤ 9.5, such as 7.5 to 9 or 7.5 to 8.5. In one embodiment, the pH of the stabilizing composition is in the range of 7.2 ≤ 8.5, such as 7.3 to ≤ 8.3 or 7.5 to ≤ 8. The pH of the stabilizing composition may furthermore be in the range of 7.7 to 9.5 or 8 to 9.25. According to a further embodiment also shown in the examples, the pH of the stabilizing composition is in the basic range of 8 to 9.

According to one embodiment, said stabilizing composition is a liquid composition that is contacted with the urine sample in a volumetric ratio selected from 1:3 to 1:10, preferably 1:5 to 1:10. It is a particular advantage that stabilization of a large urine volume and pH adjustment can be achieved with a small volume of the stabilizing composition according to the present invention. Preferably, the ratio of stabilizing composition to sample lies in a range from 1:5 to 1:8, such as about 1:6. E.g. 1-2ml of a liquid stabilizing composition, such as 1.3ml to 1.7ml, may be contacted with approx. 10ml urine.

As is demonstrated by the examples, a stabilizing composition having a higher pH value of preferably above 7 is more efficient in preserving the cell-free DNA, because degradation of cell-free DNA is more efficiently prevented compared to a stabilizing composition having an acidic pH. This is highly advantageous when intending to analyse cell-free DNA comprised in the urine sample. As can be seen from the examples a higher pH in the stabilizing composition, such as in particular a basic pH above 7, efficiently prevents cell-free DNA degradation even when stabilized urine samples are stored for several days.

The stabilizing composition preferably comprises a buffering agent for pH adjustment. The buffering agent may have its pK in the basic range. A suitable and preferred buffering agent is Tris as is demonstrated by the examples. Other buffering agents include but are not limited to MOPS, TES, HEPES, TAPSO, Tricine, Bicine, TAPS, borate buffer, CHES buffer or buffers on the basis of sodium or potassium phosphate. A further buffering agent comprises carbonate. The pH of the urine may vary from acidic to basic from donor to donor and additionally depending on the donors conditions. It is thus advantageous to comprise the buffering agent in a sufficient concentration to achieve an efficient pH adjustment and stabilization despite potential variations in the pH of the collected urine sample to be stabilized. Suitable concentrations can be determined by the skilled person.

According to one embodiment, the final pH of the stabilized urine sample lies in a range of 6 to 9.5, such as 6.3 to 9.5. The final pH of the stabilized urine sample may be ≤9, such as ≤8.75 or ≤8.5. Particularly suitable is a pH in the stabilized urine sample that lies in a range of 6.5 to 9, such as 6.5 to 8.75 or 6.5 to 8.5. In embodiments, the pH of the stabilized urine sample may lie in a range of 6.7 to 9 or 7 to 9, such as 7.2 to 9 or 7.2 to 8.75. According to one embodiment, the final pH of the stabilized urine sample is at least 7 or above 7 and may e.g. lie in a range of 7.5 to 8.5.

### Caspase inhibitor

According to one embodiment, the urine sample is furthermore contacted with at least one caspase inhibitor. It is also within the scope of the present invention to use a combination of different caspase inhibitors. The use of a caspase inhibitor may improve the achieved stabilization effect. The caspase inhibitor may be comprised in the stabilizing composition that is contacted with the urine sample for stabilization. Preferably, the stabilizing composition according to the third aspect is used for this purpose.

Preferably, the caspase inhibitor is cell-permeable. Members of the caspase gene family play a significant role in apoptosis. The substrate preferences or specificities of individual caspases have been exploited for the development of peptides that successfully compete caspase binding. It is possible to generate reversible or irreversible inhibitors of caspase activation by coupling caspase-specific peptides to e.g. aldehyde, nitrile or ketone compounds. E.g. fluoromethyl ketone (FMK) derivatized peptides such as Z-VAD-FMK act as effective irreversible inhibitors with no added cytotoxic effects. Inhibitors synthesized with a benzyloxycarbonyl group (BOC) at the N-terminus and O-methyl side chains exhibit enhanced cellular permability. Further suitable caspase inhibitors are synthesized with a phenoxy group at the C-terminus. An example is Q-VD-OPh which is a cell permeable, irreversible broad-spectrum caspase inhibitor that is even more effective in preventing apoptosis and thus supporting the stabilization than the caspase inhibitor Z-VAD-FMK.

According to one embodiment, the caspase inhibitor is a pancaspase inhibitor and thus is a broad spectrum caspase inhibitor.

According to one embodiment, the caspase inhibitor is a caspase-specific peptide. Preferably, said caspase-specific peptide is modified. It may be modified by an aldehyde, nitrile or ketone compound. According to one embodiment, the caspase specific peptide is modified, preferably at the carboxyl terminus, with an O-Phenoxy (OPh) or a fluoromethyl ketone (FMK) group. According to one embodiment, the caspase inhibitor is selected from the group consisting of Q-VD-OPh and Z-VAD(OMe)-FMK. In a preferred embodiment, Q-VD-OPh, which is a broad spectrum inhibitor for caspases, is used for stabilization. Q-VD-OPh is cell permeable and inhibits cell death by apoptosis. Q-VD-OPh is not toxic to cells even at extremely high concentrations and comprises a carboxy terminal phenoxy group conjugated to the amino acids valine and aspartate. It is equally effective in preventing apoptosis mediated by the three major apoptotic pathways, caspase-9 and caspase-3, caspase-8 and caspase-10, and caspase-12 (Caserta et al, 2003). Examples of caspase inhibitors are also listed in Table 1 of WO 2013/045457.

The stabilized urine sample, i.e. the mixture that is obtained after contacting the urine sample with the caspase inhibitor and the further additives may comprise the caspase inhibitor (or combination of caspase inhibitors) in a final concentration of at least 0.5µM. Embodiments include a concentration of at least 1µM, e.g. at least 1.5µM, at least 2µM or at least 2.5µM.

Suitable final concentration ranges for the caspase inhibitor(s) when mixed with the urine sample and the further additives include but are not limited 1µM to 25µM. Embodiments include 1.5µM to 20µM and 2µM to 15µM. Preferred are final concentration ranges of 2µM to 12.5µM, 2.5µM to 10µM and 3µM to 7.5µM. The above mentioned concentrations apply to the use of a single caspase inhibitor as well as to the use of a combination of caspase inhibitors. The aforementioned concentrations are in particular suitable when using a pancaspase inhibitor, in particular a modified caspase specific peptide such as Q-VD-OPh and/or Z-VAD(OMe)-FMK. Suitable concentration ranges for individual caspase inhibitors and/or for other urine samples can be determined by the skilled person, e.g. by testing different concentrations of the respective caspase inhibitor in the test assays described in the examples.

### Amide

According to one embodiment, the urine sample is additionally contacted with one or more primary, secondary or tertiary amide as further stabilizing agent. Also combinations of two or more primary, secondary or tertiary amides can be used. The amide preferably is a carboxylic acid amide.

Suitable concentrations for different primary, secondary or tertiary amides can be determined by the skilled person, e.g. by testing different concentrations in the test assays described in the examples. Generally, the mixture that is obtained when contacting the urine sample with the one or more primary, secondary or tertiary amides and the further additives may comprise said amide (or combination of amides) in a final concentration of at least 0.05%. E.g. the final concentration may be at least 0.1%, at least 0.25%, at least 0.5% or at least 0.75%. Suitable concentration ranges include but are not limited to 0.1% to 10%, 0.25% to 7.5%, 0.3% to 5%, 0.4% to 3%, 0.5% to 2%, 0.6% to 1.8% or 0.75% to 1.5%. Concentrations or concentration ranges indicated in percentage values as used herein are in particular given as percentage weight per volume (w/v) for solid amides and as percentage volume per volume (v/v) for liquid amides.

According to one embodiment, said amide(s) is/are comprised in the stabilizing composition that is contacted with the urine sample for stabilization. Preferably, the stabilizing composition according to the third aspect is used for this purpose.

According to one embodiment, the primary, secondary or tertiary amide is a compound according to formula 1 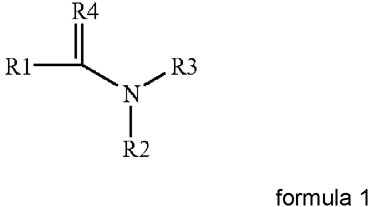 wherein R1 is a hydrogen residue or an alkyl residue, preferably a C1-C5 alkyl residue, a C1-C4 alkyl residue or a C1-C3 alkyl residue, more preferred a C1-C2 alkyl residue, R2 and R3 are identical or different and are selected from a hydrogen residue and a hydrocarbon residue, preferably an alkyl residue, with a length of the carbon chain of 1 - 20 atoms arranged in a linear or branched manner, and R4 is an oxygen, sulphur or selenium residue, preferably R4 is oxygen.

Also a combination of one or more compounds according to formula 1 can be used in addition for urine stabilization.

In embodiments, wherein R1 is an alkyl residue, a chain length of 1 or 2 is preferred for R1.

R2 and/or R3 of the compound according to formula 1 are identical or different and are selected from a hydrogen residue and a hydrocarbon residue, which preferably is an alkyl residue. According to one embodiment, R2 and R3 are both hydrogen. According to one embodiment, one of R2 and R3 is a hydrogen and the other is a hydrocarbon residue. According to one embodiment, R2 and R3 are identical or different hydrocarbon residues. The hydrocarbon residues R2 and/or R3 can be selected independently of one another from the group comprising alkyl, including short chain alkyl and long-chain alkyl, alkenyl, alkoxy, long-chain alkoxy, cycloalkyl, aryl, haloalkyl, alkylsilyl, alkylsilyloxy, alkylene, alkenediyl, arylene, carboxylates and carbonyl. The chain length n of R2 and/or R3 can in particular have the values 1, 2, 3, 4, 5, 6, 7, 8, 9, 10, 11, 12, 13, 14, 15, 16, 17, 18, 19 and 20. According to one embodiment, R2 and R3 have a length of the carbon chain of 1-10, preferably 1 to 5, more preferred 1 to 2. According to one embodiment, R2 and/or R3 are alkyl residues, preferably C1-C5 alkyl residues.

Preferably, the compound according to formula 1 is a carboxylic acid amide so that R4 is oxygen. It can be a primary, secondary or tertiary carboxylic acid amide.

According to one embodiment, the compound according to formula 1 is a N,N-dialkylcarboxylic acid amide. Preferred R1, R2, R3 and R4 groups are described above. Using a respective compound according to formula 1 may support the stabilization of intracellular nucleic acids in the urine sample. The additional stabilization of intracellular nucleic acids is advantageous as it e.g. allows the subsequent analysis of interacellular nucleic acids comprised in the stabilized cells. According to one embodiment, the compound according to formula 1 is selected from the group consisting of N,N-dimethylacetamide, N,N-diethylacetamide, N,N-dimethylformamide and N,N-diethylformamide. Also suitable are the respective thio analogues, which comprise sulphur instead of oxygen as R4. N,N-dimethlylacetamide (DMAA) e.g. achieves good stabilization results, however, is a toxic agent. Preferably, at least one compound according to formula 1 is used in combination with the poly(oxyethylene) polymer for stabilizing the urine sample which is not a toxic agent according to the GHS classification.

According to one embodiment, the urine sample is additionally contacted for stabilization with a compound according to formula 1 which is a N,N-dialkylpropanamide, such as N,N-dimethylpropanamide. As is demonstrated in the examples, a respective combination is suitable for stabilizing cell-free DNA in urine.

According to one embodiment, the urine sample is additionally contacted with at least one compound according to formula 1, which is a primary or secondary carboxylic acid amide. As can be seen from WO 2014/146781, primary and secondary carboxylic acid amides are capable of stabilizing the extracellular nucleic acid population over a broad concentration range. According to one embodiment, the primary carboxylic acid amide is selected from the group consisting of formamide, acetamide, propanamide and butanamide. According to one embodiment, the primary carboxylic acid is butanamide. The stabilization effect of butanamide on the extracellular nucleic acid population is also described in detail in WO 2014/146780 and WO 2014/146782.

According to one embodiment, the urine sample is contacted for stabilization with EDTA, the poly(oxyethylene) polymer, which preferably is a polyethylene glycol, the preservative for inhibiting bacterial growth and butanamide and/or an N,N-dialkylpropanamide, wherein said N,N-dialkylpropanamide preferably is N,N-dimethylpropanamide. Suitable and preferred concentrations and concentration ranges described above in general for the primary, secondary and tertiary amides in general also specifically apply to this embodiment. As is shown by the examples, using a stabilizing composition comprising butanamide and/or a N,N-dialkylpropanamide in a concentration that lies in these ranges provides an advantageous stabilizing effect on a urine sample.

### Extracellular (cell-free) DNA

As is demonstrated in the examples, the stabilization method according to the present invention efficiently stabilizes extracellular (cell-free) DNA which is particularly valuable for diagnostic purposes. It is therefore important to efficiently inhibit degradation of the comprised cell-free DNA in the urine sample.

The stabilization technology according to the present invention is of particular advantage in this respect because it substantially preserves the extracellular DNA comprised in the urine sample and e.g. inhibits degradation of the comprised extracellular DNA (preferably at least by 50%, at least by 60% or at least by 70% over the stabilization period compared to timepoint 0, directly after stabilization).

A further issue after collecting the urine sample may lie in the contamination and dilution of the cell-free DNA comprised in the collected urine sample by intracellular nucleic acids, in particular fragmented genomic DNA, that originates from damaged or dying cells that are contained in the urine sample. According to one embodiment, the stabilization method reduces the increase of DNA that results from a release of genomic DNA from cells contained in the urine sample during the stabilization period compared to a non-stabilized sample. The release of DNA can be determined e.g. by quantifying the ribosomal 18S DNA as is described herein in the example section. As is shown in the examples, the stabilization achievable with the teachings of the present invention may reduce this release of DNA over the stabilization period. However, as indicated above, with urine samples the degradation of the cell-free DNA during storage is more problematic so that the priority resides in preventing the degradation and thus loss of the cell-free DNA that was present at the timepoint of collection during storage. As is shown by the examples, an effective stabilization of cell-free DNA is achievable with the method of the invention for a period of at least three days and even up to 6 days and longer. However, the samples may also be further processed earlier, if desired. It is not necessary to make use of the full achievable stabilization period.

Furthermore, the examples demonstrate that cells are stabilized and suitable for cytological analyses. Furthermore, genomic DNA can be isolated from the comprised cells.

In addition, nucleic acids can be efficiently isolated from respectively stabilized samples using different standard methods as no cross-linking of the sample occurs due to the stabilization. This greatly simplifies and improves the standardization of molecular analysis that relies on the analysis of nucleic acids obtained from the stabilized urine sample.

### Further additives

Additional additives can also be used in order to further support the stabilization of the urine sample, respectively support the preservation of the extracellular nucleic acid population. Examples of respective additives include but are not limited to nuclease inhibitors, in particular RNase and DNase inhibiting compounds. As is shown in the examples, one may additionally include diethylene glycol methyl ether acetate (DEGMEA).

When choosing a respective further additive for supporting stabilization, care should be taken not to compromise and/or counteract the stabilizing effect of the core stabilizing agents. Thus, no additives such as e.g. chaotropic agents should be used in concentrations that result in or support the lysis and/or degradation of nucleated cells contained in the urine sample that is stabilized and/or which support the degradation of the cell-free DNA contained in the cell-free fraction of the urine sample. Therefore, preferably, the stabilization method described herein does not involve the use of further additives (i) that induce or promote lysis of nucleated cells, (ii) that induce or promote lysis of cells in general and/or (iii) that lead to a degradation of nucleic acids contained in the cell-free fraction of the urine sample, such as in particular cell-free DNA. As the stabilization method described herein is not based on cell lysis but allows to also preserve cells, cells can be separated from the urine sample after the stabilization period, thereby allowing to obtain a cell-free or cell-depleted urine fraction which comprises the extracellular DNA and a cell sample. The extracellular DNA population preferably substantially corresponds to or at least closely resembles the extracellular DNA population present at the time of urine collection and stabilization. Furthermore, intracellular nucleic acids such as genomic DNA can be isolated from the separated cells and are available for analysis. Hence, respectively stabilized samples allow, if desired, the separate analysis of extracellular and intracellular nucleic acids, such as cell-free DNA and genomic DNA, from the same stabilized sample. Furthermore, the cells as such can be analysed by cytological methods as demonstrated in the examples.

### Further embodiments

Further advantageous embodiments of the method according to the first aspect are described subsequently.

According to one embodiment, a method for stabilizing a urine sample according to claim 1 is provided and which comprises contacting the urine sample for stabilization with a stabilization composition comprising
- EDTA as chelating agent,
- at least one polyethylene glycol, preferably at least one high molecular weight polyethylene glycol having a molecular weight of at least 3000 and/or at least one low molecular weight polyethylene glycol having a molecular weight of 1000 or less,
- a preservative for inhibiting bacterial growth,
- a buffering agent,
- at least one caspase inhibitor, and
- optionally at least one primary, secondary or tertiary amide.

Also provided is a method for stabilizing a urine sample according to claim 1 and which comprises contacting the urine sample for stabilization with a stabilization composition comprising
- EDTA as chelating agent, wherein in the stabilized urine sample, the final concentration of EDTA lies in a range of 7mg/ml to 40 mg/ml, preferably 10mg/ml to 30mg/ml, such as 15mg/ml to 30mg/ml;
- at least one polyethylene glycol, preferably at least one high molecular weight polyethylene glycol having a molecular weight of at least 6000 and/or at least one low molecular weight polyethylene glycol having a molecular weight of 1000 or less,
- a preservative for inhibiting bacterial growth,
- a buffering agent,
- at least one caspase inhibitor, and
- optionally at least one primary, secondary or tertiary amide.

Also provided is a method for stabilizing a urine sample according to claim 1 and which comprises contacting the urine sample for stabilization with a liquid stabilizing composition which comprises
- EDTA in a concentration of at least 100mM up to the solubility limit, preferably in a concentration that lies in a range of 150mM to 500mM, wherein in the stabilized urine sample, the final concentration of EDTA lies in a range of 7mg/ml to 40 mg/ml, preferably 10mg/ml to 30mg/ml, such as 15mg/ml to 30mg/ml;
- at least one polyethylene glycol, preferably at least one high molecular weight polyethylene glycol having a molecular weight of at least 6000 and optionally at least one low molecular weight polyethylene glycol having a molecular weight of 1000 or less,
- a preservative for inhibiting bacterial growth,
- a buffering agent, and
- optionally at least one caspase inhibitor,

wherein the pH of the stabilizing composition lies in a range of > 7 to 9.5, such as 7.2 to 9, 7.2 to 8.75 or 7.3 to 8.5,
wherein the stabilizing composition is contacted for stabilization with the urine sample in a volumetric ratio selected from 1:3 to 1:10, preferably 1:5 to 1:8. The final pH in the stabilized urine sample may lie in a range of 6 to 9.5, such as 6.3 to 9.5 or 6.5 to 9. As is shown by the examples, using an according stabilization composition inter alia allows to provide a final pH in the stabilized urine sample that lies in a range of 6.5 to 8.75, such as 6.5 to 8.5. Good stabilizing effects were achieved. The final pH in the stabilized urine sample may also be above 7 is shown by the examples. Suitable embodiments and concentrations for the polyethylene glycol, the preservative and the buffering agent were disclosed above. The stabilizing composition may also comprise at least one primary, secondary or tertiary amide as disclosed herein.

The chelating agent, the poly(oxyethylene) polymer and optionally the preservative for inhibiting bacterial growth (core stabilizing agents) as well as the further stabilizing agents and/or additives that can be optionally used, such as in particular the buffering agent and the caspase inhibitor, can be present in a device, preferably a container, for receiving a urine sample. The core stabilizing agents and optionally the one or more compounds additionally used for stabilization can be present in a stabilizing composition, such as the stabilizing composition according to the third aspect, that is present in a respective device or can be present as separate entities. Furthermore, they can be added to a respective reception device prior to receiving the urine sample, or can be added to the reception device after the urine sample was received therein. It is also disclosed to add the stabilizing agents and optionally, further additive(s) used separately to the urine sample fo stabilization. However, for the ease of handling, it is preferred that the core stabilizing agents and any further stabilizing agents and/or additives used are provided in the respective reception device, e.g. in form of a single composition. However, they may also be present as separate components or compositions in the reception device. In an advantageous embodiment, the core stabilizing agents, at least one caspase inhibitor and optionally further additive(s), are present in the reception device prior to adding the urine sample. This ensures that the urine sample is rapidly stabilized upon contact with the stabilizing agents used according to the teachings of the present invention. The stabilization agents are present in the container in an amount effective to stabilize the amount of urine sample to be collected, respectively contained in said reception device. Suitable and preferred embodiments for a respective reception device are also described subsequently in conjunction with the fifth embodiment and it is referred to said disclosure. As is described herein, the urine may first be collected in a standard urine collection device and then transferred into the reception device of the present invention for stabilization of the urine.

Preferably, the urine sample is contacted with the core stabilizing agents and optionally further additives directly after the collection of the urine sample. In embodiments, contacting occurs during urine collection from the donor, e.g. by providing the stabilizing agents in a collection container such as a urine cup. As described above, preferably, the agents used for stabilization are provided in form of a stabilizing composition according to the third aspect. Preferably, said stabilizing composition is provided in a liquid form. It can be e.g. pre-filled in a sample reception device so that the urine sample is rapidly stabilized when entering the device.

Using the stabilizing method according to the present invention has the advantage that substantially irrespective of the composition of the urine sample and the number of cells contained therein, the extracellular DNA as well as exfoliated cells and intracellular nucleic acids such as genomic DNA contained in said sample can be substantially preserved, respectively stabilized, thereby allowing for standardizing the subsequent isolation and/or analysis of contained cell-free DNA, and other stabilized components. In embodiments, the stabilization method achieves a stabilization of the cells contained in the urine sample so that the cells are available for the isolation of intracellular nucleic acids such as genomic DNA and/or cytohistological analyses.

According to one embodiment, extracellular RNA is stabilized in addition to extracellular DNA.

The term "extracellular nucleic acids" or "extracellular nucleic acid" as used herein, in particular refers to nucleic acids that are not contained in cells. Respective extracellular nucleic acids are also often referred to as cell-free nucleic acids. These terms are used as synonyms herein. Hence, extracellular nucleic acids usually are present exterior of a cell or exterior of a plurality of cells within a sample. The term "extracellular nucleic acids" refers e.g. to extracellular DNA and extracellular RNA. Cell-free DNA in urine is of specific interest. Examples of typical extracellular nucleic acids that are found in the cell-free fraction (respectively portion) of urine include but are not limited to mammalian extracellular nucleic acids such as e.g. extracellular tumor-associated or tumor-derived DNA and/or RNA, other extracellular disease-related DNA and/or RNA, epigenetically modified DNA, fetal DNA and non-mammalian extracellular nucleic acids such as e.g. viral nucleic acids, pathogen nucleic acids released into the extracellular nucleic acid population e.g. from prokaryotes (e.g. bacteria), viruses, eukaryotic parasites or fungi. The extracellular nucleic acid population usually comprises certain amounts of intracellular nucleic acids that were released from damaged or dying cells either prior or during collection of the urine sample.

According to one embodiment, the term extracellular nucleic acids or extracellular DNA in particular refers to mammalian extracellular nucleic acids respectively mammalian extracellular DNA. Examples include but are not limited to disease-associated or diseasederived extracellular nucleic acids such e.g. as tumor-associated or tumor-derived extracellular nucleic acids, extracellular nucleic acids released due to inflammations or injuries, in particular traumata, extracellular nucleic acids related to and/or released due to other diseases, or extracellular nucleic acids derived from a fetus.

The term "extracellular nucleic acid population" or "extracellular DNA population" as used herein in particular refers to the collective of different extracellular nucleic acids such as DNA that are comprised in the urine sample. Of specific interest is the extracellular DNA population. A urine sample usually comprises a characteristic and thus unique extracellular nucleic acid population. Thus, the type, kind, ratio and/or the amount of one or more extracellular nucleic acids comprised in the extracellular nucleic acid population of a urine sample may be important sample characteristics. As discussed above, it is therefore advantageous to stabilize and thus to substantially preserve said extracellular DNA population at the state wherein the urine sample is collected, as the comprised type, composition and/or the amount of one or more extracellular nucleic acids comprised in the extracellular nucleic acid population of a sample can provide valuable medical, prognostic or diagnostic information. Therefore, it is advantageous if the profile of the extracellular nucleic acid population, e.g. of the cell-free DNA, is efficiently stabilized over the intended stabilization period. The stabilization technologies described herein reduces the degradation of the comprised cell-free DNA. Thus, a substantial preservation of the extracellular DNA population is achieved. In embodiments the stabilization additionally reduces contaminations and hence a dilution of the extracellular nucleic acids by intracellular nucleic acids, in particular by genomic DNA, after urine sample collection and stabilization. In embodiments, the release of genomic DNA from cells contained in the sample into the cell-free portion of the urine sample is reduced due to the stabilization.

The stabilization method described herein in particular reduces the risk that extracellular DNA is rapidly degraded in the collected urine sample. A degradation of cell-free DNA in the urine sample is reduced due to the stabilization. It is therefore particularly advantageous to directly transfer the collected urine sample into a reception device that already comprises the stabilizing composition thereby stabilizing the urine sample directly upon collection. This reduces alterations of the extracellular DNA population because of the *ex vivo* handling. If the urine is first collected and handled before it is stabilized, the risk increases that extracellular DNA is rapidly degraded in the intermediate time period.

As is described above and as is demonstrated by the examples, using the methods of the present invention allows for stabilizing the urine sample without refrigeration or freezing for a prolonged time period. Thus, the samples can be kept at room temperature or even at elevated temperatures e.g. up to 30°C or even up to 40°C. According to one embodiment, a stabilization effect is achieved for at least three days, preferably at least four days, more preferred at least 6 days or up to 9 days.

Generally, it may occur though that the stabilization effect may decrease over time, which may also depend on the source, e. g. the donor from which the urine sample is derived, it generally will be still sufficient to preserve the urine sample. Thus, urine samples that were stabilized according to the methods of the present invention were still suitable for isolating and analysing the extracellular DNA contained therein even after prolonged storage at room temperature. Thus, even longer storage/shipping times are conceivable. However, usually, longer periods are not necessary, as the regular storage and e.g. shipping time to the laboratory, wherein the nucleic acid isolation and optionally further analysis is performed, usually does not exceed 3, 6 or 7 days. As is shown in the examples, a substantial stabilization effect is achieved over this time period. The long stabilization times and stabilization efficiencies that are achievable with the method according to the present invention provides an important safety factor.

The method and also the subsequently described stabilizing composition allow the stabilization of a large volume of a urine sample with a small volume/amount of added stabilizing agents because the core stabilizing agents and the described combinations of stabilizing agents used are highly active in particular in combination. This is an important advantage because the volume of the urine sample poses considerable restrains on the subsequent nucleic acid isolation procedure in particular when intending to use automated processes for isolating the extracellular nucleic acids such as cell-free DNA contained in the samples. Furthermore, one has to consider that extracellular nucleic acids are usually only comprised in small amounts in the urine sample. Thus, processing larger volumes of a urine sample has the advantage that more extracellular nucleic acids can be isolated from the sample and thus are available for a subsequent analysis.

According to one embodiment, the urine volume that is stabilized using the method of the first aspect is selected from 1ml to 150ml, 2ml to 100ml, 2.5ml to 75ml or 3ml to 50ml. For many analytical purposes, the stabilization of a lower urine volume is sufficient and the use of an evacuated reception device such as a tube comprising the stabilizing agents for performing the stabilization advantageous. The urine may be e.g. transferred from the original urine collection container (which may receive a larger volume up to e.g. 200ml) into a reception device, such as an evacuated tube, which comprises the stabilizing agents, preferably the stabilizing composition according to the third aspect. According to one embodiment, the reception device comprising the stabilizing agents may receive e.g. 2ml to 50ml, 3ml to 30ml, 4ml to 25ml, or 5ml to 20ml, e.g. up to 10ml, urine.

The method may have one or more of the following characteristics:
i) the stabilization does not involve the use of additives in a concentration wherein said additives would induce or promote lysis of nucleated cells;
   and/or
ii) the stabilized sample is retained for at least 1 day or at least 2 days prior to processing the stabilized sample.

The stabilization method as disclosed herein provides a significant advantage over state-of-the-art stabilization methods that are used for stabilizing the extracellular nucleic acid population in a urine sample which are based on the use of cross-linking reagents, such as formaldehyde, formaline, formaldehyde releasers and the like (see e.g. Streck). Crosslinking reagents cause inter- or intra-molecular covalent bonds between nucleic acid molecules or between nucleic acids and proteins. This cross-linking effect can significantly impair the subsequent isolation of nucleic acids from such stabilized samples and usually requires specifically adapted nucleic acid isolation procedures that allow the isolation from such samples. As, for example, the concentration of cell-free DNA or a target sequence in a urine sample may be already relatively low, any measure which further reduces the yield of such nucleic acids should be avoided. This may be of particular importance when detecting and analyzing very rare nucleic acid molecules derived e.g. from malignant tumors or from a developing fetus. As is shown by the examples, the urine stabilization method of the invention does not require cross-linking agents for stabilization. The stabilization according to the present invention does not involve the use of a cross-linking agent that induces protein-nucleic acid and/or protein-protein crosslinks such as formaldehyde, formaline, paraformaldehyde or a formaldehyde releaser.

According to a preferred embodiment, the urine sample, which may have been first collected in a different device, is subsequently contacted for stabilization exclusively with a stabilizing composition comprising the stabilizing agents, such as the stabilizing composition according to the third aspect. Thereby, the stabilizing conditions are established by the stabilizing composition.

After the stabilization period, the method may comprise one or more of the following: Extracellular nucleic acids, preferably cell-free DNA, is/are isolated from the stabilized urine sample, and optionally further analysed and/or detected. Cells comprised in the stabilized urine sample may be removed. According to one embodiment, cells comprised in the stabilized urine sample are removed and cell-free DNA is isolated from the cell-free or cell-depleted portion of the stabilized sample. According to one embodiment, the removed cells are discarded. However, the present method may also comprise removing cells from the stabilized urine sample and isolating nucleic acids, preferably genomic DNA, from cells that were removed from the stabilized urine sample. In addition, the method may comprise removing cells from the stabilized urine sample and analyzing removed cells using a cytological method.

According to one embodiment, (i) the stabilized urine sample, (ii) the stabilized urine sample from which cells have been removed and/or (iii) cells removed from the stabilized urine sample are stored.

Hence, the stabilized urine sample can be analysed in a nucleic acid analytic and/or detection method and/or may be further processed and the present method enables numerous analytical options for analysing the stabilized cell-free DNA, cells and/or intracellular nucleic acids. Details regarding the nucleic acid isolation and analysis are also described below in conjunction with the second aspect of the present invention and it is referred to said disclosure.

### B. METHOD FOR PROCESSING A URINE SAMPLE

According to a second aspect, a method for processing a urine sample is provided, comprising the steps of
a) stabilizing the urine sample according to the method defined in the first aspect; and
b) separating cells from the stabilized urine sample, thereby providing (i) a cell sample and (ii) a cell-depleted supernatant.

Details of the method are also defined in claim 15. The individual steps are described in further detail in the following.

### Step a)

In step a), the urine sample is stabilized according to the method described in the first aspect of the present invention as described in detail above, in particular using the method according to any one of claims 1 to 14. As discussed above, the stabilization according to the present invention has the effect that cell-free DNA is substantially preserved from degradation over the stabilization period. Furthermore, cells as well as intracellular nucleic acids such as genomic DNA are preserved as is demonstrated in the examples.

The stabilization according to the present invention does not require and preferably does not involve the use of cross-linking agents. As claimed, the stabilization does not involve the use of a cross-linking agent that induces protein-nucleic acid and/or protein-protein crosslinks such as formaldehyde, formaline, paraformaldehyde or a formaldehyde releaser. This is an important advantage over prior art methods which involve the use of cross-linking agents such as formaldehyde, formaline or formaldehyde releasers, because these reagents often reduce the recoverable amount of extracellular nucleic acids due to cross-linking when using standard nucleic acid isolation techniques. Furthermore, as described above, the stabilization described herein allows the sample to be stored and/or handled, e.g. transported - even at room temperature - for a prolonged period of time prior to separating the cells contained in the urine sample in step b).

With respect to the details of the stabilization that is performed in step a), it is referred to the above disclosure which also applies here. Preferably, the urine sample is contacted for stabilization with a stabilizing composition according to the third aspect.

### Step b)

In step b), cells are separated from the stabilized urine sample, thereby providing (i) a cell sample and (ii) a cell-depleted supernatant.

Cells can be separated and thus removed e.g. by centrifugation, preferably high speed centrifugation, or by using means other than centrifugation, such as e.g. filtration, sedimentation or binding to surfaces e.g. on (optionally magnetic) particles if a centrifugation step is to be avoided. Respective cell separation methods are well-known in the prior art and thus, do not need to be described in detail. Respective cell removal steps can also be easily included into an automated sample preparation protocol. Preferably, essentially all cells are removed to provide an essentially cell-free or cell-free supernatant.

The removed cells may also be processed further if desired as described below. The cells can e.g. be stored, analysed (e.g. using cytological methods) and/or biomolecules such as e.g. nucleic acids or proteins can be isolated from the removed cells.

According to one embodiment, step b) is performed within minutes or hours after stabilizing the urine sample. According to one embodiment, step b) is performed at least one day up to 3 days or two days up to 9 days after the urine sample has been collected, respectively was stabilized according to the teachings of the present invention.

### Further steps

Furthermore, it is also within the scope of the present invention to include further steps to work up the stabilized urine sample, the obtained cell sample and/or the cell-depleted supernatant.

According to one embodiment, the method further comprises purifying extracellular nucleic acids, such as preferably extracellular DNA, from the cell-depleted supernatant.

According to one embodiment, the method further comprises purifying intracellular nucleic acids, preferably genomic DNA, from the obtained cell sample. As is demonstrated in the examples, the present method allows the processing and analysis of extracellular and intracellular nucleic acids, e.g. extracellular DNA and genomic DNA, comprised in the stabilized urine sample. As is shown by the examples the extracellular DNA fraction may be analyzed separate from the genomic DNA.

According to one embodiment, nucleic acid isolation is performed within one day up to 3 days or within 2 days up to 9 days after the urine sample was collected and stabilized according to the method according to the present invention.

For isolating nucleic acids, any suitable nucleic acid isolation method can be used and suitable methods are well-known and commercially available. Examples for respective purification methods include but are not limited to extraction, solid-phase extraction, silicabased purification methods, magnetic particle-based purification, phenol-chloroform extraction, chromatography, anion-exchange chromatography (using anion-exchange surfaces), electrophoresis, filtration, precipitation and combinations thereof. It is also within the scope of the present invention to specifically isolate specific target extracellular nucleic acids, e.g. by using appropriate probes coupled to a solid support that enable a sequence specific binding. Also any other nucleic acid isolating technique known by the skilled person can be used.

According to one embodiment, nucleic acids are isolated from the cell-depleted supernatant using a chaotropic agent and/or alcohol. Preferably, the nucleic acids are isolated by binding them to a solid phase, preferably a solid phase comprising silica or carrying anion exchange functional groups. Respective methods are well-known in the prior art and thus, do not need any detailed description. Suitable methods and kits for isolating extracellular nucleic acids are also commercially available such as the QIAamp^{®} Circulating Nucleic Acid Kit (QIAGEN), the Chemagic Circulating NA Kit (Chemagen), the NucleoSpin Plasma XS Kit (Macherey-Nagel), the Plasma/Serum Circulating DNA Purification Kit (Norgen Biotek), the Plasma/Serum Circulating RNA Purification Kit (Norgen Biotek), the High Pure Viral Nucleic Acid Large Volume Kit (Roche) and other commercially available kits suitable for extracting and purifying extracellular nucleic acids.

According to one embodiment, nucleic acids are isolated by binding them to a solid phase comprising anion exchange groups. Suitable anion exchange groups are for example provided by amine groups. Binding preferably occurs at a pH below 7. Such anion exchange based nucleic acid isolation methods are known to the skilled person. According to one embodiment, the nucleic acids are extracellular nucleic acids. Suitable anion exchange based methods are e.g. described in WO 2013/045432.

According to one embodiment, total nucleic acids are isolated from the cell-depleted supernatant. It is also within the scope of the present invention to isolate at least predominantly a specific target nucleic acid. A target nucleic acid can be e.g. a certain type of extracellular nucleic acid, e.g. DNA or RNA (includes miRNA, snRNA, lincRNA), preferably DNA. E.g. the target extracellular nucleic acid can be DNA and the non-target extracellular nucleic acid can be RNA. Target specific nucleic acid isolation methods which specifically aim at isolating DNA or RNA are also well known in the prior art and thus, do not need any detailed description herein. According to one embodiment, the non-target nucleic acid is destroyed by adding an appropriate enzyme which specifically destroys the non-target nucleic acid, e.g. a RNase if the target nucleic acid is DNA or a DNase if the target nucleic acid is RNA. Said enzyme can be added to the lysis or binding mixture or can be added after extracellular nucleic acids were bound to a solid phase. Suitable embodiments for performing a respective non-target nucleic acid digestion step are known in the prior art and thus, do not need any further description herein. According to one embodiment which is feasible if DNA and RNA are bound to a solid support, elution conditions selective for the target nucleic acid can be applied to predominantly and thus selectively recover the target nucleic acid from the solid support. According to one embodiment, an isolation method is used, wherein the target nucleic acid, e.g. DNA, is selectively bound to a solid phase under conditions wherein non-target nucleic acids, e.g. RNA do not bind. Suitable binding conditions are well-known in the prior art and are e.g. described in WO 95/21849. Methods for selectively removing a non-target nucleic acid from a target nucleic acid are for example described in EP 0 880 537 and WO 95/21849. If desired, said non-target nucleic acid may also be further used, e.g. further processed such as e.g. eluted from the solid phase. However, it may also be discarded. It is also within the scope of the present invention to e.g. digest the non-target nucleic acid or remainders thereof using nucleases after isolation of the target nucleic acid. The term target nucleic acid may also refer to a specific kind of nucleic acid, e.g. a specific extracellular nucleic acid that is known to be a certain disease marker. As discussed above, the isolation of extracellular nucleic acids may also comprise the specific isolation of a respective target nucleic acid e.g. by using appropriate capture probes which support the selective isolation of the target nucleic acid.

The term target nucleic acid may also refer to nucleic acids having a certain length. Size selective nucleic acid, in particular DNA isolation methods, are known in the art. A classic method for isolating DNA of a target size involves the separation of the DNA in a gel, cutting out the desired gel band(s) and then isolating the DNA of the target size from the gel fragment(s). Another widely used technology is the size selective precipitation with polyethylene glycol based buffers (Lis and Schleif Nucleic Acids Res. 1975 Mar;2(3):383-9) or the binding/precipitation on carboxyl-functionalized beads (DeAngelis et al, Nuc. Acid. Res. 1995, Vol 23(22), 4742-3; US 5,898,071 und US 5,705,628, commercialized by Beckman-Coulter (AmPure XP; SPRIselect) and US 6,534,262). Furthermore, size selective isolation methods that are based on the use of solid supports comprising anion exchange groups and varying pH values are known. A size selective isolation provides further opportunities in order to reduce the amount of intracellular nucleic acids in the isolated extracellular nucleic acids. For example, when the target extracellular nucleic acid of interest is DNA, the removal of genomic DNA during nucleic acid isolation could also supplement or even replace a separate high g-force centrifugation before starting the nucleic acid extraction in order to remove residual cells. Genomic DNA that is released from said residual cells is prevented from becoming massively degraded due to the stabilization according to the present invention, in particular if a caspase inhibitor is additionally used, and accordingly, said unfragmented or less fragmented genomic DNA can be depleted by using a size-selective nucleic acid isolation protocol. This option is of particular advantage, as many clinical laboratories do not have a centrifuge capable of performing such a high g-force centrifugation or other means for removing in particular trace amounts of residual cells. Furthermore, an according size selective nucleic acid isolation protocol also allows to deplete during the nucleic isolation step intracellular DNA such as genomic DNA that might have been released during stabilization based on its larger size. This allows to subsequently purify the stabilized (shorter) extracellular DNA with less genomic DNA contaminations.

According to one embodiment, the nucleic acids isolated from (i) the cell sample and/or (ii) the cell-free supernatant can then be analysed and/or further processed, e.g. using a suitable assay and/or analytical methods. E.g. they can be identified, modified, contacted with at least one enzyme, amplified, reverse transcribed, cloned, sequenced, contacted with a probe, be detected (their presence or absence) and/or can be quantified. Respective methods are well-known in the prior art and are commonly applied in the medical, diagnostic and/or prognostic field in order to analyse extracellular nucleic acids such as cell-free DNA. Thus, after the extracellular nucleic acid of interest was isolated from the cell-free supernatant, optionally as part of total nucleic acids, such as total DNA and/or total RNA, it can be analysed e.g. to identify the presence, absence or severity of a disease state including but not being limited to a multitude of neoplastic diseases, in particular premalignancies and malignancies such as different forms of tumors or cancers. E.g. the isolated extracellular nucleic acids can be analysed in order to detect diagnostic and/or prognostic markers (e.g., fetal- or tumor-derived extracellular nucleic acids) in many fields of application, including but not limited to noninvasive prenatal genetic testing respectively screening, disease screening, pathogen screening, oncology, cancer screening, early stage cancer screening, cancer therapy monitoring, genetic testing (genotyping), infectious disease testing, injury diagnostics, trauma diagnostics, transplantation medicine or many other diseases and, hence, are of diagnostic and/or prognostic relevance. According to one embodiment, the isolated extracellular nucleic acids, such as cell-free DNA, are analyzed to identify and/or characterize a disease.

The analysis/further processing of the isolated nucleic acids can be performed using any nucleic acid analysis/processing method including, but not limited to amplification technologies, polymerase chain reaction (PCR), isothermal amplification, reverse transcription polymerase chain reaction (RT-PCR), quantitative real time polymerase chain reaction (Q-PCR), digital PCR, gel electrophoresis, capillary electrophoresis, mass spectrometry, fluorescence detection, ultraviolet spectrometry, hybridization assays, DNA or RNA sequencing, next generation sequencing, restriction analysis, reverse transcription, nucleic acid sequence based amplification (NASBA), allele specific polymerase chain reaction, polymerase cycling assembly (PCA), asymmetric polymerase chain reaction, linear after the exponential polymerase chain reaction (LATE-PCR), helicase-dependent amplification (HDA), hot-start polymerase chain reaction, intersequence-specific polymerase chain reaction (ISSR), inverse polymerase chain reaction, ligation mediated polymerase chain reaction, methylation specific polymerase chain reaction (MSP), multiplex polymerase chain reaction, nested polymerase chain reaction, solid phase polymerase chain reaction, or any combination thereof. Respective technologies are well-known to the skilled person and thus, do not need further description here.

According to one embodiment, cells comprised in the separated cell sample are analysed. According to one embodiment, cells are analysed by cytological methods. Methods include, but are not limited to centrifugation or smearing on slides followed by histochemical or cytochemical staining such as H&E, Periodic acid schiff (PAS), resorcin fuchsin (RF; elastin stain), sirius red (SR; collagen stain), Gomori (GOM; reticulin stain) and Papanicolaou staining procedure. Furthermore, advanced staining methods such a immunocytology staining or in situ hybridization (ISH) such as FISH (fluorescence), SISH (silver), CISH (chromogenic) may be used. In addition cells can be resuspended and sorted by fluorescence activated cell sorting (FACS) followed by molecular analyses. According to one embodiment, the method comprises preparing exfoliated cells for cytology from the obtained cell sample. As is demonstrated in the examples, the stabilization according to the present invention allows a subsequent cytological analysis of the stabilized cells.

### C. STABILIZING COMPOSITION

According to a third aspect, disclosed but not claimed as such is a composition for stabilizing a urine sample, in particular extracellular DNA comprised therein, wherein bacterial growth is inhibited in the stabilized urine sample, wherein the stabilizing composition has a pH in the range of > 7 to ≤ 9.5, and wherein the stabilizing composition does not comprise a crosslinking agent that induces protein-DNA and/or protein-protein crosslinks such as formaldehyde, formaline, paraformaldehyde or a formaldehyde releaser, the composition comprising
- a chelating agent, preferably EDTA,
- at least one poly(oxyethylene) polymer, preferably polyethylene glycol, and optionally
- a preservative for inhibiting bacterial growth.

The advantages and suitable and preferred embodiments of these core stabilizing compounds, as well as further stabilizing agents that may optionally be comprised in the stabilizing composition, such as a caspase inhibitor, a buffering agent and/or at least one primary, secondary or tertiary amide, are discussed above in conjunction with the stabilization method according to the first aspect and it is referred to the above disclosure which also applies here.

While the composition of the third aspect is not claimed as such, claimed in claim 18 is the composition mixed with a urine sample.

Suitable and preferred concentrations of the individual agents that can be used for stabilization in the stabilization mixture comprising the stabilizing composition and the urine sample were described above and also apply here. The skilled person can chose appropriate concentrations of said agents in the stabilizing composition to achieve said concentrations in the mixture comprising the urine sample when the intended amount of the stabilizing composition is mixed with the intended amount of urine sample to be stabilized following the teachings of the present disclosure. It is referred to the above disclosure which also applies here with respect to the stabilizing composition.

According to one embodiment, the stabilizing composition is a liquid. The components of the stabilizing composition can be comprised, respectively can be dissolved in a solvent, e.g. water, a buffer, e.g. a biological buffer such as TRIS, MOPS, PBS and the like. Suitable buffering agents are also listed above. Furthermore, the components may be dissolved in, or the stabilizing composition may comprise a polar aprotic solvent such as dimethyl sulfoxide (DMSO). E.g. a liquid stabilisation composition of 0.5ml to 2.5ml, 0.5ml to 2ml, preferably 1ml to 2ml such as 1.3 ml to 1.7ml can be used. Such stabilization composition comprising the stabilizing agents, in particular in the concentrations indicated below, can be used for stabilizing e.g. 10ml urine.

### pH

The pH of the stabilizing composition lies in the range of > 7 to ≤ 9.5. The pH may be in the range of 7.2 to 9.5, 7.3 to 9.5 or 7.4 to 9.5. The pH of the stabilizing composition may also be ≤ 9.25, ≤ 9 or ≤ 8.75, such as ≤ 8.5, ≤ 8.3 or ≤ 8. These pH values may be combined as upper value with the pH ranges indicated before and the resulting ranges also form part of the present disclosure. In one embodiment, the pH of the stabilizing composition is in the range of 7.5 to ≤ 9.5, such as 7.5 to 9 or 7.5 to 8.5. In one embodiment, the pH of the stabilizing composition is in the range of 7.2 ≤ 8.5, such as 7.3 to ≤ 8.3 or 7.5 to ≤ 8. The pH of the stabilizing composition may furthermore be in the range of 7.7 to 9.5 or 8 to 9.25.

According to a further embodiment also shown in the examples, the pH of the stabilizing composition is in the basic range of 8 to 9.

The stabilizing composition preferably comprises a suitable buffering agent that allows to maintain the pH within the desired range when the urine sample is added. Suitable and preferred embodiments for the buffering agent and the pH range in the stabilized urine sample have been described above in conjunction with the method according to the first aspect to which it is referred. As discussed, the chosen pH can have an important impact on the stabilization result.

### The chelating agent, preferably EDTA

The chelating agent that preferably is EDTA has been described in detail above and it is referred to the above disclosure. It may be comprised in the stabilising composition in a concentration up to the solubility limit.

According to one embodiment, a liquid composition is provided as stabilizing composition which comprises a chelating agent, preferably EDTA such as K₂EDTA, in a final concentration of at least 15mM, such as e.g. at least 25mM, at least 50mM, at least 75mM or at least 100mM. A high concentration of the chelating agent in the stabilizing composition is advantageous. According to one embodiment, the stabilizing composition comprises the chelating agent, preferably EDTA, in a concentration of 100mM up to the solubility limit or 150mM up to the solubility limit. Suitable final concentration ranges include 100mM to 500mM, 125mM to 450mM and 150mM to 400mM. Also higher concentrations may be used that lie in a range of 175mM to 400mM, 200mM to 400mM or 250mM to 350mM in the stabilizing composition. These concentrations are particularly suitable for EDTA.

### Poly(oxyethylene) polymer, preferably polyethylene glycol

The stabilization composition comprises a poly(oxyethylene) polymer, which preferably is a polyethylene glycol. Suitable embodiments were disclosed in conjunction with the first aspect and it is referred to this disclosure.

According to one embodiment a high molecular weight poly(oxyethylene) polymer is comprised in the stabilizing composition. Details are described above in conjunction with the stabilization method according to the first aspect and it is referred to the respective disclosure which also applies here. The high molecular weight poly(oxyethylene) polymer is preferably a polyethylene glycol, such as a unsubstituted polyethylene glycol. In embodiments, the molecular weight lies in a range selected of 1500 to 40000, e.g. 2000 to 35000, 2500 to 30000, 3000 to 25000 or 3500 to 20000. Preferred is a range of 4000 to 20000, such as 5000 to 15000.

According to one embodiment, the stabilization composition is a liquid and comprises a high molecular weight poly(oxyethylene) polymer which preferably is a polyethylene glycol in a final concentration of 0.4% to 35% (w/v). Exemplary ranges include, but are not limited to 0.8% to 25% (w/v), 1.5% to 20% (w/v), 2.5% to 17.5% (w/v), 3% to 15% (w/v), 4% to 10% (w/v) and 3% to 5% (w/v). Suitable concentrations can be determined by the skilled person based on the present disclosure and may *inter alia* depend on whether the high molecular weight poly(oxyethylene) polymer is used alone or in combination with a further poly(oxyethylene) polymer such as a low poly(oxyethylene) polymer and the amount, e.g. the volume, of the stabilization composition used to stabilize a certain amount of urine sample. Examples of concentration ranges suitable when using a high molecular weight poly(oxyethylene) polymer alone include but are not limited to 2.2% to 33.0% (w/v), such as e.g. 4.4% to 22.0 (w/v)%, 6.6% to 16.5% (w/v) or 8.8% to 13.2% (w/v). Examples of concentration ranges suitable when using a high molecular weight poly(oxyethylene) polymer in combination with a low molecular weight poly(oxyethylene) polymer include but are not limited to 0.4% to 30.7%(w/v), such as e.g. 0.8% to 15.3%(w/v), 1% to 10%(w/v), 1.5% to 7.7%(w/v), 2.5% to 6%(w/v), 3.1% to 5.4% (w/v) and 3% to 4%(w/v).

In a specific embodiment, the liquid stabilization composition comprises 5 mg to 500 mg, in particular 10 mg to 250 mg, 25 mg to 150 mg or 40 mg to 100 mg of the high molecular weight poly(oxyethylene) polymer which preferably is a polyethylene glycol. In particular, the liquid stabilization composition may comprise 0,5 µmol to 50 µmol, in particular 1 µmol to 25 µmol, 2 µmol to 20 µmol, or 3 µmol to 10 µmol of the high molecular weight poly(oxyethylene) polymer. Such liquid stabilization composition can be filled e.g. in a reception device and is e.g. suitable for stabilizing a sample unit urine.

According to one embodiment, the stabilization composition comprises a poly(oxyethylene) polymer that has a molecular weight of 1500 or less and in embodiments is a low molecular poly(oxyethylene) polymer having a molecular weight of 1000 or less. The low molecular weight poly(oxyethylene) polymer may have a molecular weight that lies in a range selected from 100 to 1000, e.g. 150 to 800 or 150 to 700. Preferred is a range of 200 to 600 and more preferably 200 to 500 such as 200 to 400. Preferably, polyethylene glycol is used.

According to one embodiment, said liquid stabilization composition comprises a low molecular weight poly(oxyethylene) polymer, which preferably is a polyethylene glycol, in a final concentration selected from 0.8% to 92.0%. Examples include 3.8% to 76.7%, 11.5% to 53.7%, 19.2% to 38.3% and 20% to 35%, such as 20% to 30%. The aforementioned concentrations refer to (w/v) or (v/v) depending on whether the low molecular weight poly(oxyethylene) polymer is a liquid or not.

In a specific embodiment, the liquid stabilization composition comprises 40 µl to 4000 µl, in particular 100 µl to 2000 µl, 150 µl to 1500 µl, 200µl to 1000µl or 250 µl to 750 µl of the low molecular weight poly(oxyethylene) polymer. In particular, the liquid stabilization composition may comprise 0.2 mmol to 15 mmol, in particular 0.5 mmol to 10 mmol, 0.75 mmol to 5 mmol, 1 mmol to 3 mmol, or 1.2 mmol to 2 mmol of the low molecular weight poly(oxyethylene) polymer. Such liquid stabilization composition can be filled e.g. in a reception device and is e.g. suitable for stabilizing a sample unit.

According to one embodiment, the stabilization composition comprises a high molecular weight poly(oxyethylene) polymer and additionally comprises at least one further poly(oxyethylene) polymer having a molecular weight that is at least 100, preferably at least 200, at least 300 or at least 400 below the molecular weight of the high molecular weight poly(oxyethylene) polymer. Embodiments are also described in conjunction with the method according to the first aspect. According to one embodiment, the difference in the molecular weight is at least 2500, at least 3500, at least 5000 or at least 7500. As described above in conjunction with the stabilization method according to the first aspect, using a combination of poly(oxyethylene) polymers that differ in their molecular weights is advantageous. Preferably, both poly(oxyethylene) polymers are polyethylene glycols such as unsubstituted polyethylene glycol. The stabilization composition may comprise a high molecular weight poly(oxyethylene) polymer having a molecular weight of at least 1500 and a low molecular weight poly(oxyethylene) polymer having a molecular weight of 1000 or less. The low molecular weight poly(oxyethylene) polymer is preferably a polyethylene glycol, such as a unsubstituted polyethylene glycol. The molecular weight of the low molecular weight poly(oxyethylene) polymer may lie in a range selected from 100 to 1000, 150 to 800 and preferably lies in the range of 200 to 600. Suitable concentration ranges for the high and low molecular weight poly(oxyethylene) polymer in the stabilization composition were described above.

### Preservative for inhibiting bacterial growth

The stabilization composition comprises in one embodiment a preservative for inhibiting bacterial growth. As disclosed above, this preservative may be used in addition to the chelating agent. Suitable and preferred embodiments were described above in conjunction with the method according to the first aspect and it is referred to the above disclosure which also applies here.

The preservative is comprised in a concentration in the stabilization composition so that bacterial growth is inhibited in the stabilized urine sample. Suitable concentrations can be determined by the skilled person for individual preservatives. The final concentration in the stabilization composition may lie e.g. in the range of 0.1 to 5.5% w/v, e.g. 0.2 to 3.75 % w/v or may lie in the range of 0.1 to 5.5% v/v, e.g. 0.2 to 3.75 % v/v.

According to one embodiment, the preservative may comprise or consist of sodium azide. As is demonstrated by the examples, sodium azide is particularly suitable. The stabilization composition may comprise sodium azide in a final concentration of e.g. 0.15% to 3.75% (w/v) or 0.2% to 3.5% (w/v). According to embodiments, the final concentration in the stabilization composition is 0.3% to 3% (w/v) or 0.3% to 2.5% (w/v).

Furthermore, the preservative may comprise or consist of at least one isothiazolinone compound, preferably methylchloroisothiazolinone and/or methylisothiazolinone. These compounds may be mixed in a ratio of 3:1. A commercially available preservative for inhibiting bacterial growth that comprises isothiazoline compounds is ProClin300. As is demonstrated by the examples, it is suitable for use in the stabilization of urine. It may be comprised in the stabilization composition in a final concentration of e.g. 0.2% to 1% (v/v) or 0.3 to 0.5% (v/v) such as e.g. 0.375% (v/v).

Further examples of preservatives that can be used include but are not limited to chlorhexidine or sodium borate. Further preservatives for inhibiting bacterial growth and useful concentrations can be determined by the skilled person following the disclosure of the present application.

### Caspase inhibitor

According to one embodiment, the stabilizing composition comprising a chelating agent and a poly(oxyethylene) polymer additionally comprises a caspase inhibitor. The advantages of using a caspase inhibitor as well as suitable and preferred embodiments of the caspase inhibitor were described in detail above in conjunction with the stabilization method according to the first aspect and it is referred to the above disclosure which also applies here. Preferably, the caspase inhibitor is a pancaspase inhibitor. Preferably, the caspase inhibitor is a modified caspase specific peptide, preferably modified at the C-terminus with an O-phenoxy group such as Q-VD-OPh. According to an advantageous embodiment, a high molecular weight poly(oxyethylene) polymer having a molecular weight of at least 1500 is used as poly(oxyethylene) polymer. Preferably, polyethylene glycol is used. Preferably, unsubstituted polyethylene glycol is used.

According to one embodiment, the stabilization composition comprises a caspase inhibitor in a concentration from 1µM to 220 µM, e.g. 5 µM to 200 µM, 10 µM to 150 µM and 20.0 µM to 100 µM. In a specific embodiment, the stabilization composition comprises 1 nmol to 1000 nmol, in particular 5 nmol to 500 nmol, 10 nmol to 200 nmol or 25 nmol to 100 nmol of the caspase inhibitor. The stabilizing composition is preferably a liquid that can be filled e.g. in a reception device and is e.g. suitable for stabilizing a sample unit urine.

The stabilizing composition may also comprise further additives as described above in conjunction with the stabilization method according to the first aspect. According to one embodiment, the composition comprises mono-ethylene glycol (1,2-ethanediol) as stabilizing agent. Mono-ethylene glycol may be used in the concentrations described above for the low molecular weight poly(oxyethylene) polymer.

### Amide

According to one embodiment, the stabilizing composition additionally comprises at least one primary, secondary or tertiary amide. Suitable and preferred embodiments were described in detail above in conjunction with the stabilization method according to the first aspect and it is referred to the above disclosure which also applies here. According to one embodiment, the at least one primary, secondary or tertiary amide comprised in the stabilizing composition is a compound according to formula 1 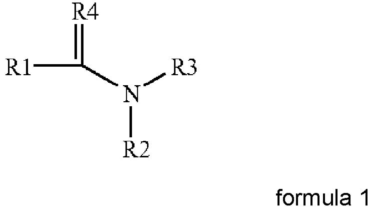 wherein R1 is a hydrogen residue or an alkyl residue, preferably a C1-C5 alkyl residue, a C1-C4 alkyl residue or a C1-C3 alkyl residue, more preferred a C1-C2 alkyl residue, R2 and R3 are identical or different and are selected from a hydrogen residue and a hydrocarbon residue, preferably an alkyl residue, with a length of the carbon chain of 1 - 20 atoms arranged in a linear or branched manner, and R4 is an oxygen, sulphur or selenium residue. Preferably, the amide is a carboxylic acid amide so that R4 is oxygen. Preferred embodiments were described above in conjunction with the stabilization method and it is referred to the above disclosure which also applies here. Preferably, a compound according to formula 1 is used which is not classified as toxic agent. Preferably, said stabilizing composition comprising a compound according to formula 1 additionally comprises a caspase inhibitor.

The compound according to formula 1 may be a carboxylic acid amide selected from primary carboxylic acid amides and secondary carboxylic acid amides. According to one embodiment, the composition comprises a primary carboxylic acid amide selected from the group consisting of formamide, acetamide, propanamide and butanamide. The carboxylic acid may be selected from butanamide and formamide.

According to one embodiment, the at least one compound according to formula 1 is a N,N-dialkyl-carboxylic acid amide. According to one embodiment, the compound according to formula 1 is a N,N-dialkylpropanamide, preferably N,N-dimethlypropanamide.

According to one embodiment, the stabilization composition comprises butanamide and/or an N,N-dialkylpropanamide, wherein said N,N-dialkylpropanamide preferably is N,N-dimethylpropanamide. As is demonstrated by the examples, both amides alone and in combination can support the stabilization effect.

According to one embodiment, the stabilization composition is a liquid composition and comprises one or more primary, secondary or tertiary amides in a concentration selected from 0.4% to 38.3%, e.g. 0.8% to 23.0%, 2.3% to 11.5% and 3.8% to 9.2%. Further ranges include 5% to 15% and 7.5% to 12.5%. The aforementioned concentrations refer to (w/v) or (v/v) depending on whether the primary, secondary or tertiary amide is a liquid or not.

In a specific embodiment, a liquid stabilizing composition comprises 10 µl to 2000 µl, in particular 50 µl to 1000 µl, 100 µl to 750 µl, or 125 µl to 500 or 150 to 250 µl of the primary, secondary or tertiary amide. In particular, the liquid stabilization composition may comprise 0,2 mmol to 15 mmol, in particular 0.5 mmol to 10 mmol, 0.75 mmol to 5 mmol, 1 mmol to 3 mmol, or 1.2 mmol to 2 mmol of the primary, secondary or tertiary amide. Such liquid stabilization composition can be filled e.g. in a reception device and is e.g. suitable for stabilizing a sample unit urine.

### Further embodiments

Further advantageous embodiments of the stabilizing composition according to the third aspect are described subsequently.

According to one embodiment, the stabilizing composition comprises:
- EDTA as chelating agent,
- at least one polyethylene glycol, preferably at least one high molecular weight polyethylene glycol having a molecular weight of at least 3000 and/or at least one low molecular weight polyethylene glycol having a molecular weight of 1000 or less,
- a preservative for inhibiting bacterial growth,
- a buffering agent,
- optionally at least one caspase inhibitor, and
- optionally at least one primary, secondary or tertiary amide.

According to one embodiment, the stabilizing composition is a liquid composition comprising
- EDTA in a concentration of at least 100mM up to the solubility limit, preferably in a concentration that lies in a range of 150mM to 500mM,
- at least one polyethylene glycol, preferably at least one high molecular weight polyethylene glycol having a molecular weight of at least 6000 and optionally at least one low molecular weight polyethylene glycol having a molecular weight of 1000 or less,
- a preservative for inhibiting bacterial growth,
- a buffering agent, and
- optionally at least one caspase inhibitor,
wherein the pH of the stabilizing composition lies in a range of > 7 to 9.5, 7.2 to 9.5, 7.3 to 9.5 or 7.5 to 9.5 and wherein the stabilizing composition is contacted with the urine sample in a volumetric ratio selected from 1:3 to 1:10, preferably 1:5 to 1:8. The pH of the stabilizing composition may be in a range of 7.2 to 9, 7.2 to 8.75 or 7.3 to 8.5. The final pH in the stabilized urine sample may lie in a range of 6 to 9.5, such as 6.3 to 9.5 or 6.5 to 9. As is shown by the examples, using an according stabilization composition inter alia allows to provide a final pH in the stabilized urine sample that lies in a range of 6.5 to 8.75, such as 6.5 to 8.5. Good stabilizing effects were achieved. The final concentration of EDTA in the urine sample stabilized with the stabilization composition preferably lies in a range of 7mg/ml to 40 mg/ml, more preferably in a range of 10mg/ml to 30mg/ml. Suitable embodiments and concentrations for the polyethylene glycol, the preservative and the buffering agent were disclosed above. The stabilizing composition may also comprise at least one primary, secondary or tertiary amide as disclosed herein.

### Composition forms

The stabilizing composition may be provided in a solid form, a semi-liquid form or as liquid. The advantage of using a solid stabilizing composition is that solids are often chemically more stable. Liquid compositions have the advantage that the mixture with the sample to be stabilised can be quickly achieved, thereby basically providing an immediate stabilizing effect as soon as the urine sample comes into contact with the liquid stabilizing composition. The use of a liquid composition is preferred to also allow to adjust the pH of the urine sample. Preferably, stabilizing agent(s) present in the liquid stabilizing composition remain stable in solution and require no pre-treatment-such as for example the dissolving of precipitates of limited solubility-by the user.

The present invention also provides a mixture comprising the stabilizing composition according to the third aspect of the invention mixed with a urine sample. Accordingly, while the composition of the third aspect is not claimed as such, claim 18 is directed to the composition of the third aspect mixed with a urine sample. Suitable and preferred concentrations of the stabilizing agent(s) when mixed with the urine sample and thus in the stabilized urine sample are described above *inter alia* in conjunction with the stabilizing method according to the invention. It is referred to the above disclosure which also applies here.

According to one embodiment, the stabilizing composition of the invention is pre-filled in a sample reception device, e.g. an evacuated tube, so that the sample is immediately stabilized upon including the urine sample into the reception device. Details of such reception device are disclosed in conjunction with the fifth aspect. According to one embodiment, a liquid stabilizing composition is contacted with the urine sample in a volumetric ratio selected from 1:3 to 1:10, such as 1:5 to 1:10, 1:5 to 1:8, in particular about 1:6. It is a particular advantage of the stabilizing composition of the present invention that stabilization of a large sample volume can be achieved with a small volume of the stabilizing composition. Therefore, preferably, the ratio of stabilizing composition to sample lies in a range from 1:10 to 1:5, in particular 1:7 to 1:5, such as e.g. about 1:6.

### Advantages

The advantages have been discussed above in conjunction with the method according to the first aspect and it is referred to the respective disclosure which also applies here. The stabilizing composition provided by the present invention stabilizes important components of the urine sample and allows e.g. the subsequent analysis of cell-free DNA, genomic DNA and/or urine cells.

In embodiments, the stabilization composition does not comprise additives in a concentration wherein said additives would induce or promote cell lysis of nucleated cells and preferably cells in general. As is demonstrated by the examples, urine cells are stabilized and available for cytological analysis and intracellular nucleic acids such as genomic DNA can be isolated and analysed. The stabilization composition does not comprise a cross-linking agent that induces protein-DNA and/or protein-protein crosslinks such as formaldehyde, formaline, paraformaldehyde or a formaldehyde releaser or similar crosslinking agents. Preferably, the stabilization composition of the invention is capable of stabilizing the urine sample without refrigeration, preferably at room temperature, for a time period selected from at least two days, at least three days, at least four days, at least five days and/or at least six days. In particular, one or more, preferably all of the above-described stabilizing effects are achieved during the defined stabilization periods.

The stabilizing composition of the present invention may also be incorporated into a sample collection device, in particular a urine collection assembly, such as a urine collection cup, thereby providing for a new and useful version of such a device. This allows to stabilize the sample directly upon urine collection. In a further embodiment, it is provided in a reception device, preferably an evacuated tube, into which already collected urine is transferred after collection. Details are also disclosed in conjunction with the fifth and sixth aspect.

### D. USE

According to fourth aspect, the present invention is directed to the use of the stabilizing composition according to the third aspect for stabilizing a urine sample, as claimed in claim 16. In particular, the stabilizing composition according to the third aspect can be used in the method according to the first aspect of the present invention. Details of said method were described above and it is referred to the above disclosure which also applies here.

### E. RECEPTION DEVICE

According to a fifth aspect, disclosed but not claimed as such is a reception device for receiving a urine sample, wherein the reception device comprises
- a chelating agent, preferably EDTA,
- at least one poly(oxyethylene) polymer, preferably polyethylene glycol, and
- optionally a preservative for inhibiting bacterial growth.

The reception device is suitable for stabilizing a urine sample. Bacterial growth can be inhibited in the stabilized urine sample. The reception device may comprise one or more of the further stabilizing agents discussed above, such as a caspase inhibitor, which preferably is a caspase inhibitor specific peptide. At least one primary, secondary or tertiary amide (preferably one or more compounds according to formula 1) may also be included. Details have been described above and it is referred to the above disclosure.

According to a preferred embodiment, the reception device comprises the stabilizing composition according to the third aspect which has been described in detail above and the disclosure also applies here.

Providing a respective reception device has the advantage that the urine sample is quickly stabilized when the sample is transferred into said reception device. The urine may be transferred into the reception device for stabilization after it has been collected into a container such as e.g. into a urine cup or pediatric bag. Alternatively, the urine is directly collected into the reception device. The reception device may be used for stabilization in the method according to the first aspect. The reception device can be provided by a container and preferably is an evacuated tube.

### A chelating agent

The reception device comprises a chelating agent, preferably EDTA. Suitable chelating agents and concentrations in the stabilized urine were described above in conjunction with the method according to the first aspect and it is referred to the above disclosure which also applies here. According to one embodiment, the container comprises a chelating agent, preferably EDTA, in a concentration so that when the urine sample is collected into the reception device, the final concentration of the chelating agent in the resulting mixture with urine that is then comprised in the reception device, i.e. in the stabilized urine, is at least 4mg/ml, at least 5 mg/ml or at least 7mg/ml. The use of a chelating agent, such as preferably EDTA, in higher concentrations for stabilizing urine is advantageous to achieve a strong stabilizing effect. Therefore, in embodiments, the final concentration of the chelating agent, which preferably is EDTA, in the stabilized urine sample is at least 7mg/ml, preferably at least 10mg/ml. In the examples, a concentration of at least 15mg/ml was used. According to one embodiment, the final concentration in the stabilized urine sample lies in a range of 10mg/ml to 35 mg/ml or 10mg/ml to 30mg/ml. Also suitable is a final concentration in a range of 12mg/ml to 30mg/ml or 15mg/ml to 25mg/ml. In the examples, a reception device was used which comprised EDTA in a concentration so that upon urine collection, a final concentration of EDTA that lies in a range of 15mg/ml to 20mg/ml was achieved in the stabilized urine.

### Poly(oxyethylene) polymer

The reception device comprises a poly(oxyethylene) polymer, preferably a polyethylene glycol. Suitable and preferred embodiments were described above in conjunction with the method according to the first aspect and it is referred to the above disclosure which also applies here.

According to one embodiment a high molecular weight poly(oxyethylene) polymer is comprised in the device. In one embodiment, the high molecular weight poly(oxyethylene) polymer, which preferably is a polyethylene glycol, is comprised in the reception device in a concentration so that when the urine sample is collected into said device, the final concentration of the high molecular weight poly(oxyethylene) polymer in the resulting mixture lies in a range of 0.05% to 5% (w/v). Suitable ranges include, but are not limited to 0.1% to 4% (w/v), 0.2% to 3% (w/v), 0.25% to 2.5% (w/v), 0.3% to 2% (w/v) and preferably 0.35% to 1.5% (w/v). According to one embodiment, the high molecular weight poly(oxyethylene) polymer is comprised in the reception device in a concentration so that when the urine sample is collected into said device, the final concentration of the high molecular weight poly(oxyethylene) polymer in the resulting mixture lies in a range as 0.2% to 1.5% (w/v), 0.25% to 1.25% (w/v), 0.3% to 1% (w/v) and 0.4% to 0.75% (w/v).

According to one embodiment, the reception device comprises a low molecular weight poly(oxyethylene) polymer having a molecular weight of 1000 or less. The low molecular weight poly(oxyethylene) polymer may have a molecular weight that lies in a range selected from 100 to 1000, 150 to 800 and 150 to 700. Preferably, the molecular weight lies in a range of 200 to 600 and more preferably 200 to 500 such as 200 to 400. According to one embodiment, the low molecular weight poly(oxyethylene) polymer is advantageously comprised in the reception device in a concentration so that when the urine sample is collected into said device, the final concentration of low molecular weight poly(oxyethylene) polymer in the resulting mixture lies in a range selected from 0.5% to 10%, 1.5% to 9%, 2% to 8% and 2.5% to 7% and 3% to 6%. The percentage values refer to (w/v) in case the poly(oxyethylene) polymer is a solid and to (v/v) in case the poly(oxyethylene) polymer is a liquid. Preferably, the poly(oxyethylene) polymer is a polyethylene glycol.

According to one embodiment, the reception device comprises a high molecular weight poly(oxyethylene) polymer as defined above which has a molecular weight of at least 1500 and a low molecular weight poly(oxyethylene) polymer having a molecular weight of 1000 or less, so that the urine sample is contacted with both types of poly(oxyethylene) polymers when urine is added to the reception device. Suitable and preferred embodiments as well as the advantages associated with this embodiment, wherein a high molecular weight poly(oxyethylene) polymer is used in combination with a low molecular weight poly(oxyethylene) polymer, was described in detail above and it is referred to the respective disclosure which also applies here.

In an advantageous embodiment, the reception device comprises the high molecular weight poly(oxyethylene) polymer and the low molecular weight poly(oxyethylene) polymer in a concentration so that when the urine sample is collected into said device, the concentration of the high molecular weight poly(oxyethylene) polymer in the resulting mixture lies in a range of 0.2% to 1.5% (w/v), 0.3% to 1.25% (w/v) and 0.4% to 0.75% (w/v) and the concentration of the low molecular weight poly(oxyethylene) polymer lies in a range selected from 1.5% to 8%, 2% to 7% and 2.5% to 6%. Preferably, the high as well as the low molecular weight poly(oxyethylene) polymer is a polyethylene glycol as described above.

According to one embodiment, the mixture that is obtained after contacting the urine sample with the high and the low molecular weight poly(oxyethylene) polymer and optionally further additives used for stabilization, comprises the high molecular weight poly(oxyethylene) polymer, which may have e.g. a molecular weight in the range of e.g. 4000 to 20000, in a final concentration that lies in a range of 0.15% to 1.5%, e.g. 0.2% to 1% (w/v), preferably 0.25% to 0.75% (w/v) such as 0.5% (w/v) and the low molecular weight poly(oxyethylene) polymer, which preferably has a molecular weight that lies in a range of 200 to 800, preferably 200 to 600, in a final concentration that lies in a range selected from 1.5% to 8%, preferably 2% to 7%, more preferred 2.5% to 6%. Polyethylene glycol, preferably unsubstituted, is preferably used as high and as low poly(oxyethylene) polymer. Suitable embodiments for the high and low molecular weight poly(oxyethylene) polymer are also described above and in conjunction with the different aspects and embodiments disclosed herein.

### Preservative for inhibiting bacterial growth

The reception device may furthermore comprise a preservative for inhibiting bacterial growth. The preservative is used in addition to the chelating agent. Suitable and preferred embodiments of the preservative were described above in conjunction with the method according to the first aspect and it is referred to the above disclosure which also applies here.

The preservative is comprised in an amount so that bacterial growth is inhibited in the stabilized urine sample. Suitable concentrations can be determined by the skilled person for individual preservatives.

The preservative may be comprised in a stabilization composition according to the third aspect that may be included into the reception device. The final concentration in the stabilization composition may lie e.g. in the range of 0.1 to 5.5% w/v, e.g. 0.2 to 3.75 % w/v or may lie in the range of 0.1 to 5.5% v/v, e.g. 0.2 to 3.75 % v/v.

According to one embodiment, the preservative may comprise or consist of sodium azide. As is demonstrated by the examples, sodium azide is particularly suitable. The stabilization composition comprised in the reception device may comprise sodium azide in a final concentration of e.g. 0.15% to 3.75% (w/v) or 0.2% to 3.5% (w/v). According to embodiments, the final concentration in the stabilization composition is 0.3% to 3% (w/v) or 0.3% to 2.5% (w/v).

Furthermore, the preservative may comprise or consist of at least one isothiazolinone compound, preferably methylchloroisothiazolinone and/or methylisothiazolinone. These compounds may be mixed in a ratio of 3:1. A commercially available preservative for inhibiting bacterial growth that comprises isothiazoline compounds is ProClin300. As is demonstrated by the examples, it is suitable for use in the stabilization of urine. It may be comprised in the stabilization composition contained in the reception device in a final concentration of e.g. 0.2% to 1% (v/v) or 0.3 to 0.5% (v/v) such as e.g. 0.375% (v/v).

Further examples of preservatives that can be used include but are not limited to chlorhexidine or sodium borate. Further preservatives for inhibiting bacterial growth and useful concentrations can be determined by the skilled person following the disclosure of the present application.

The preservative may be comprised in the reception device in a concentration so that when the urine sample is collected into the reception device, the final concentration of the preservative in the resulting mixture, i.e. the stabilized urine, lies in the range of 0.01 to 0.7% w/v, e.g. 0.03 to 0.5 % w/v, or may lie in the range of 0.01 to 0.7% v/v, e.g. 0.03 to 0.5 %v/v. Suitable concentrations for the preservative in the stabilized urine sample and embodiments were also disclosed in conjunction with the method according to the first aspect and it is referred to this disclosure which also applies here.

### Caspase inhibitor

In one embodiment, at least one caspase inhibitor is additionally comprised in the reception device. Suitable and preferred embodiments were described above in conjunction with the method according to the first aspect and it is referred to the above disclosure which also applies here.

The caspase inhibitor may be comprised in a concentration so that when the urine sample is collected into the reception device, the final concentration of the caspase inhibitor in the resulting mixture, i.e. the stabilized urine, is at least 0.5µM or at least 1µM, e.g. at least 1.5µM, at least 2µM or at least 2.5µM. Suitable final concentration ranges for the caspase inhibitor(s) when mixed with the urine sample and the further additives include but are not limited 1µM to 25µM, 1.5µM to 20µM and 2µM to 15µM. Preferred are final concentration ranges of 2µM to 12.5µM, 2.5µM to 10µM and 3µM to 7.5µM. It may lie in a range of 0.1µM to 20µM, more preferred 0.5µM to 10µM, more preferred 1µM to 10µM, more preferred 3µM to 7.5µM or 3µM to 5µM. According to one embodiment, at least one primary, secondary or tertiary amide as described above is additionally used.

### Amide

In one embodiment, at least one primary, secondary or tertiary amide is comprised in the reception device. Suitable and preferred embodiments were described above in conjunction with the method according to the first aspect and it is referred to the above disclosure which also applies here. The amide may be used in a concentration so that when the urine sample is collected into said device, the final concentration of the amide (or combination of amides) in the resulting mixture, i.e. the stabilized urine, lies in a range of at least 0.05%. E.g. the final concentration may be at least 0.1%, at least 0.25%, at least 0.5% or at least 0.75%. Suitable concentration ranges include but are not limited to 0.1% to 10%, 0.25% to 7.5%, 0.3% to 5%, 0.4% to 3%, 0.5% to 2%, 0.6% to 1.8% or 0.75% to 1.5%. Concentrations or concentration ranges indicated in percentage values as used herein are in particular given as percentage weight per volume (w/v) for solid amides and as percentage volume per volume (v/v) for liquid amides.

The at least one amide preferably is a compound according to formula 1 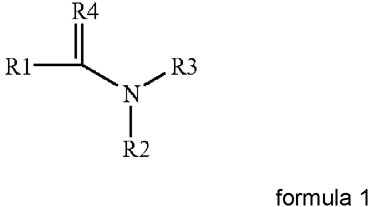 wherein R1 is a hydrogen residue or an alkyl residue, preferably a C1-C5 alkyl residue, a C1-C4 alkyl residue or a C1-C3 alkyl residue, more preferred a C1-C2 alkyl residue, R2 and R3 are identical or different and are selected from a hydrogen residue and a hydrocarbon residue, preferably an alkyl residue, with a length of the carbon chain of 1 - 20 atoms arranged in a linear or branched manner, and R4 is an oxygen, sulphur or selenium residue. Preferably, the amide is a carboxylic acid amide so that R4 is oxygen. Preferred embodiments of the compound according to formula 1 are described above in conjunction with the stabilization method and it is referred to the above disclosure which also applies here. According to one embodiment, the reception device comprises butanamide and/or a an N,N-dialkylpropanamide which preferably is N,N-dimethylproanamide as compound according to formula 1.

According to one embodiment, the reception device comprises mono-ethylene glycol as stabilizing agent. Mono-ethylene glycol may be used in the concentrations described above for the low molecular weight poly(oxyethylene) polymer. It is referred to the respective disclosure which also applies here.

### pH

As is demonstrated in the examples, the pH influences the stabilization result.

The pH of the stabilizing composition that is comprised in the reception device lies in the range of > 7 to ≤ 9.5. The pH may be in the range of 7.2 to 9.5, 7.3 to 9.5 or 7.4 to 9.5. The pH of the stabilizing composition may also be ≤ 9.25, ≤ 9 or ≤ 8.75, such as ≤ 8.5, ≤ 8.3 or ≤ 8. These pH values may be combined as upper value with the pH ranges indicated before and the resulting ranges also form part of the present disclosure. In one embodiment, the pH of the stabilization composition is in the range of 7.5 to ≤ 9.5, such as 7.5 to 9 or 7.5 to 8.5. In one embodiment, the pH of the stabilizing composition is in the range of 7.2 ≤ 8.5, such as 7.3 to ≤ 8.3 or 7.5 to ≤ 8. The pH of the stabilizing composition may furthermore be in the range of 7.7 to 9.5 or 8 to 9.25. According to a further embodiment also shown in the examples, the pH of the stabilizing composition is in the basic range of 8 to 9.

The stabilizing composition preferably comprises a suitable buffering agent that allows to maintain the pH within the desired range when the urine sample is added. Suitable embodiments for the pH range in the stabilized urine sample have been described above in conjunction with the method according to the first aspect to which it is referred, as well as the important effect of the pH on the stabilization result.

According to one embodiment, said stabilizing composition is a liquid composition and the reception device is designed (e.g. evacuated) so that the liquid composition is contacted with the received urine sample in a volumetric ratio selected from 1:3 to 1:10, preferably 1:5 to 1:10. It is a particular advantage of teachings of the present invention that stabilization of a large urine volume and pH adjustment can be achieved with a small volume of the stabilizing composition according to the present invention. Preferably, the ratio of stabilizing composition to sample lies in a range from 1:5 to 1:8, such as about 1.6. E.g. 1-2ml liquid stabilising composition may be contacted with approx. 10ml urine.

As is demonstrated by the examples, the higher pH values are more efficient in preserving the cell-free DNA, because degradation of cell-free DNA is more efficiently prevented compared to an acidic pH. This is highly advantageous when intending to analyse cell-free DNA comprised in the urine sample. As can be seen from the examples a higher pH, such as in particular a basic pH, efficiently prevents cell free DNA degradation even when stabilized urine samples are stored for several days.

The stabilizing composition comprises a buffering agent for pH adjustment. The buffering agent may have its pK in the basic range. A suitable and preferred buffering agent is Tris as is demonstrated by the examples. Other buffering agents include but are not limited to MOPS, TES, HEPES, TAPSO, Tricine, Bicine, TAPS, borate buffer, CHES buffer or buffers on the basis of sodium or potassium phosphate. A further buffering agent comprises carbonate. The pH of the urine may vary from acidic to basic from donor to donor and additionally depending on the donors conditions. It is thus advantageous to comprise the buffering agent in a sufficient concentration to achieve an efficient pH adjustment and stabilization despite potential variations in the pH of the collected urine sample to be stabilized. Suitable concentrations can be determined by the skilled person.

According to one embodiment, the final pH of the stabilized urine sample comprised in the reception device lies in a range of 6 to 9.5, such as 6.3 to 9.5. The final pH of the stabilized urine sample may be ≤9, such as ≤8.75 or ≤8.5. Particularly suitable is a pH in the stabilized urine sample that lies in the range of 6.5 to 9 such as 6.5 to 8.75 or 6.5 to 8.5. In embodiments, the pH of the stabilized urine sample lies in a range of 6.7 to 9 or 7 to 9, such as 7.2 to 9 or 7.2 to 8.75. According to one embodiment, the final pH of the stabilized urine sample is at least 7 or above 7 and may lie in a range of 7.5 to 8.5.

### Further embodiments

The stabilizing composition and/or the individual compounds used for stabilization comprised in the reception device can be provided in a liquid; semi-liquid or in a dry form. The compounds used for stabilization may be provided as separate entities in the reception device and may also be provided in different forms in the reception device. E.g. one component may be provided in dry form while the other compound may be provided as liquid. Other combinations are also feasible. Suitable formulation and manufacturing options are known to the skilled person. As discussed above, the core stabilizing compounds and also the further additives used for stabilization may be provided in the reception device in form of a stabilizing composition, preferably the stabilization composition according to the third aspect.

The advantage of using a solid stabilizing composition is that solids are usually chemically more stable than liquids. According to one embodiment, the inner wall of the reception device is treated/covered with a stabilizing composition according to the present invention or with individual components thereof, such as e.g. EDTA. Said composition or component can be applied to the inner walls using e.g. a spray-dry-method. Liquid removal techniques can be performed on the stabilizing composition in order to obtain a substantially solid state protective composition. Liquid removal conditions may be such that they result in removal of at least about 50% by weight, at least about 75% by weight, or at least about 85% by weight of the original amount of the dispensed liquid stabilizing composition. Liquid removal conditions may be such that they result in removal of sufficient liquid so that the resulting composition is in the form of a film, gel or other substantially solid or highly viscous layer. For example it may result in a substantially immobile coating (preferably a coating that can be redissolved or otherwise dispersed upon contact with the urine sample). It is possible that lyophilization or other techniques may be employed for realizing a substantially solid form of the protective agent (e.g., in the form of one or more pellet). Thus, liquid removal conditions may be such that they result in a material that upon contact with the urine sample the protective agent will disperse in the sample, and substantially preserve components (e.g., extracellular nucleic acids) in the sample. Liquid removal conditions may be such that they result in a remaining composition that is substantially free of crystallinity, has a viscosity that is sufficiently high that the remaining composition is substantially immobile at ambient temperature; or both.

According to a preferred embodiment, the reception device comprises a liquid stabilizing composition. Liquid compositions have the advantage that the mixture with the urine sample to be stabilised can be quickly achieved, thereby basically providing an immediate stabilizing effect as soon as the urine sample comes into contact with the liquid stabilizing composition. Furthermore, liquid compositions are advantageous if larger amounts of stabilization compositions are used which accordingly, cannot or are difficult to spray-dry or if the composition hampers providing a dry composition. Preferably, the stabilizing agents present in the liquid stabilizing composition remain stable in solution and require no pre-treatment - such as for example the dissolving of precipitates of limited solubility - by the user because pre-treatments of this kind pose a risk of variations in the stabilizing efficiency.

The stabilizing composition is comprised in the reception device in an amount effective to provide the stabilization of the amount of urine sample to be received by the reception device. According to one embodiment, the liquid stabilizing composition is contacted with the urine sample in a volumetric ratio selected from 10:1 to 1:20, 5:1 to 1:15, 1:1 to 1:10, 1:10 to 1:5 and 1:7 to 1:5, in particular about 1:6. It is a particular advantage of the stabilizing composition of the present invention and the reception device comprising such stabilizing composition that stabilization of a large sample volume can be achieved with a small volume of the stabilizing composition. Therefore, preferably, the ratio of the liquid stabilizing composition to the urine sample lies in a range from 1:10 to 1:5, in particular 1:7 to 1:5, such as about 1:6. Embodiments were also described above and it is referred to this disclosure which also applies here.

According to one embodiment, the reception device is evacuated. The evacuation is preferably effective for drawing a specific volume of the urine sample, which may have been collected in a urine collection cup in advance, into the interior. Thereby, it is ensured that the correct amount of urine is contacted with the pre-filled amount of the stabilizing agents comprised in the reception device, and accordingly, that the stabilization is efficient. According to one embodiment, the reception device comprises a tube having an open end sealed by a septum. E.g. the reception device is pre-filled with a defined amount of the stabilizing composition either in solid or liquid form and is provided with a defined vacuum and sealed with a septum. The septum is constructed such that it is compatible with the standard sampling accessories (e.g. cannula, etc.). When contacted with urine, e.g. via a transfer device (e.g. a tube), an amount of urine that is predetermined by the vacuum is collected into the reception device. According to one embodiment, the reception device is for receiving an amount of urine that lies in the range of 5ml to 20ml, preferably 7 to 15ml, such as 10ml.

The reception device according to the present invention can be made of glass, plastic or other suitable materials. Plastic materials can be oxygen impermeable materials or may contain an oxygen impermeable layer. Alternatively, the reception device can be made of airpermeable plastic material. The reception device may be made of a transparent material. Examples of suitable transparent thermoplastic materials include polycarbonates, polyethylene, polypropylene and polyethyleneterephthalate. The reception device may have a suitable dimension selected according to the required volume of the urine sample to be received. As described above, preferably, the reception device is evacuated to an internal pressure below atmospheric pressure. The pressure is preferably selected to draw a predetermined volume of the urine sample into the reception device.

According to one embodiment, the reception device has an open top, a bottom, and a sidewall extending therebetween defining a chamber. The chelating agent, which preferably is EDTA, the poly(oxyethylene) polymer, which preferably is a polyethylene glycol, the preservative for inhibiting bacterial growth and optionally the one or more further stabilizing agents mentioned above or the stabilization composition according to the third aspect may be comprised in the chamber. It may be comprised therein in liquid or solid form. According to one embodiment, it is a liquid. According to one embodiment the reception device is a tube, the bottom is a closed bottom, the reception device further comprises a closure in the open top, and the chamber is at a reduced pressure. The advantages of a reduced pressure in the chamber were described above. Preferably, the closure is capable of being pierced with a needle or cannula, and the reduced pressure is selected to draw a specified volume of a liquid sample into the chamber. According to one embodiment, the chamber is at a reduced pressure selected to draw a specified volume of the urine sample into the chamber, and the stabilizing composition is a liquid and is disposed in the chamber such that the volumetric ratio of the stabilizing composition to the specified volume of the urine sample is selected from 10:1 to 1:20, 5:1 to 1:15 and 1:1 to 1:10 and preferably is 1:10 to 1:5, more preferably 1:7 to 1:5. The associated advantages were described above.

According to one embodiment, the reception device is for receiving 10ml urine. According to one embodiment, the stabilizing composition is a liquid and the volume is 2ml or less and may lie in a range of 0.5ml to 2ml, 0.75ml to 1.75ml and 1ml to 1.7ml.

### F. METHOD FOR COLLECTING A URINE SAMPLE

According to a sixth aspect, disclosed but not claimed as such is a method for collecting a urine sample.

According to one embodiment, the urine sample is collected into a chamber of a reception device according to the fifth aspect of the present invention, wherein the reception device preferably comprises a stabilizing composition according to the third aspect. Details with respect to the reception device, the stabilizing composition and the stabilized urine sample were described above. It is referred to the respective disclosure.

The urine may also be first collected in a urine cup or other container prior to stabilization. Hence, according to one embodiment, the present invention provides a method for collecting a urine sample, comprising
a) collecting urine in a container, preferably a urine cup; and
b) stabilizing the urine sample using the method according to the first aspect.

In step a), the urine sample is collected from a donor into a container. Suitable containers for collecting urine are known in the art and widely used for urine collection from a donor. The container may be e.g. urine cup or pediatric bag. In one embodiment, the container comprises one or more stabilizing agents in dry form, preferably spray-dried form. This provides the advantage that an initial stabilization effect is already achieved upon first urine collection. The urine dissolves the dry stabilizing agent(s).

According to one embodiment, the container used in step a) comprises a chelating agent, such as preferably EDTA. The chelating agent may be incorporated in spray-dried form. Incorporating a chelating agent such as EDTA into the container that is used for initial urine collection, such as a urine cup, provides the further advantage that the final concentration of the chelating agent in the stabilized urine sample, i.e. after performing step b), can be increased.

In step b), the collected urine sample is then stabilized using the method according to the first aspect of the present invention described in detail above, in particular by using the method according to any one of claims 1 to 14. The transferred urine sample may already comprise dissolved stabilizing agent(s) such as EDTA, if such agent(s) was/were incorporated during step a).

The urine collected in step a) may be transferred for stabilization in step b) into a reception device according to the fifth aspect, wherein the reception device preferably comprises a stabilizing composition according to the third aspect. The reception device used in step b) is preferably an evacuated tube for receiving a defined amount of urine sample. This allows to stabilize a defined volume in step b). The stabilization composition is preferably a liquid composition. Details were described above and it is referred to the above disclosure.

After stabilization in step b), the stabilized urine sample may be further processed, as is disclosed e.g. in conjunction with the method according to second aspect. It is referred to the respective disclosure which also applies here.

### G. MANUFACTURING METHOD

According to a seventh aspect, disclosed but not claimed as such is a method of manufacturing a stabilizing composition according to the third aspect of the present invention, wherein the components of the stabilizing composition are combined. Preferably, they are mixed to provide a liquid solution.

Numeric ranges are inclusive of the numbers defining the range. The headings provided herein are not limitations of the various aspects or embodiments of this invention which can be read by reference to the specification as a whole.

Unless the context indicates otherwise, percentage values indicated herein refer to (w/v) in case of solid compounds contained in a liquid mixture or composition and to (v/v) in case of liquid compounds contained in a liquid mixture or composition such as e.g. the mixture resulting from contacting the stabilizing agents or the stabilizing composition containing said agents with the urine sample.

As used in the subject specification, the singular forms "a", "an" and "the" include plural aspects unless the context clearly dictates otherwise. Thus, for example, reference to "a poly(oxyethylene) polymer" includes a single type of poly(oxyethylene) polymer, as well as two or more poly(oxyethylene) polymers. Likewise, reference to an "agent", "additive", "compound" and the like includes single entities and combinations of two or more of such entities. Reference to "the disclosure" and "the invention" and the like includes single or multiple aspects taught herein; and so forth. Aspects taught herein are encompassed by the term "invention".

The term "solution" as used herein in particular refers to a liquid composition, preferably an aqueous composition. It may be a homogenous mixture of only one phase but it is also within the scope of the present invention that a solution comprises solid additives such as e.g. precipitates, in particular of contained chemicals such as stabilizing agents.

The terms "stabilization composition" and "stabilizing composition" are used as synonyms herein.

The sizes, respectively size ranges indicated herein with reference to nucleotides (nt), refer to the chain length and thus are used in order to describe the length of single-stranded as well as double-stranded molecules. In double-stranded molecules said nucleotides are paired.

As used herein, the term "comprising" is to be construed as encompassing both "including" and "consisting of", both meanings being specifically intended, and hence individually disclosed embodiments in accordance with the present invention. According to one embodiment, subject matter described herein as comprising certain steps in the case of methods or as comprising certain ingredients in the case of compositions, solutions and/or buffers refers to subject matter consisting of the respective steps or ingredients. It is preferred to select and combine preferred embodiments described herein and the specific subject-matter arising from a respective combination of preferred embodiments also belongs to the present disclosure.

### EXAMPLES

It should be understood that the following examples are for illustrative purpose only and are not to be construed as limiting this invention in any manner. Subject-matter of the examples not pertaining to the claimed invention is for illustrative purposes.

### Abbreviations used

cfDNA: cell-free DNA
PEG: Polyethylene glycol
EDTA: Ethylenediaminetetraacetic acid
DMPA: Dimethylpropionamide
DEGMEA: Diethlyeneglycol-monoethylether-acetate
PBS: Phosphate buffered saline

### I. EXPERIMENTAL PROCEDURES

### 1. Stabilization of urine

If not stated otherwise, urine was collected in 200ml bottles. 10ml of urine was mixed with 1.7ml of
(i) stabilization composition,
(ii) PBS,
(iii) EDTA solution,
   within 15 min after urine collection or
(iv) kept unstabilized.

Urine and reagents were mixed by inverting the tube ten times. Stabilized and unstabilized urine samples were stored at room temperature standing in an upright position.

**Table 1 gives an overview over the used stabilization compositions (reagents) and characteristics of the stabilized urine sample:**

| **Component - approx. final concentration in the stabilized urine sample. The pH were indicated refers to the pH of the stabilizing reagent (stock solution) Regarding PEG: 10k = 10000Da; 300 = 300Da** | | | | | | |
|---|---|---|---|---|---|---|
| Reagent | K2-EDTA | Caspase Inh. (Q-VD-OPh) | PEG | Buffer | Preservative | Other |
| R1 | 18 mg/ml | 5 µM | 0.5% (10k, w/v) | - | 0.05% ProClin300 | 1.5% DMPA (v/v) |
| | | | 3.5% (300, v/v) | | | |
| R2 | 18 mg/ml | 5 µM | 0.5% (10k, w/v) | - | 0.05% ProClin300 | 1.5% DEGMEA (v/v) |
| | | | 3.5% (300, v/v) | | | |
| R3 | 18 mg/ml | 5 µM | 2% (10k, w/v) | - | 0.05% ProClin300 | 1.5% Butanamide (w/v) |
| R4 | 18 mg/ml | 5 µM | 0.5% (10k, w/v) | - | 0.05% ProClin300 | - |
| | | | 3.5% (300, v/v) | | | |
| R5 | 18 mg/ml | 5 µM | 4% (10k, w/v) | - | 0.05% | - |
| | | | | | ProClin300 | |
| R6 | 18 mg/ml | 5 µM | 8% (300, w/v) | - | 0.05% ProClin300 | - |
| R7 | 18 mg/ml | 5 µM | 0.5% (10k, w/v) | - | 0.05% ProClin300 | 0.2% Diazolidinyl-urea (v/v) |
| | | | 3.5% (300, v/v) | | | |
| R8 | EDTA pH7 | 5 µM | 0.5% (10k, w/v) | Tris/HCl pH7 | 0.04% NaN3 | - |
| | 14.7mM | | 3.5% (300, v/v) | | | |
| | | | | 14.8mM | | |
| R9 | EDTA pH8 | 5 µM | 0.5% (10k, w/v) | Tris/HCl pH8 | 0.04% NaN3 | - |
| | 14.7mM | | 3.5% (300, v/v) | | | |
| | | | | 14.8mM | | |
| R10 | EDTA pH9 | 5 µM | 0.5% (10k, w/v) | Tris/HCl pH9 | 0.04% NaN3 | - |
| | 14.7mM | | 3.5% (300, v/v) | | | |
| | | | | 14.8mM | | |
| R11 | 18 mg/ml | 5 µM | 6% (10k, w/v) | - | 0.05% ProClin300 | - |
| R12 | EDTA pH8 | 5 µM | 0.5% (10k, w/v) | Tris/HCl pH8 | 0.04% NaN3 | - |
| | 29.4mM | | 3.5% (300, v/v) | | | |
| | | | | 14.8mM | | |
| R13 | EDTA pH8 | 5 µM | 0.5% (10k, w/v) | Tris/HCl pH8 | 0.04% NaN3 | Butanamide 1.5% (v/v) |
| | 29.4mM | | 3.5% (300, v/v) | | | |
| | | | | 14.8mM | | |
| R14 | EDTA pH9 | 5 µM | 0.5% (10k, w/v) | Tris/HCl pH9 | 0.04% NaN3 | - |
| | 29.4mM | | 3.5% (300, v/v) | | | |
| | | | | 14.8mM | | |
| R15 | EDTA pH9 | 5 µM | 0.5% (10k, w/v) | Tris/HCl pH9 | 0.04% NaN3 | Butanamide 1.5% (v/v) |
| | 29.4mM | | 3.5% (300, v/v) | | | |
| | | | | 14.8mM | | |
| R16 | 18 mg/ml | 5 µM | 0.5% (10k, w/v) | - | 0.05% ProClin300 | - |
| | | | 3.5% (300, v/v) | | | |
| R17 | EDTA pH7 | 5 µM | 0.5% (10k, w/v) | Tris/HCl pH7 | 0.04% NaN3 | - |
| | 29.4mM | | 3.5% (300, v/v) | | | |
| | | | | 14.8mM | | |
| R18 | 18 mg/ml | 5 µM | 0.5% (10k, w/v) | Citrate buffer pH4 | 0.05% ProClin300 | - |
| | | | 3.5% (300, v/v) | | | |
| | | | | 20.9 mM | | |
| R19 | 18 mg/ml | 5 µM | 0.5% (10k, w/v) | Citrate buffer pH3 | 0.05% ProClin300 | - |
| | | | 3.5% (300, v/v) | | | |
| | | | | 20.9 mM | | |
| EDTA | 1.8 mg/ml | | | | | |

### 2. Urine processing

If not stated otherwise, urine samples were processed by centrifugation at ambient temperature for 15min at 1.900xg. The cellular free supernatant was transferred into a fresh centrifuge tube. The cell pellet was resuspended in PBS and used for gDNA isolation and/or cytocentrifugation on glass slides for cytology staining.

The supernatant was centrifuged a second round for another 10min at 1.900xg. The supernatant from the second centrifugation was transferred into a new tube and either directly used for purification of cell-free DNA or stored at -20°C until use.

### 3. Manual purification of gDNA

Up to 200µl from the resuspended cell pellets derived from urine first centrifugation were used to purify genomic DNA using the QIAamp Mini kit (QIAGEN). Resuspended pellet was mixed with lysis buffer AL, incubated with Proteinase K, ethanol was added to adjust binding conditions, loaded on a silica membrane, washed twice and eluted with buffer AE according to the handbook.

The DNA yield was measured on a Nandrop (Thermo-Fisher). Integrity was analyzed by agarose gelelectrophoresis. 400ng genomic DNA were loaded on 1.5% TAE gels with lamda Hind Ill marker, separated for 2 hours at 100V (150mA) and stained with ethidium-bromide.

### 4. Cytological analysis of cells within urine

Up to 100µl resuspended urine cell pellets were filled in an EZ Funnel and directly centrifuged onto a microscopic slide with a Cytospin 4 cytocentrifuge (Shandon). Slides were air-dried for a few seconds and used for staining according to Papanicoloau (1min nuclei staining with Papanicolaou Hematoxylin, 3min keratin staining with Papanicolaou Lsg OG6 (2a) and 3min cytoplasma staining with Papanicolaou Lsg EA50) or by H&E staining (30sec hematoxylin and 1min eosin) using kits or staining reagents from Merck.

### 4. Purification of cell-free DNA

Cell-free DNA from 4 - 4.8ml stabilized or unstabilized urine was isolated with the QIAsymphony PAXgene Blood ccfDNA Kit (QIAGEN kit) protocol. The QlAsymphony robot mixed urine with proteinase K, binding buffer and magnetic beads. Beads with bound DNA were washed three times and DNA was eluted using 60 µl elution buffer.

### 5. Quantitative, real time PCR assay and calculation of 18s rDNA copies

To measure the amount of cell-free DNA a real time PCR assay on Abi Prism HT7900 (Life technologies) was performed with 8µl of eluate. In a 20µl assay volume using QuantiTect Multiplex PCR Kit reagents (QIAGEN GmbH) two fragments of the human 18S rDNA gene were amplified in a multiplex PCR. Total quantification was achieved by comparison with a standard curve generated with human genomic DNA samples equivalent to 10.000 to 10 copies of human 18s rDNA gene.

**Table 2: DNA target sequences detected by real time PCR**

| Target description | Position | position | Sequence 5' - 3' | Dye |
|---|---|---|---|---|
| h 18S rDNA 66bp amplicon | p12 - region of chromosome 13, 14, 15, 21, 22 | Forward | GCCGCTAGAGGTGAAATTCTTG | 5' Bodipy - BHQ 3' |
| | | reverse | CATTCTTGGCAAATGCTTTCG | |
| | | Probe | ACCGGCGCAAGACGGACCAGA | |
| h18S rDNA 500bp amplicon | p12 - region of chromosome 13, 14, 15, 21, 22 | forward | GTCGCTCGCTCCTCTCCTACTT | 5' FAM - BHQ 3' |
| | | reverse | GGCTGCTGGCACCAGACTT | |
| | | Probe | CTAATACATGCCGACGGGCGCTGAC | |

Quantification of the 66bp fragment was used to reflect the total amount of 18S rDNA copies in the urine. Quantification of the 500bp was used to determine the amount of 18S rDNA copies which derived from apoptotic or mechanically lysed urine cells. Cell-free DNA in urine has a typical length of 60 - 170bp. Therefore 500bp fragments can be considered to be derived from apoptotic, lysed or otherwise destructed urine cells.

### 6. Quantitative, real time PCR assay for Y-chromosmale DSY14 gene

To measure the degradation of cell-free DNA, the relative yield of male cell-free DNA spiked into female samples was analyzed. A real time PCR assay on RotorGene Q (QIAGEN GmbH) was performed with 8µl of eluate. In a 20µl assay volume using QuantiTect Multiplex PCR Kit reagents (QIAGEN GmbH) one 66bp fragments of the human Y-chromosomale DYS14 gene was amplified in a real time PCR.

| Target | Position | Position | Sequence 5' - 3' | Dye |
|---|---|---|---|---|
| DYS14 | Y-Chromosome | Forward | GAGCAGGCGTGGGTACTATTG | 5' JOE-BHQ1 3' |
| | | Reverse | GTCTGCTGCTCGGCATCAC | |
| 66bp amplicon | | Probe | CCTGCATGCGGCAGAGAAACCC | |

### II. PERFORMED EXAMPLES

### 1. Example 1 - Stabilization of DNA in urine

200ml urine each from four female and two male donors was collected. Aliquots of 10ml urine were mixed within 15min after sampling with 1.7ml PBS or stabilizing reagent. After urine processing, DNA was isolated from the cell-free sample on the QlAsymphony either directly after mixture with reagents (t0) or after four days incubation at room temperature. All samples were processed in duplicates. The copy numbers of 18s rDNA were determined from duplicate samples in triplicates by real time PCR. The results for the 66bp fragment are shown in Fig. 1; the results for the 500bp fragment are shown in Fig. 2.

### Discussion

The results demonstrate that cell-free DNA degrades rapidly in unstabilized urine i.e. buffered with PBS. The release of DNA from apoptotic or lysed cells cannot be monitored in unstabilized urine (PBS), *inter alia* because of the rapid degradation even at timepoint 0. Both processes, degradation of cell-free and release of DNA from cells, appear to happen at the same time. Therefore, the degradation of the cell-free DNA might be even more severe in PBS than already shown in Fig. 2. The total amount of cell-free DNA, small as well as large molecules, is preserved in urine that is stabilized with reagents according to the present disclosure.

### 2. Example 2 - Sampling of urine into BD Vacutainer Urine Collection Cups and transfer into different vacutainers comprising different stabilizing reagents

Urine from one male donor was centrifuged twice to remove all cells from the urine for spike-in into female urine. Urine was collected in larger bottles from two female and two male donors. In case of the two female donors 25ml centrifuged and therefore cell-free male urine was added to the 200ml female urine. Approximately 120ml urine from each donor was filled into BD Vacutainer Urine Collection Cups. Urine was transferred from the cups into two spray dried EDTA tubes, one PAXgene Blood ccfDNA tube and one Streck cell-free DNA BCT tube per donor. One of the EDTA tubes per donor was opened and 1.7 ml of stabilization reagent R1 was added. All tubes were carefully inverted 5 times to mix the urine with the components.

Cell-free DNA was subsequently purified on the QIAsymphony either directly after collection (t0) or after 1, 3 and 9 days incubation at room temperature. Copy numbers of 18s rDNA were determined from duplicate samples in triplicates by real time PCR. Relative yield of Y-chromosomale DYS14 DNA was determined from duplicate samples by real time PCR (no PCR replicates). The results are shown in Figure 3 and 4.

### Discussion

This example shows that the BD Vacutainer Urine Collection Cup can be integrated into the urine workflow. Furthermore, the data shows that EDTA alone neither prevents sufficiently cell free DNA degradation (Figure 4) nor an increase in total DNA comprised in the urine due to DNA release from cells (Figure 3) over the storage period. In contrast, the PAXgene Blood ccfDNA Tube, which is intended for use with blood samples, stabilizes total DNA and cell-free DNA in urine up to 3 days. After 9 days an increase in total DNA and decrease in spiked in cell-free DNA can be seen.

The Streck cfDNA BCT reasonably stabilizes total DNA in urine, however, it leads to a reduction of spiked in cell-free DNA, similar to K2-EDTA, thereby demonstrating that the original cell-free DNA is degraded and that DNA is released from cells during the stabilization period (see combination of Figure 3 and 4).

The stabilization reagent R1 added to urine collected in K2-EDTA tubes efficiently stabilized DNA yield and preserves spiked in male DYS14 copies in female urine, thereby demonstrating that a degradation of cell-free DNA is efficiently prevented/reduced as well as a release of genomic DNA from cells. These beneficial results were also maintained over prolonged stabilization periods.

### 3. Example 3: Multicomponent testing performed with one urine sample

200ml urine each from three donors was collected. Aliquots of 10ml urine were mixed within 15min after sampling with 1.7ml reagent R1 or kept unstabilized. Samples were processed immediately or stored at room temperature for 3 and 7 days. For processing, the samples were centrifuged 15min at 1900 xg. Supernatant was centrifuged again (10min 1900 xg) and the supernatant from the second centrifugation was used for duplicate purification of cell-free DNA on a QlAsymphony system. The cell pellet obtained after the first centrifugation was resolved in 300µl PBS. 200µl of the resuspended cell pellet was used for extraction of the genomic DNA using the QIAamp Blood Mini kit. The remaining 100µl were centrifuged on a glass slide and stained with Papanicolaou (see Figure 7). Copy numbers of 18s rDNA in the ccfDNA were determined from duplicate samples in triplicates by real time PCR. Genomic DNA yield was measured on a Nanodrop instrument. The results are shown in Figures 5 to 7.

### Discussion

Multimodality testing, including cell-free and genomic DNA testing as well as conventional cytological staining, is possible with urine samples that are stabilized with the technology according to the present invention.

The cell-free DNA degrades rapidly in unstabilized urine but is preserved in urine stabilized according to the present invention. In unstabilized urine, genomic DNA yield can be dramatically reduced after storage. This is avoided by the present method. Furthermore, in unstabilized urine cell cytology can be destroyed after storage. However, the cytology is preserved when using the technology according to the present invention.

### 4. Example 4: Effect of pH on cell-free (cf) DNA preservation in urine

Urine from one male donor was centrifuged twice to remove all cells from the urine for spike in into female urine. 200ml urine each from six female donors were collected in large bottles and mixed with 25ml cell-free male urine directly after collection. Aliquots of 10ml urine (female plus male) were mixed with 1.7ml reagent. Cell-free DNA was isolated on a QIAsymphony system either directly after mixture with reagents (t0) or after four days incubation at room temperature. All samples were processed in duplicates. Real time PCR for y-chromosomale DYS14 DNA was performed from duplicate samples. The results are shown in Figure 8.

### Discussion

The results shown in Figure 8 show that cell-free DNA degrades rapidly in unstabilized urine or urine buffered with PBS. In contrast, the cell-free DNA is better preserved in urine stabilized with reagents according to the present invention. A basic pH further improved the stability of the cell-free DNA, as demonstrated by the male urine derived cell-free DNA spiked in female urine.

### 5. Example 5 - Effect of pH on cell-free DNA preservation in urine

Urine from six donors was collected. Aliquots of 10ml urine were mixed within 15min after sampling with 1.7ml stabilizing reagents R1, R8 (pH7), R9 (pH8), R10 (pH9), R11, R12 (pH8), R16, R17 (pH7), R18 (pH4) and R19 (pH3). The pH of the urine-reagent mixtures was determined either directly after mixture with reagents (t0) or after four (t4d) or seven (t7d) days incubation at room temperature (Tab. 2).

**Tab. 2: The pH of the 10 mL urine sample after addition of 1.7ml reagent were determined for 6 donors directly following addition of the respective reagents (t0) or 4 days (t4d) and in embodiments 7 days (t7d) after addition. Mean pH values and standard deviation (SD) are indicated.**

| **Reagent** | **t** | **Mean pH** | **SD** |
|---|---|---|---|
| **R1** | t0 | 4.9 | 0.2 |
| | t4d | 4.9 | 0.2 |
| | t7d | 4.9 | 0.2 |
| **R8 (pH7)** | t0 | 6.8 | 0.3 |
| | t4d | 6.6 | 0.1 |
| | t7d | 6.7 | 0.3 |
| **R9 (pH8)** | t0 | 7.3 | 0.4 |
| | t4d | 7.4 | 0.5 |
| | t7d | 7.3 | 0.6 |
| **R10 (pH9)** | t0 | 8.3 | 0.5 |
| | t4d | 8.4 | 0.5 |
| | t7d | 8.4 | 0.5 |
| **R11** | t0 | 5 | 0.2 |
| | t4d | 4.9 | 0.3 |
| | t7d | 4.9 | 0.2 |
| **R16** | t0 | 4.7 | 0.0 |
| | t4d | 4.7 | 0.0 |
| **R12 (pH8)** | t0 | 6.9 | 0.5 |
| | t4d | 7.0 | 0.5 |
| **R17 (pH7)** | t0 | 6.6 | 0.1 |
| | t4d | 6.6 | 0.1 |
| **R18 (pH4)** | t0 | 4.4 | 0.0 |
| | t4d | 4.4 | 0.0 |
| **R19 (pH3)** | t0 | 4.0 | 0.0 |
| | t4d | 4.0 | 0.0 |

Aliquots of 10ml urine were mixed within 15min after sampling with 1.7ml PBS or stabilizing reagents R1, R8 (pH7), R9 (pH8) and R10 (pH9). Cell-free DNA was isolated on the QIAsymphony either directly after mixture with reagents (t0) or after four (t4d) or seven (t7d) days incubation at room temperature. All samples were processed in duplicates. The copy numbers of 18S rDNA were determined from duplicate samples in triplicates by real time PCR. The results for the 66bp fragment are shown in Figure 9; the results for the 500bp fragment are shown in Figure 10.

While cell-free DNA degrades rapidly in unstabilized, PBS-buffered urine, the total amount of cell-free DNA, small as well as large molecules, is preserved in urine stabilized with reagents according to the invention.

Aliquots of 10ml urine were mixed within 15min after sampling with 1.7ml reagent R1, R8 (pH7), R9 (pH8) and R10 (pH9). Samples were processed immediately or stored after room temperature for 7 days. For processing samples were centrifuged 15min at 1900 xg. Supernatant was centrifuged again (10min 1900 xg). The cell pellets achieved from the first centrifugation were resolved in 200µl PBS and were used for extraction of the genomic DNA using the QIAamp Blood Mini kit. gDNA integrity was analyzed by agarose gelelectrophoresis (Fig. 11). The gDNA integrity obtained from urine samples stabilized with reagents set to an acidic pH (R1) was reduced after seven days storage compared to the yield from urine samples stabilized with stabilizing reagents set to a neutral (R8 (pH7)) or basic pH (R9 (pH8), R10 (pH9)).

To analyse the effect of pH on cell-free DNA preservation, urine from one male donor was centrifuged twice to remove all cells from the urine for spike in into female urine. 200ml urine each from four female donors were collected in large bottles and mixed with 25ml cell-free male urine directly after collection. Aliquots of 10ml urine (female plus male) were mixed with 1.7ml PBS or reagents R1, R8 (pH7), R9 (pH8) and R10 (pH9). Cell-free DNA was isolated on a QlAsymphony system either directly after mixture with reagents (t0) or after four (t4d) or seven (t7d) days incubation at room temperature. All samples were processed in duplicates. Real time PCR for y-chromosomal DYS14 DNA was performed from duplicate samples (Fig. 12).

To further analyse the effect of the pH on the stabilization of exfoliated cells and cell-free DNA in urine samples, reagents with even more acidic pH (R18 (pH4) and R19 (pH3)) were applied on urine samples. To this end, aliquots of 10ml urine were mixed within 15min after sampling with 1.7ml reagents R16, R12 (pH8), R17 (pH7), R18 (pH4) and R19 (pH3). Samples were processed immediately or stored at room temperature for 4 days. For processing samples were centrifuged 15min at 1900xg. Supernatant was centrifuged again (10 min at 1900xg) and the supernatant from the second centrifugation was used for duplicate purification of cell-free DNA on the QlAsymphony. The copy numbers of 18s rDNA were determined from duplicate samples in triplicates by real time PCR. The results for the 66bp fragment are shown in Fig. 14; the results for the 500bp fragment are shown in Fig. 15.

EDTA shows a significant decrease in the 500bp fragment (approx. 32 fold) while showing a small increase in the 66bp fragment (see Fig. 14 and 15). This indicates that larger fragments are more affected by degradation.

It was furthermore observed that reagents set to an acidic pH (R16, R18 (pH4) and R19 (pH3)) enabled after storage the recovery of less genomic DNA from the cells pellets as compared to stabilizing reagents set to a neutral or basic pH (see Figure 11). Furthermore, as shown elsewhere, the cell-free DNA already present in the urine at the time of collection and stabilization is significantly better preserved at non-acidic pH values as shown by the spike-in experiments.

To further analyse the effect of pH on cell-free DNA preservation, urine from one male donor was centrifuged twice to remove all cells from the urine for spike in into female urine. 200ml urine each from six female donors were collected in large bottles and mixed with 25ml cell-free male urine directly after collection. Aliquots of 10ml urine (female plus male) were mixed with 1.7ml reagent R16, R12 (pH8), R17 (pH7), R18 (pH4) and R19 (pH3). Cell-free DNA was isolated on a QIAsymphony system either directly after mixture with reagents (t0) or after four (t4d) days incubation at room temperature. All samples were processed in duplicates. Real time PCR for y-chromosomal DYS14 DNA was performed from duplicate samples (Fig. 16).

Cell-free DNA preservation was significantly improved in stabilizing reagents with neutral to basic pH compared to acidic pH.

### Discussion

The results show that cell-free DNA degrades more rapidly in urine samples stabilized with reagents set to an acidic pH value. The cell-free DNA comprised in the urine upon collection/stabilization is better preserved in urine stabilized with neutral or basic stabilizing compositions/reagents according to the present invention. A neutral or basic pH further improves the stabilization of cells contained within the urine sample and improves the obtainable yield of genomic DNA from the stabilized cells (see also Fig. 11).

Furthermore, the results of Figs. 14 to 16 indicate that the increase in DNA resulting from cells is advantageously reduced approx. 15-20fold when using the stabilization according to the invention compared to urine samples that were just stabilized with EDTA. For EDTA stabilized samples the overall increase in DNA can be deduced from the observed degradation (Dys 14 - see Fig. 16) and increase (18s - see Figs. 14 and 15). This is reflected in Table 3 below:

| | EDTA | R12/R17 |
|---|---|---|
| Delta Ct t0-t4d Dys 14 | ~2 | ~0.4 |
| Reduction | 4 fold | 1.3 fold (2^0.4) |
| Delta Ct t0-t4d 18s 66bp | 2 | 1 |
| Increase | 4 fold | 2 fold |
| Overall increase | 4x4 = 16 fold | 2 x 1.3 = 2.6 fold |

### 6. Urine processing workflow

The above experiments demonstrate that the stabilizing technology of the invention effectively stabilizes cell-free DNA in urine. It surprisingly also preserves the cytology of exfoliated, nucleated cells. These cells can thus be retrieved from a cell pellet after centrifugation of the stabilized urine and can be used for cytohistological test methods. Urine cells appear essentially unaffected and well preserved due to the stabilization, which was a rather unexpected observation. In addition to cell-free DNA and urine-cells, also genomic DNA of high quality can be isolated from such stabilized urine samples. For stabilization of urine for transport and short term storage it is advantageous to use an evacuated collection container, preferably a tube, comprising the stabilizing composition according to the third aspect, preferably in form of a solution. Preferably, the evacuated reception container is compatible with common urine collection devices, such as BD Vacutainer Urine Collection solutions (see background). Due to its advantages, the present stabilization technology enables a workflow which allows multimodality testing using one urine sample.

An exemplary urine processing workflow starts with the collection of human urine in a collection device (e.g. BD Vacutainer Urine Collection Cup, 120mL). The collected urine is then transferred into an preferably evacuated reception device comprising the stabilization composition according to the present invention (e.g. urine stabilization tube). Preferably, the transfer occurs directly from the BD Vacutainer Urine Collection Cup without the need to open the collection cup and to mix urine and reagent in a certain ratio, as this reduces handling errors and contamination risks. That an according direct transfer is possible with the method according to the invention, e.g. when using an evacuated reception device comprising the stabilizing agents, is an advantage over prior art urine stabilization devices (such as Streck). Urine could also be collected into any other container or bag and then transferred using a suitable device (e.g. with the BD Vacutainer Urine Transfer Straw) into the evacuated reception device comprising the stabilization composition according to the invention which effectively stabilizes the urine sample for transport and storage.

The stabilizing composition comprised in the evacuated collection tube stabilizes nucleated cells within the urine, prevents bacterial to grow and inhibits degradation of cell-free DNA. For purification of DNA, cells within the urine sample are separated, e.g. pelleted via centrifugation. Purification of gDNA from the separated cell sample or cell-free DNA from the cell-depleted supernatant can be performed using established purification methods, e.g. manually with QIAamp Kits such as the QIAamp Mini kit, QIAamp circulating Nucleic Acid Kit or QIAamp MinElute ccfDNA Kits as well as automated methods, such as e.g. the QS PAXgene Blood ccfDNA or QS DSP DNA Mini kit. Also other methods are well-known in the art.

Furthermore, the cell pellet or part of the cell pellet can be resuspended and centrifuged on a slide with i.e. a cyto-centrifuge for conventional cytological staining with e.g. Papanicolaou.

As discussed above, it is very important to rapidly stabilize urine samples, as cell-free DNA rapidly degrades in unstabilized urine. The stabilization composition according to the present invention achieves this result. As discussed above, a stabilization composition which comprises a high EDTA concentration and preferably has a pH of at least 7, such as at least 7.2, at least 7.3 or at least 7.5 is highly effective in stabilizing urine and in particular preserves the cell-free DNA from degradation. Good results were also achieved with stabilizing composition having a pH of at least 8 as demonstrated herein. Furthermore, the inhibition of bacterial growth that is achieved with the stabilization according to the invention is highly advantageous because it *inter alia* reduces the degradation of cell-free DNA comprised in the urine sample. This can be achieved e.g. by using a high concentration of EDTA and/or by including a preservative for inhibiting bacterial growth, such as sodium azide or ProClin.

According to one embodiment, the processing according to the present invention comprises the following modules, to cover the complete preanalytical urine workflow:
- A urine collection device. A suitable example is e.g. a BD Vacutainer Urine Collection Cup which can receive up to 120mL urine. Preferably, the container enables transfer of urine into a reception device comprising a stabilizing composition according to the invention, e.g. an evacuated tube, without the necessity to open the evacuated tube or the container. The urine collection device may also comprise one or more stabilizing agents, such as e.g. a chelating agent (preferably EDTA) and/or a preservative for inhibiting bacterial growth. Such stabilizing agents are preferably comprised in dry form, e.g. spray-dried form, in the primary urine collection device such as a urine cup.
- Optionally a transfer device that allows for the transfer of urine from a specimen cup or pediatric bag to one or more reception devices comprising the stabilizing composition, e.g. an evacuated tube, without exposure to the specimen. A suitable example is a BD Vacutainer Urine Collection Straw.
- A reception device for receiving the collected urine, which comprises the stabilizing agents used according to the present invention, preferably in from of a stabilizing composition according to the third aspect. The urine sample is stabilized in this reception device, which preferably is an evacuated tube suitable to draw a defined amount of urine into the tube. The reception tube is preferably made of shatter resistant plastic that guarantees safe shipment, storage and centrifugation of the urine sample at room temperature. It preferably includes a safety cap which prevents inside of the tube to spread around when opening the cap. According to one embodiment, an evacuated reception tube, e.g. an evacuated plastic BD Vacutainer tube, enables the draw of 10 ml urine. Advantageously, commercially available reception/collection and transfer devices can be used. The comprised stabilizing composition stabilizes cytology of exfoliated, nucleated cells in urine, genomic DNA within the nucleated cells as well as cell-free DNA in the urine sample at room temperature for transport and preferably short term storage. As discussed above, the stabilizing composition according to the invention may be free of crosslinking agents (such as formaldehyde or formaldehyde releasers) and in particular prevents growing of bacteria and degradation of DNA (i.e. cfDNA). It furthermore allows the subsequent cytological analysis of comprised cells. Preferably, the stabilizing composition is a liquid stabilizing composition, such as a solution.

After stabilization of the urine sample, the stabilized urine sample can be further processed. According to one embodiment, cells are separated from the stabilized urine sample to provide (i) a cell sample and (ii) a cell-depleted supernatant. The obtained cell sample and/or the supernatant can be further processed. The work-flow may comprise one or more of the following:
(aa) Purification of cell-free DNA from the cell-depleted or cell-free supernatant. Here, common methods may be used and commercial kits are also available (e.g. QIAamp Circulating Nucleic Acid Kit for manual purification of cfDNA from urine; QIAamp MinElute ccfDNA Kits for manual purification of cfDNA from urine with use of a centrifuge, and automated on QlAcube and EZ1; QIAsymphony PAXgene Blood ccfDNA kit for purification of cfDNA from urine).
(bb) Purification of genomic DNA from separated cells. Again, common methods for genomic DNA purification may be used and such method are also commercially available (e.g. QIAamp Mini Kit for purification of gDNA from urine; QIAsymphony DPS Mini kit for purification of gDNA from urine).
(cc) Cytology analyses of separated cells. E.g. a urine exfoliated cell preparation for cytology analyses may be prepared from the obtained cell pellet.

The isolated DNA (cell-free DNA and/or gDNA) can be analysed by analytical methods such as real-time PCR, sequencing (e.g. next generation sequencing) or other analytical techniques. This workflow for urine collection, stabilization and processing advantageously enables multimodality testing with one sample.

## Claims

1. A method for stabilizing a urine sample, in particular extracellular DNA comprised therein, the method comprising contacting the urine sample for stabilization with a stabilizing composition which comprises a chelating agent and at least one poly(oxyethylene) polymer, wherein bacterial growth is inhibited in the stabilized urine sample,
wherein the stabilizing composition has a pH in the range of > 7 to ≤ 9.5,
and wherein the stabilization does not involve the use of a cross-linking agent that induces protein-nucleic acid and/or protein-protein crosslinks such as formaldehyde, formaline, paraformaldehyde or a formaldehyde releaser.

2. The method according to claim 1, wherein the urine sample is contacted with a preservative for inhibiting bacterial growth,
optionally wherein the preservative for inhibiting bacterial growth is a preservative that has one or more of the following characteristics:
(a) it is an antimicrobial and preferably has antibacterial and antifungal effects;
(b) the preservative comprises or consists of a chemical compound or two or more chemical compounds;
(c) the preservative comprises or consists of sodium azide;
(d) the preservative comprises at least one isothiazolinone compound, preferably methylchloroisothiazolinone and/or methylisothiazolinone;
(e) the preservative comprises chlorhexidine;
(f) the preservative comprises sodium borate.

3. The method according to claim 1 or 2, wherein the chelating agent is EDTA.

4. The method according to claim 2 or 3, wherein the stabilizing composition comprises the preservative for inhibiting bacterial growth.

5. The method according to one or more of claims 1 to 4, wherein:
(i) the stabilizing composition has a pH in the range of 7.2 to 9.5, 7.3 to 9.5, 7.4 to 9.5 or 7.5 to 9.5;
(ii) the stabilizing composition comprises a buffering agent;
(iii) the stabilizing composition comprises a buffering agent and has a pH in the range of 7.2 to 9.5, 7.3 to 9.5, 7.4 to 9.5 or 7.5 to 9.5;
(iv) the stabilizing composition is contacted with the urine sample in a volumetric ratio selected from 1:3 to 1:10, preferably 1:5 to 1:8; and/or
(v) the pH of the stabilized urine sample lies in a range of 6 to 9.5, such as 6.3 to 9.5, 6.5 to 9.5, 6.7 to 9, 7 to 9 or 7.2 to 9.

6. The method according to one or more of claims 3 to 5, having one or more of the following characteristics:
(i) wherein in the stabilized urine sample, the final concentration of EDTA lies in a range of 2mg/ml to 40 mg/ml, preferably 10mg/ml to 30mg/ml;
(ii) the method comprises contacting the urine sample with a stabilizing composition that comprises EDTA in a final concentration that lies in a range of 100mM up to the solubility limit, preferably 150mM to 500mM.

7. The method according to one or more of claims 1 to 6, wherein:
(i) the at least one poly(oxyethylene) polymer is a polyethylene glycol; and/or
(ii) the urine sample is contacted with a high molecular weight poly(oxyethylene) polymer having a molecular weight of at least 1500 and a low molecular weight poly(oxyethylene) polymer having a molecular weight of 1000 or less and wherein preferably these polymers are comprised in the stabilization composition according to claim 1.

8. The method according to one or more of claims 1 to 7, wherein the urine sample is additionally contacted with at least one caspase inhibitor and wherein preferably, the caspase inhibitor is comprised in the stabilizing composition according to claim 1.

9. The method according to one or more of claims 1 to 8, wherein the stabilizing composition comprises:
- EDTA,
- at least one polyethylene glycol, preferably at least one high molecular weight polyethylene glycol having a molecular weight of at least 3000 and optionally at least one low molecular weight polyethylene glycol having a molecular weight of 1000 or less,
- a preservative for inhibiting bacterial growth,
- a buffering agent,
- at least one caspase inhibitor, and
- optionally at least one primary, secondary or tertiary amide.

10. The method according to claim 9, wherein the stabilizing composition is a liquid composition comprising:
- EDTA in a concentration of 100mM up to the solubility limit, preferably 150mM to 500mM,
- at least one polyethylene glycol, preferably at least one high molecular weight polyethylene glycol having a molecular weight of at least 6000 and optionally at least one low molecular weight polyethylene glycol having a molecular weight of 1000 or less,
- a preservative for inhibiting bacterial growth,
- a buffering agent,
- at least one caspase inhibitor, and
- optionally at least one primary, secondary or tertiary amide,
wherein the pH of the stabilizing composition lies in the range of > 7 to 9.5, and wherein the stabilizing composition is contacted with the urine sample in a volumetric ratio selected from 1:3 to 1:10, preferably 1:5 to 1:8.

11. The method according to one or more of claims 1 to 10, wherein the stabilizing composition has one or more of the following characteristics:
i) the stabilizing composition stabilizes extracellular DNA comprised in the urine sample against degradation;
ii) the stabilizing composition stabilizes cytology of exfoliated, nucleated cells in the urine sample, genomic DNA within the nucleated cells and extracellular DNA in the urine sample;
iii) the stabilizing composition stabilizes extracellular RNA in addition to extracellular DNA.

12. The method according to one or more of claims 1 to 11, wherein the urine sample, which may have been first collected in a different device, is subsequently contacted for stabilization exclusively with the stabilizing composition, whereby the stabilizing conditions are established by the stabilizing composition.

13. The method according to one or more of claims 1 to 12, wherein, after a stabilization period:
(i) extracellular nucleic acids, preferably cell-free DNA, is/are isolated from the stabilized urine sample, and optionally are further analyzed and/or detected; and/or
(ii) cells comprised in the stabilized urine sample are removed.

14. The method according to claim 13, wherein cells comprised in the stabilized urine sample are removed, and wherein:
(i) cell-free DNA is isolated from the cell-free or cell-depleted portion of the stabilized sample;
(ii) nucleic acids, preferably genomic DNA, are isolated from cells that were removed from the stabilized urine sample; and/or
(iii) removed cells are analyzed using a cytological method.

15. A method for processing a urine sample, comprising the steps of
a) stabilizing the urine sample according to the method defined in one or more of claims 1 to 11; and
b) separating cells from the stabilized urine sample, thereby providing (i) a cell sample and (ii) a cell-depleted supernatant;
optionally wherein the method further comprises:
- purifying extracellular DNA from the cell-depleted supernatant;
- purifying genomic DNA from the obtained cell sample, and/or
- preparing exfoliated cells for cytology from the obtained cell sample.

16. Use of a stabilizing composition for stabilizing a urine sample, wherein the stabilizing composition is suitable for stabilizing a urine sample, in particular extracellular DNA comprised therein, wherein bacterial growth is inhibited in the stabilized urine sample, the composition comprising
- a chelating agent, preferably EDTA,
- at least one poly(oxyethylene) polymer, and optionally
- a preservative for inhibiting bacterial growth,
wherein the stabilizing composition has a pH in the range of > 7 to ≤ 9.5,
and wherein the stabilizing composition does not comprise a cross-linking agent that induces protein-DNA and/or protein-protein crosslinks such as formaldehyde, formaline, paraformaldehyde or a formaldehyde releaser.

17. The use according to claim 16, wherein:
(i) the stabilizing composition comprises a preservative for inhibiting bacterial growth, wherein preferably the preservative has one or more of the characteristics as defined in claim 2;
(ii) the stabilizing composition has a pH in the range of 7.2 to 9.5, 7.3 to 9.5, 7.4 to 9.5 or 7.5 to 9.5;
(iii) the stabilizing composition comprises a buffering agent;
(iv) the stabilizing composition comprises a buffering agent and has a pH in the range of 7.2 to 9.5, 7.3 to 9.5, 7.4 to 9.5 or 7.5 to 9.5;
(v) the poly(oxyethylene) polymer is a polyethylene glycol, wherein optionally, the stabilizing composition comprises at least one high molecular weight polyethylene glycol having a molecular weight of at least 3000 and optionally at least one low molecular weight polyethylene glycol having a molecular weight of 1000 or less;
(vi) the stabilizing composition comprises at least one caspase inhibitor;
(vii) the stabilizing composition has the characteristics as defined in claim 9, 10 or 11; and/or
(viii) the stabilizing composition is liquid and comprises a chelating agent, preferably EDTA, in a concentration of at least 15 mM, optionally in a concentration of 100 mM up to the solubility limit.

18. A stabilizing composition, wherein the stabilizing composition is mixed with a urine sample,
wherein the stabilizing composition is a stabilizing composition for stabilizing a urine sample, in particular extracellular DNA comprised therein, wherein bacterial growth is inhibited in the stabilized urine sample, the composition comprising
- a chelating agent, preferably EDTA,
- at least one poly(oxyethylene) polymer, and optionally
- a preservative for inhibiting bacterial growth,
wherein the stabilizing composition has a pH in the range of > 7 to ≤ 9.5,
and wherein the stabilizing composition does not comprise a cross-linking agent that induces protein-DNA and/or protein-protein crosslinks such as formaldehyde, formaline, paraformaldehyde or a formaldehyde releaser,
optionally wherein the stabilizing composition has one or more of the following characteristics:
(i) the stabilizing composition comprises a preservative for inhibiting bacterial growth, wherein preferably the preservative has one or more of the characteristics as defined in claim 2;
(ii) the stabilizing composition has a pH in the range of 7.2 to 9.5, 7.3 to 9.5, 7.4 to 9.5 or 7.5 to 9.5;
(iii) the stabilizing composition comprises a buffering agent;
(iv) the stabilizing composition comprises a buffering agent and has a pH in the range of 7.2 to 9.5, 7.3 to 9.5, 7.4 to 9.5 or 7.5 to 9.5;
(v) the poly(oxyethylene) polymer is a polyethylene glycol, wherein optionally, the stabilizing composition comprises at least one high molecular weight polyethylene glycol having a molecular weight of at least 3000 and optionally at least one low molecular weight polyethylene glycol having a molecular weight of 1000 or less;
(vi) the stabilizing composition comprises at least one caspase inhibitor;
(vii) the stabilizing composition has the characteristics as defined in claim 9, 10 or 11; and/or
(viii) the stabilizing composition is liquid and comprises a chelating agent, preferably EDTA, in a concentration of at least 15 mM, optionally in a concentration of 100 mM up to the solubility limit.

## Patentansprüche

1. Verfahren zur Stabilisierung einer Urinprobe, insbesondere der darin enthaltenen extrazellulären DNA, wobei das Verfahren das Inkontaktbringen der Urinprobe zur Stabilisierung mit einer Stabilisierungszusammensetzung umfasst, die einen Chelator und mindestens ein Poly(oxyethylen)-Polymer enthält, wobei das Bakterienwachstum in der stabilisierten Urinprobe gehemmt wird,
wobei die Stabilisierungszusammensetzung einen pH-Wert im Bereich von > 7 bis ≤ 9,5 aufweist, und wobei die Stabilisierung nicht die Verwendung eines Vernetzungsmittels beinhaltet, das Protein-Nukleinsäure- und/oder Protein-Protein-Vernetzungen induziert, wie beispielsweise Formaldehyd, Formalin, Paraformaldehyd oder einen Formaldehyd-Freisetzer.

2. Verfahren nach Anspruch 1, wobei die Urinprobe mit einem Konservierungsmittel zur Hemmung des Bakterienwachstums in Kontakt gebracht wird,
wobei optional das Konservierungsmittel zur Hemmung des Bakterienwachstums ein Konservierungsmittel ist, das eine oder mehrere der folgenden Eigenschaften aufweist:
(a) es ist ein antimikrobielles Mittel und weist vorzugsweise antibakterielle und antimykotische Wirkungen auf;
(b) das Konservierungsmittel umfasst oder besteht aus einer chemischen Verbindung oder zwei oder mehr chemischen Verbindungen;
(c) das Konservierungsmittel umfasst oder besteht aus Natriumazid;
(d) das Konservierungsmittel mindestens eine Isothiazolinon-Verbindung, vorzugsweise Methylchloroisothiazolinon und/oder Methylisothiazolinon, umfasst;
(e) das Konservierungsmittel Chlorhexidin umfasst;
(f) das Konservierungsmittel Natriumborat umfasst.

3. Verfahren nach Anspruch 1 oder 2, wobei der Chelator EDTA ist.

4. Verfahren nach Anspruch 2 oder 3, wobei die Stabilisierungszusammensetzung das Konservierungsmittel zur Hemmung des Bakterienwachstums enthält.

5. Verfahren nach einem oder mehreren der Ansprüche 1 bis 4, wobei:
(i) die Stabilisierungszusammensetzung einen pH-Wert im Bereich von 7,2 bis 9,5, 7,3 bis 9,5, 7,4 bis 9,5 oder 7,5 bis 9,5 aufweist;
(ii) die Stabilisierungszusammensetzung eine Puffersubstanz enthält;
(iii) die Stabilisierungszusammensetzung eine Puffersubstanz umfasst und einen pH-Wert im Bereich von 7,2 bis 9,5, 7,3 bis 9,5, 7,4 bis 9,5 oder 7,5 bis 9,5 aufweist;
(iv) die Stabilisierungszusammensetzung mit der Urinprobe in einem Volumenverhältnis in Kontakt gebracht wird, das im Bereich von 1:3 bis 1:10, vorzugsweise von 1:5 bis 1:8, liegt; und/oder
(v) der pH-Wert der stabilisierten Urinprobe in einem Bereich von 6 bis 9,5, beispielsweise von 6,3 bis 9,5, von 6,5 bis 9,5, von 6,7 bis 9, von 7 bis 9 oder von 7,2 bis 9 liegt.

6. Verfahren nach einem oder mehreren der Ansprüche 3 bis 5, das eines oder mehrere der folgenden Merkmale aufweist:
(i) wobei in der stabilisierten Urinprobe die Endkonzentration von EDTA in einem Bereich von 2 mg/ml bis 40 mg/ml, vorzugsweise von 10 mg/ml bis 30 mg/ml, liegt;
(ii) das Verfahren umfasst das Inkontaktbringen der Urinprobe mit einer Stabilisierungszusammensetzung, die EDTA in einer Endkonzentration enthält, die in einem Bereich von 100 mM bis zur Löslichkeitsgrenze, vorzugsweise von 150 mM bis 500 mM, liegt.

7. Verfahren nach einem oder mehreren der Ansprüche 1 bis 6, wobei:
(i) das mindestens eine Poly(oxyethylen)-Polymer ein Polyethylenglykol ist; und/oder
(ii) die Urinprobe mit einem hochmolekularen Poly(oxyethylen)-Polymer mit einem Molekulargewicht von mindestens 1500 und einem niedermolekularen Poly(oxyethylen)-Polymer mit einem Molekulargewicht von 1000 oder weniger in Kontakt gebracht wird, wobei diese Polymere vorzugsweise in der Stabilisierungszusammensetzung gemäß Anspruch 1 enthalten sind.

8. Verfahren nach einem oder mehreren der Ansprüche 1 bis 7, wobei die Urinprobe zusätzlich mit mindestens einem Caspase-Inhibitor in Kontakt gebracht wird und wobei der Caspase-Inhibitor vorzugsweise in der Stabilisierungszusammensetzung nach Anspruch 1 enthalten ist.

9. Verfahren nach einem oder mehreren der Ansprüche 1 bis 8, wobei die Stabilisierungszusammensetzung umfasst:
- EDTA,
- mindestens ein Polyethylenglykol, vorzugsweise mindestens ein hochmolekulares Polyethylenglykol mit einem Molekulargewicht von mindestens 3000 und gegebenenfalls mindestens ein niedermolekulares Polyethylenglykol mit einem Molekulargewicht von 1000 oder weniger,
- ein Konservierungsmittel zur Hemmung des Bakterienwachstums,
- eine Puffersubstanz,
- mindestens einen Caspase-Inhibitor und
- optional mindestens ein primäres, sekundäres oder tertiäres Amid.

10. Verfahren nach Anspruch 9, wobei die Stabilisierungszusammensetzung eine flüssige Zusammensetzung ist, die Folgendes umfasst:
- EDTA in einer Konzentration von 100 mM bis zur Löslichkeitsgrenze, vorzugsweise 150 mM bis 500 mM,
- mindestens ein Polyethylenglykol, vorzugsweise mindestens ein hochmolekulares Polyethylenglykol mit einem Molekulargewicht von mindestens 6000 und gegebenenfalls mindestens ein niedermolekulares Polyethylenglykol mit einem Molekulargewicht von 1000 oder weniger,
- ein Konservierungsmittel zur Hemmung des Bakterienwachstums,
- eine Puffersubstanz,
- mindestens einen Caspase-Inhibitor, und
- optional mindestens ein primäres, sekundäres oder tertiäres Amid,
wobei der pH-Wert der Stabilisierungszusammensetzung im Bereich von > 7 bis 9,5 liegt und wobei die Stabilisierungszusammensetzung mit der Urinprobe in einem Volumenverhältnis von 1:3 bis 1:10, vorzugsweise von 1:5 bis 1:8, in Kontakt gebracht wird.

11. Verfahren nach einem oder mehreren der Ansprüche 1 bis 10, wobei die Stabilisierungszusammensetzung eine oder mehrere der folgenden Eigenschaften aufweist:
i) die Stabilisierungszusammensetzung schützt die in der Urinprobe enthaltene extrazelluläre DNA vor Degradation;
ii) die Stabilisierungszusammensetzung stabilisiert die Zytologie der abgeschilferten, kernhaltigen Zellen in der Urinprobe, die genomische DNA innerhalb der kernhaltigen Zellen sowie die extrazelluläre DNA in der Urinprobe;
iii) die Stabilisierungszusammensetzung stabilisiert neben der extrazellulären DNA auch die extrazelluläre RNA.

12. Verfahren nach einem oder mehreren der Ansprüche 1 bis 11, wobei die Urinprobe, die möglicherweise zuerst in einer anderen Vorrichtung gesammelt wurde, anschließend zur Stabilisierung ausschließlich mit der Stabilisierungszusammensetzung in Kontakt gebracht wird, wodurch die Stabilisierungsbedingungen durch die Stabilisierungszusammensetzung hergestellt werden.

13. Verfahren nach einem oder mehreren der Ansprüche 1 bis 12, wobei nach einer Stabilisierungsperiode:
(i) extrazelluläre Nukleinsäuren, vorzugsweise zellfreie DNA, aus der stabilisierten Urinprobe isoliert und gegebenenfalls weiter analysiert und/oder nachgewiesen werden; und/oder
(ii) die in der stabilisierten Urinprobe enthaltenen Zellen entfernt werden.

14. Verfahren nach Anspruch 13, wobei die in der stabilisierten Urinprobe enthaltenen Zellen entfernt werden und wobei:
(i) die zellfreie DNA aus dem zellfreien oder zelldepletierten Anteil der stabilisierten Probe isoliert wird;
(ii) Nukleinsäuren, vorzugsweise genomische DNA, aus Zellen isoliert werden, die aus der stabilisierten Urinprobe entnommen wurden; und/oder
(iii) die entnommenen Zellen mittels einer zytologischen Methode untersucht werden.

15. Verfahren zur Prozessierung einer Urinprobe, umfassend die folgenden Schritte:
a) Stabilisierung der Urinprobe gemäß dem in einem oder mehreren der Ansprüche 1 bis 11 definierten Verfahren; und
b) Abtrennung von Zellen aus der stabilisierten Urinprobe, wodurch (i) eine Zellprobe und (ii) ein zellfreier Überstand gewonnen werden;
wobei das Verfahren optional ferner umfasst:
- Reinigung extrazellulärer DNA aus dem zelldepletierten Überstand;
- Aufreinigung der genomischen DNA aus der gewonnenen Zellprobe und/oder
- Aufbereitung der abgeschilferten Zellen für die Zytologie aus der entnommenen Zellprobe.

16. Verwendung einer Stabilisierungszusammensetzung zur Stabilisierung einer Urinprobe, wobei die Stabilisierungszusammensetzung zur Stabilisierung einer Urinprobe, insbesondere der darin enthaltenen extrazellulären DNA, geeignet ist, wobei das Bakterienwachstum in der stabilisierten Urinprobe gehemmt wird, wobei die Zusammensetzung umfasst
- ein Chelator, vorzugsweise EDTA,
- mindestens ein Poly(oxyethylen)-Polymer und gegebenenfalls
- ein Konservierungsmittel zur Hemmung des Bakterienwachstums,
wobei die Stabilisierungszusammensetzung einen pH-Wert im Bereich von > 7 bis ≤ 9,5 aufweist,
und wobei die Stabilisierungszusammensetzung kein Vernetzungsmittel enthält, das Protein-DNA- und/oder Protein-Protein-Vernetzungen induziert, wie beispielsweise Formaldehyd, Formalin, Paraformaldehyd oder einen Formaldehyd-Freisetzer.

17. Verwendung gemäß Anspruch 16, wobei:
(i) die Stabilisierungszusammensetzung ein Konservierungsmittel zur Hemmung des Bakterienwachstums umfasst, wobei das Konservierungsmittel vorzugsweise eine oder mehrere der in Anspruch 2 definierten Eigenschaften aufweist;
(ii) die Stabilisierungszusammensetzung einen pH-Wert im Bereich von 7,2 bis 9,5, 7,3 bis 9,5, 7,4 bis 9,5 oder 7,5 bis 9,5 aufweist;
(iii) die Stabilisierungszusammensetzung eine Puffersubstanz enthält;
(iv) die Stabilisierungszusammensetzung enthält eine Puffersubstanz und weist einen pH-Wert im Bereich von 7,2 bis 9,5, 7,3 bis 9,5, 7,4 bis 9,5 oder 7,5 bis 9,5 auf;
(v) das Poly(oxyethylen)-Polymer ein Polyethylenglykol ist, wobei die Stabilisierungszusammensetzung gegebenenfalls mindestens ein hochmolekulares Polyethylenglykol mit einem Molekulargewicht von mindestens 3000 und gegebenenfalls mindestens ein niedermolekulares Polyethylenglykol mit einem Molekulargewicht von 1000 oder weniger umfasst;
(vi) die Stabilisierungszusammensetzung umfasst mindestens einen Caspase-Inhibitor;
(vii) die Stabilisierungszusammensetzung die in Anspruch 9, 10 oder 11 definierten Merkmale aufweist; und/oder
(viii) die Stabilisierungszusammensetzung flüssig ist und einen Chelator enthält, vorzugsweise EDTA, in einer Konzentration von mindestens 15 mM, gegebenenfalls in einer Konzentration von 100 mM bis zur Löslichkeitsgrenze.

18. Eine Stabilisierungszusammensetzung, wobei die Stabilisierungszusammensetzung mit einer Urinprobe vermischt wird,
wobei es sich bei der Stabilisierungszusammensetzung um eine Zusammensetzung zur Stabilisierung einer Urinprobe, insbesondere der darin enthaltenen extrazellulären DNA, handelt, wobei in der stabilisierten Urinprobe das Bakterienwachstum gehemmt wird, wobei die Zusammensetzung umfasst
- ein Chelator, vorzugsweise EDTA,
- mindestens ein Poly(oxyethylen)-Polymer und gegebenenfalls
- ein Konservierungsmittel zur Hemmung des Bakterienwachstums,
wobei die Stabilisierungszusammensetzung einen pH-Wert im Bereich von > 7 bis ≤ 9,5 aufweist,
und wobei die Stabilisierungszusammensetzung kein Vernetzungsmittel enthält, das Protein-DNA- und/oder Protein-Protein-Vernetzungen induziert, wie beispielsweise Formaldehyd, Formalin, Paraformaldehyd oder einen Formaldehyd-Freisetzer,
wobei die Stabilisierungszusammensetzung optional eine oder mehrere der folgenden Eigenschaften aufweist:
(i) die stabilisierende Chelator umfasst ein Konservierungsmittel zur Hemmung des Bakterienwachstums, wobei das Konservierungsmittel vorzugsweise eine oder mehrere der in Anspruch 2 definierten Eigenschaften aufweist;
(ii) die stabilisierende Chelator weist einen pH-Wert im Bereich von 7,2 bis 9,5, 7,3 bis 9,5, 7,4 bis 9,5 oder 7,5 bis 9,5 auf;
(iii) die Stabilisierungszusammensetzung enthält eine Puffersubstanz;
(iv) die Stabilisierungszusammensetzung enthält eine Puffersubstanz und weist einen pH-Wert im Bereich von 7,2 bis 9,5, 7,3 bis 9,5, 7,4 bis 9,5 oder 7,5 bis 9,5 auf;
(v) das Poly(oxyethylen)-Polymer ist ein Polyethylenglykol, wobei die Stabilisierungszusammensetzung gegebenenfalls mindestens ein hochmolekulares Polyethylenglykol mit einem Molekulargewicht von mindestens 3000 und gegebenenfalls mindestens ein niedermolekulares Polyethylenglykol mit einem Molekulargewicht von 1000 oder weniger umfasst;
(vi) die Stabilisierungszusammensetzung umfasst mindestens einen Caspase-Inhibitor;
(vii) die Stabilisierungszusammensetzung weist die in Anspruch 9, 10 oder 11 definierten Merkmale auf; und/oder
(viii) die Stabilisierungszusammensetzung ist flüssig und enthält einen Chelator, vorzugsweise EDTA, in einer Konzentration von mindestens 15 mM, gegebenenfalls in einer Konzentration von 100 mM bis zur Löslichkeitsgrenze.

## Revendications

1. Procédé de stabilisation d'un échantillon d'urine, en particulier de l'ADN extracellulaire compris dans celui-ci, le procédé comprenant la mise en contact de l'échantillon d'urine pour la stabilisation avec une composition de stabilisation qui comprend un agent chélateur et au moins un polymère de poly(oxyéthylène), lequel inhibe la croissance bactérienne dans l'échantillon d'urine stabilisé,
dans lequel la composition de stabilisation a un pH dans la plage de > 7 à ≤ 9,5,
et dans lequel la stabilisation n'implique pas l'utilisation d'un agent de réticulation qui induit des liaisons croisées protéine-acide nucléique et/ou protéine-protéine tel que le formaldéhyde, la formaline, le paraformaldéhyde ou un libérateur de formaldéhyde.

2. Procédé selon la revendication 1, dans lequel l'échantillon d'urine est mis en contact avec un agent de conservation pour inhiber la croissance bactérienne,
éventuellement dans lequel l'agent de conservation pour inhiber la croissance bactérienne est un agent de conservation qui présente une ou plusieurs des caractéristiques suivantes :
(a) il est un agent antimicrobien et présente de préférence des effets antibactériens et antifongiques;
(b) l'agent de conservation comprend ou consiste en un composé chimique ou deux ou plusieurs composés chimiques ;
(c) l'agent de conservation comprend ou consiste en de l'azoture de sodium ;
(d) l'agent de conservation comprend au moins un composé d'isothiazolinone, de préférence la méthylchloroisothiazolinone et/ou la méthylisothiazolinone ;
(e) l'agent de conservation comprend la chlorhexidine ;
(f) l'agent de conservation comprend le borate de sodium.

3. Procédé selon la revendication 1 ou 2, dans lequel l'agent chélateur est l'EDTA.

4. Procédé selon la revendication 2 ou 3, dans lequel la composition de stabilisation comprend l'agent de conservation pour inhiber la croissance bactérienne.

5. Procédé selon l'une quelconque des revendications 1 à 4, dans lequel :
(i) la composition de stabilisation a un pH dans la plage de 7,2 à 9,5, 7,3 à 9,5, 7,4 à 9,5 ou 7,5 à 9,5 ;
(ii) la composition de stabilisation comprend un agent tampon ;
(iii) la composition de stabilisation comprend un agent tampon et a un pH dans la plage de 7,2 à 9,5, 7,3 à 9,5, 7,4 à 9,5 ou 7,5 à 9,5 ;
(iv) la composition de stabilisation est mise en contact avec l'échantillon d'urine dans un rapport volumique choisi parmi 1:3 à 1:10, de préférence 1:5 à 1:8 ; et/ou
(v) le pH de l'échantillon d'urine stabilisé se situe dans une plage de 6 à 9,5, tel que 6,3 à 9,5, 6,5 à 9,5, 6,7 à 9, 7 à 9 ou 7,2 à 9.

6. Procédé selon l'une quelconque des revendications 3 à 5, présentant une ou plusieurs des caractéristiques suivantes :
(i) dans lequel, dans l'échantillon d'urine stabilisé, la concentration finale d'EDTA se situe dans une plage de 2 mg/ml à 40 mg/ml, de préférence 10 mg/ml à 30 mg/ml ;
(ii) le procédé comprend la mise en contact de l'échantillon d'urine avec une composition de stabilisation qui comprend de l'EDTA dans une concentration finale qui se situe dans une plage de 100 mM jusqu'à la limite de solubilité, de préférence 150 mM à 500 mM.

7. Procédé selon l'une quelconque des revendications 1 à 6, dans lequel :
(i) le ou les polymères de poly(oxyéthylène) sont un polyéthylène glycol ; et/ou
(ii) l'échantillon d'urine est mis en contact avec un polymère de poly(oxyéthylène) de haut poids moléculaire ayant un poids moléculaire d'au moins 1500 et un polymère de poly(oxyéthylène) de bas poids moléculaire ayant un poids moléculaire de 1000 ou moins et, de préférence, ces polymères sont compris dans la composition de stabilisation selon la revendication 1.

8. Procédé selon l'une quelconque des revendications 1 à 7, dans lequel l'échantillon d'urine est en outre mis en contact avec au moins un inhibiteur de caspase et, de préférence, l'inhibiteur de caspase est compris dans la composition de stabilisation selon la revendication 1.

9. Procédé selon l'une quelconque des revendications 1 à 8, dans lequel la composition de stabilisation comprend :
- de l'EDTA,
- au moins un polyéthylène glycol, de préférence au moins un polyéthylène glycol de haut poids moléculaire ayant un poids moléculaire d'au moins 3000 et éventuellement au moins un polyéthylène glycol de bas poids moléculaire ayant un poids moléculaire de 1000 ou moins,
- un agent de conservation pour inhiber la croissance bactérienne,
- un agent tampon,
- au moins un inhibiteur de caspase, et
- éventuellement au moins un amide primaire, secondaire ou tertiaire.

10. Procédé selon la revendication 9, dans lequel la composition de stabilisation est une composition liquide comprenant :
- de l'EDTA dans une concentration de 100 mM jusqu'à la limite de solubilité, de préférence 150 mM à 500 mM,
- au moins un polyéthylène glycol, de préférence au moins un polyéthylène glycol de haut poids moléculaire ayant un poids moléculaire d'au moins 6000 et éventuellement au moins un polyéthylène glycol de bas poids moléculaire ayant un poids moléculaire de 1000 ou moins,
- un agent de conservation pour inhiber la croissance bactérienne,
- un agent tampon,
- au moins un inhibiteur de caspase, et
- éventuellement au moins un amide primaire, secondaire ou tertiaire,
dans lequel le pH de la composition de stabilisation se situe dans la plage de > 7 à 9,5, et dans lequel la composition de stabilisation est mise en contact avec l'échantillon d'urine dans un rapport volumique choisi parmi 1:3 à 1:10, de préférence 1:5 à 1:8.

11. Procédé selon l'une quelconque des revendications 1 à 10, dans lequel la composition de stabilisation présente une ou plusieurs des caractéristiques suivantes :
i) la composition de stabilisation stabilise l'ADN extracellulaire compris dans l'échantillon d'urine contre la dégradation ;
ii) la composition de stabilisation stabilise la cytologie des cellules exfoliées et nucléées dans l'échantillon d'urine, l'ADN génomique à l'intérieur des cellules nucléées et l'ADN extracellulaire dans l'échantillon d'urine ;
iii) la composition de stabilisation stabilise l'ARN extracellulaire en plus de l'ADN extracellulaire.

12. Procédé selon l'une quelconque des revendications 1 à 11, dans lequel l'échantillon d'urine, qui peut avoir été d'abord recueilli dans un dispositif différent, est ensuite mis en contact pour la stabilisation exclusivement avec la composition de stabilisation, les conditions de stabilisation étant ainsi définies par ladite composition stabilisante.

13. Procédé selon l'une quelconque des revendications 1 à 12, dans lequel, après une période de stabilisation:
(i) des acides nucléiques extracellulaires, de préférence de l'ADN libre de cellule, sont isolés de l'échantillon d'urine stabilisé, et éventuellement sont en outre analysés et/ou détectés ; et/ou
(ii) les cellules comprises dans l'échantillon d'urine stabilisé sont éliminées.

14. Procédé selon la revendication 13, dans lequel les cellules comprises dans l'échantillon d'urine stabilisé sont éliminées, et dans lequel :
(i) l'ADN libre de cellule est isolé de la fraction sans cellule ou du surnageant appauvri en cellules de l'échantillon stabilisé ;
(ii) des acides nucléiques, de préférence de l'ADN génomique, sont isolés à partir des cellules qui ont été éliminées de l'échantillon d'urine stabilisé ; et/ou
(iii) les cellules éliminées sont analysées à l'aide d'une méthode cytologique.

15. Procédé de traitement d'un échantillon d'urine, comprenant les étapes de
a) stabilisation de l'échantillon d'urine selon le procédé défini dans l'une quelconque des revendications 1 à 11 ; et
b) séparation des cellules de l'échantillon d'urine stabilisé, fournissant ainsi (i) un échantillon de cellules et (ii) un surnageant appauvri en cellules ;
éventuellement dans lequel le procédé comprend en outre :
- la purification de l'ADN extracellulaire à partir du surnageant appauvri en cellules ;
- la purification de l'ADN génomique à partir de l'échantillon de cellules obtenu, et/ou
- la préparation de cellules exfoliées pour la cytologie à partir de l'échantillon de cellules obtenu.

16. Utilisation d'une composition de stabilisation pour la stabilisation d'un échantillon d'urine, dans laquelle la composition de stabilisation est adaptée à la stabilisation d'un échantillon d'urine, en particulier de l'ADN extracellulaire compris dans celui-ci, dans lequel la croissance bactérienne est inhibée dans l'échantillon d'urine stabilisé, la composition comprenant
- un agent chélateur, de préférence l'EDTA,
- au moins un polymère de poly(oxyéthylène), et éventuellement
- un agent de conservation pour inhiber la croissance bactérienne,
dans lequel la composition de stabilisation a un pH dans la plage de > 7 à ≤ 9,5,
et dans lequel la composition de stabilisation ne comprend pas d'agent de réticulation qui induit des liaisons croisées protéine-ADN et/ou protéine-protéine tel que le formaldéhyde, la formaline, le paraformaldéhyde ou un libérateur de formaldéhyde.

17. Utilisation selon la revendication 16, dans laquelle :
(i) la composition de stabilisation comprend un agent de conservation pour inhiber la croissance bactérienne, ce conservateur présentant, de préférence, une ou plusieurs des caractéristiques telles que définies dans la revendication 2 ;
ii) la composition de stabilisation a un pH dans la plage de 7,2 à 9,5, 7,3 à 9,5, 7,4 à 9,5 ou 7,5 à 9,5 ;
(iii) la composition de stabilisation comprend un agent tampon ;
(iv) la composition de stabilisation comprend un agent tampon et a un pH dans la plage de 7,2 à 9,5, 7,3 à 9,5, 7,4 à 9,5 ou 7,5 à 9,5 ;
(v) le polymère de poly(oxyéthylène) est un polyéthylène glycol, dans lequel, éventuellement, la composition de stabilisation comprend au moins un polyéthylène glycol de haut poids moléculaire ayant un poids moléculaire d'au moins 3000 et éventuellement au moins un polyéthylène glycol de bas poids moléculaire ayant un poids moléculaire de 1000 ou moins ;
(vi) la composition de stabilisation comprend au moins un inhibiteur de caspase ;
(vii) la composition de stabilisation présente les caractéristiques telles que définies dans la revendication 9, 10 ou 11 ;
et/ou
(viii) la composition de stabilisation est liquide et comprend un agent chélateur, de préférence l'EDTA, dans une concentration d'au moins 15 mM, éventuellement dans une concentration de 100 mM jusqu'à la limite de solubilité.

18. Composition de stabilisation, dans laquelle la composition de stabilisation est mélangée avec un échantillon d'urine,
dans laquelle la composition de stabilisation est une composition de stabilisation pour la stabilisation d'un échantillon d'urine, en particulier de l'ADN extracellulaire compris dans celui-ci, où la croissance bactérienne est inhibée dans l'échantillon d'urine stabilisé, la composition comprenant
- un agent chélateur, de préférence l'EDTA,
- au moins un polymère de poly(oxyéthylène), et éventuellement
- un agent de conservation pour inhiber la croissance bactérienne,
où la composition de stabilisation a un pH dans la plage de > 7 à ≤ 9,5,
et où la composition de stabilisation ne comprend pas d'agent de réticulation qui induit des liaisons croisées protéine-ADN et/ou protéine-protéine tel que le formaldéhyde, la formaline, le paraformaldéhyde ou un libérateur de formaldéhyde,
éventuellement dans lequel la composition de stabilisation présente une ou plusieurs des caractéristiques suivantes :
(i) la composition de stabilisation comprend un agent de conservation pour inhiber la croissance bactérienne, où, de préférence, l'agent de conservation présente une ou plusieurs des caractéristiques telles que définies dans la revendication 2 ;
(ii) la composition de stabilisation a un pH dans la plage de 7,2 à 9,5, 7,3 à 9,5, 7,4 à 9,5 ou 7,5 à 9,5 ;
(iii) la composition de stabilisation comprend un agent tampon ;
(iv) la composition de stabilisation comprend un agent tampon et a un pH dans la plage de 7,2 à 9,5, 7,3 à 9,5, 7,4 à 9,5 ou 7,5 à 9,5 ;
(v) le polymère de poly(oxyéthylène) est un polyéthylène glycol, dans lequel, éventuellement, la composition de stabilisation comprend au moins un polyéthylène glycol de haut poids moléculaire ayant un poids moléculaire d'au moins 3000 et éventuellement au moins un polyéthylène glycol de bas poids moléculaire ayant un poids moléculaire de 1000 ou moins ;
(vi) la composition de stabilisation comprend au moins un inhibiteur de caspase ;
(vii) la composition de stabilisation présente les caractéristiques telles que définies dans la revendication 9, 10 ou 11 ;
et/ou
(viii) la composition de stabilisation est liquide et comprend un agent chélateur, de préférence l'EDTA, dans une concentration d'au moins 15 mM, éventuellement dans une concentration de 100 mM jusqu'à la limite de solubilité.
